(19) Europäisches Patentamt
European Patent Office
Office européen des brevets

(11) **EP 3 004 251 B1**

(12) # EUROPÄISCHE PATENTSCHRIFT

(45) Veröffentlichungstag und Bekanntmachung des Hinweises auf die Patenterteilung:
**13.03.2019 Patentblatt 2019/11**

(21) Anmeldenummer: **14721765.7**

(22) Anmeldetag: **29.04.2014**

(51) Int Cl.:
***C09B 23/10*** *(2006.01)*
***C07D 417/04*** *(2006.01)*
***C07D 495/04*** *(2006.01)*
***H01L 51/00*** *(2006.01)*
***C09B 7/00*** *(2006.01)*
***C09B 57/00*** *(2006.01)*
***C09B 69/00*** *(2006.01)*
***C07D 417/14*** *(2006.01)*
***C07D 285/14*** *(2006.01)*
***C09K 19/60*** *(2006.01)*
***C09K 19/30*** *(2006.01)*

(86) Internationale Anmeldenummer:
**PCT/EP2014/001149**

(87) Internationale Veröffentlichungsnummer:
**WO 2014/187529 (27.11.2014 Gazette 2014/48)**

(54) **VORRICHTUNG ZUR REGULIERUNG DES ENERGIE-DURCHTRITTS ENTHALTEND EINE DICHROITISCHE FARBSTOFFVERBINDUNG**

DEVICE FOR CONTROLLING THE PASSAGE OF ENERGY, CONTAINING A DICHROIC DYE COMPOUND

DISPOSITIF DE RÉGULATION DU PASSAGE D'ÉNERGIE CONTENANT UN COMPOSÉ DE COLORANT DICHROÏQUE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **24.05.2013 EP 13002711**

(43) Veröffentlichungstag der Anmeldung:
**13.04.2016 Patentblatt 2016/15**

(73) Patentinhaber: **Merck Patent GmbH**
**64293 Darmstadt (DE)**

(72) Erfinder:
- **KIRSCH, Peer**
  **64342 Seeheim-Jugenheim (DE)**
- **HAHN, Alexander**
  **64584 Biebesheim (DE)**
- **RUHL, Andreas**
  **64380 Rossdorf (DE)**
- **BECK, Susann**
  **64283 Darmstadt (DE)**
- **PAN, Junyou**
  **60320 Frankfurt am Main (DE)**
- **JUNGE, Michael**
  **64319 Pfungstadt (DE)**
- **BEYER, Andreas**
  **63452 Hanau (DE)**
- **PATWAL, Ursula**
  **64354 Reinheim (DE)**

(56) Entgegenhaltungen:
**WO-A1-2004/002970**
**WO-A1-2009/058877**
**WO-A1-2013/021315**
**WO-A1-2013/097919**
**WO-A2-2013/005177**
**JP-A- 2003 104 976**

- **XUELONG ZHANG ET AL: "Highly dichroic benzo-2,1,3-thiadiazole dyes containing five linearly pi-conjugated aromatic residues, with fluorescent emission ranging from green to red, in a liquid crystal guest host system", JOURNAL OF MATERIALS CHEMISTRY, ROYAL SOCIETY OF CHEMISTRY, GB, Bd. 16, Nr. 8, 28. Februar 2006 (2006-02-28), Seiten 736-740, XP008105127, ISSN: 0959-9428, DOI: 10.1039/B512493J [gefunden am 2005-12-02]**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 3 004 251 B1

## Beschreibung

Die vorliegende Erfindung betrifft eine Vorrichtung zur Regulierung des Energie-Durchtritts von einem Außenraum in einen Innenraum, wobei die Vorrichtung eine Schaltschicht enthaltend mehrere dichroitische Farbstoffe einer Formel (I) oder Formel (II) enthält.

Unter einer Vorrichtung zur Regulierung des Energie-Durchtritts wird vorliegend allgemein eine Vorrichtung verstanden, welche den Durchtritt von Energie durch eine Fläche reguliert, welche relativ gesehen hoch energiedurchlässig ist. Bevorzugt ist diese relativ gesehen hoch energiedurchlässige Fläche innerhalb einer relativ gesehen weniger energiedurchlässigen Struktur angeordnet. Beispielsweise kann die hoch energiedurchlässige Fläche eine Glasfläche oder eine offene Fläche sein, und die weniger energiedurchlässige Struktur, welche die hoch energiedurchlässige Fläche enthält, kann eine Wand sein.

Erfindungsgemäß wird durch die Vorrichtung der Durchtritt von Energie aus Sonneneinstrahlung, entweder direkt oder indirekt, reguliert.

Der regulierte Energiedurchtritt erfolgt von einem Außenraum, insbesondere der unmittelbar der Sonneneinstrahlung ausgesetzten Umwelt, in einen Innenraum, beispielsweise ein Gebäude oder ein Fahrzeug, oder eine andere von der Umgebung weitgehend abgeschlossene Einheit.

Unter dem Begriff Energie wird im Rahmen der vorliegenden Erfindung insbesondere Energie durch elektromagnetische Strahlung im UV-A-, VIS-, sowie NIR-Bereich verstanden. Insbesondere wird darunter Energie durch Strahlung verstanden, welche von den üblicherweise in Fenstern verwendeten Materialien (z. B. Glas) nicht oder nur in einem vernachlässigbaren Umfang absorbiert wird. Gemäß den üblicherweise verwendeten Definitionen wird unter dem UV-A-Bereich eine Wellenlänge von 320 bis 380 nm verstanden, unter dem VIS-Bereich eine Wellenlänge von 380 nm bis 780 nm verstanden und unter dem NIR-Bereich eine Wellenlänge von 780 nm bis 2000 nm verstanden. Entsprechend wird unter dem Begriff Licht allgemein elektromagnetische Strahlung mit Wellenlängen zwischen 320 und 2000 nm verstanden.

Unter einem dichroitischen Farbstoff wird im Rahmen der vorliegenden Erfindung eine lichtabsorbierende Verbindung verstanden, bei der die Absorptionseigenschaften von der Ausrichtung der Verbindung zur Polarisationsrichtung des Lichts abhängig sind. Typischerweise weist eine dichroitische Farbstoffverbindung gemäß der vorliegenden Erfindung eine langgestreckte Form auf, d.h. die Verbindung ist in einer Raumrichtung deutlich länger (Längsachse) als in den anderen beiden Raumrichtungen.

Auf dem Gebiet der Vorrichtungen zur Regulierung des Energie-Durchtritts von einem Außenraum in einen Innenraum sind in den vergangenen Jahren mehrere unterschiedliche technische Lösungen vorgeschlagen worden.

Eine vorteilhafte Lösung ist die Verwendung von Schaltschichten enthaltend ein flüssigkristallines Medium in Kombination mit einem oder mehreren dichroitischen Farbstoffen. Durch Anlegen einer Spannung kann bei diesen Schaltschichten eine Veränderung der räumlichen Ausrichtung der Moleküle der dichroitischen Verbindung erzielt werden, welche eine Änderung ihrer Absorption und damit der Transmission durch die Schaltschicht bewirkt. Eine entsprechende Vorrichtung ist beispielsweise in WO 2009/141295 beschrieben.

Alternativ kann eine solche Transmissionsänderung auch ohne elektrische Spannung durch einen Temperatur-induzierten Übergang von einem isotropen Zustand des flüssigkristallinen Mediums zu einem flüssigkristallinen Zustand erzielt werden, wie beispielsweise in US 2010/0259698 beschrieben.

Weiterhin ist es bekannt, Vorrichtungen enthaltend eine Schaltschicht enthaltend ein flüssigkristallines Medium mit mindestens einem dichroitischen Farbstoff so zu gestalten, dass die von dem Farbstoff absorbierte Energie teilweise als Fluoreszenzstrahlung wieder emittiert wird, die ihrerseits auf eine Solarzelle geleitet wird, welche sie in elektrische Energie umwandelt (WO 2009/141295).

Zur Verwendung in den genannten Vorrichtungen sind Rylen-Farbstoffe beschrieben worden, beispielsweise in WO 2009/141295, WO 2013/004677 und der noch nicht offengelegten EP 12008320.9.

Weiterhin sind bereits allgemein Benzothiadiazol-Verbindungen für die verschiedensten Verwendungen bekannt, beispielsweise in ChemPhysChem 2012, 13, 597 ff. zur Verwendung als organische Halbleiter, in WO 2004/002970 A1 und in Chem. Eur. J. 2008, 14, 11231 ff. zur Verwendung als OLED-Materialien, in J. Am. Chem. Soc. 1995, 117, 6791 ff. zur Verwendung als Bestandteile von Polymeren oder in J. Mater. Chem. 2006, 16 736 ff. zur Verwendung in Guest-Host-Flüssigkristall-Systemen.

Bei den oben genannten Vorrichtungen zur Regulierung des Energie-Durchtritts besteht hohes Interesse an der Entwicklung verbesserter Vorrichtungen, insbesondere bezüglich der Lebensdauer und des Schalthubs (d. h. des Unterschieds der Transmission im Hellzustand zum Dunkelzustand). Weiterhin besteht bei Vorrichtungen, welche die Fluoreszenzemission der Farbstoffe zur Wiedergewinnung von Energie mittels einer Solarzelle nutzen, Verbesserungspotential bezüglich der Energieausbeute. Im optimalen Fall sollte die durch die Solarzelle bereitgestellte Energie ausreichen, um die gesamte für den Betrieb der Vorrichtung benötigte Energie bereitzustellen, bzw. sogar über diese Menge hinausgehen.

In diesem Zusammenhang besteht hohes Interesse an der Entwicklung alternativer dichroitischer Farbstoffe,

bevorzugt solcher mit mindestens einer, besonders bevorzugt mehrerer der folgenden Eigenschaften: gute Löslichkeit im flüssigkristallinen Medium, gute Lichtstabilität und hohe Anisotropie der Absorption. Zudem sollten die Farbstoffe eine starke Lichtabsorption im VIS- und/oder NIR-Bereich des Lichts aufweisen. Für die oben genannten Vorrichtungen, die emittiertes Fluoreszenzlicht in elektrische Energie umwandeln, ist es weiterhin von großem Interesse, dass die Verbindungen eine hohe Fluoreszenzquantenausbeute, eine hohe relative Fluoreszenz aus Wellenleitung und einen hohen Stokes-Shift aufweisen.

Im Rahmen von Untersuchungen zu Farbstoffverbindungen wurde nun überraschend gefunden, dass eine oder mehrere der oben genannten technischen Aufgaben durch eine Vorrichtung enthaltend mehrere dichroitische Farbstoffe einer Formel (I) oder Formel (II) gelöst wird.

Gegenstand der vorliegenden Erfindung ist somit die Vorrichtung zur Regulierung des Energie-Durchtritts von einem Außenraum in einen Innenraum gemäß Anspruch 1, wobei die Vorrichtung eine Schaltschicht enthaltend mehrere dichroitische Farbstoffe einer Formel (I)

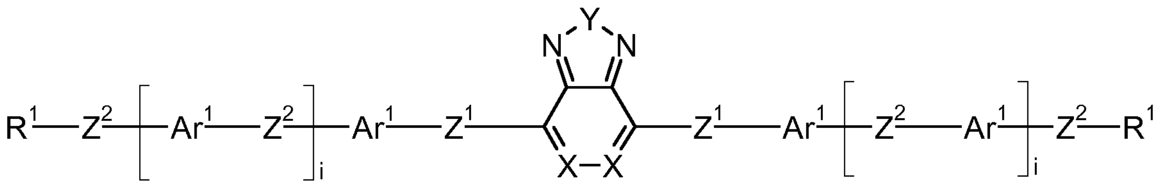

Formel (I),

oder einer Formel (II)

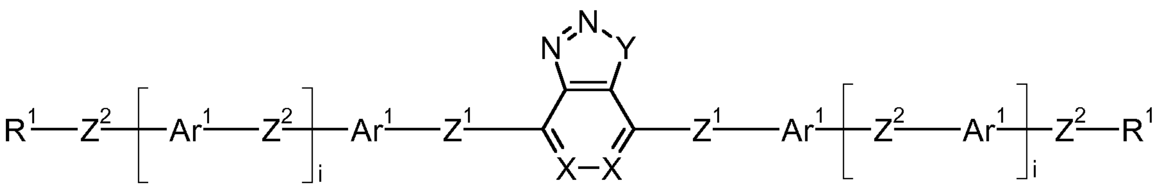

Formel (II),

enthält, wobei gilt:

X ist bei jedem Auftreten gleich oder verschieden $CR^2$ oder N;

Y ist gleich S oder Se;

$Z^1$ ist eine Einfachbindung;

$Z^2$ ist bei jedem Auftreten gleich oder verschieden eine Einfachbindung, O, S, $C(R^3)_2$, $-CR^3=CR^3-$ oder -C≡C-; oder zwei, drei, vier oder fünf miteinander kombinierte Gruppen, gewählt aus den Gruppen O, S, $C(R^3)_2$, $-CR^3=CR^3-$ und -C≡C-;

$Ar^1$ ist bei jedem Auftreten gleich oder verschieden eine Aryl- oder Heteroarylgruppe mit 5 bis 30 aromatischen Ringatomen, die mit einem oder mehreren Resten $R^4$ substituiert sein kann, wobei jeweils mindestens ein $Ar^1$ gewählt ist aus einer schwefelhaltigen Heteroarylgruppe, die mit einem oder mehreren Resten $R^4$ substituiert sein kann;

$R^1$ ist bei jedem Auftreten gleich oder verschieden H, D, F, CN, $N(R^5)_2$, oder eine Alkyl-, Alkoxy- oder Thioalkoxygruppe mit 1 bis 10 C-Atomen, die mit einem oder mehreren Resten $R^5$ substituiert sein kann, wobei eine oder mehrere $CH_2$-Gruppen in den Alkyl-, Alkoxy- oder Thioalkoxygruppen durch $-R^5C=CR^5-$, -C≡C-, C=O, C=S, -C(=O)O-, -OC(=O)-, $Si(R^5)_2$, $NR^5$, -O- oder -S- ersetzt sein können;

$R^2$ ist bei jedem Auftreten gleich oder verschieden H, D, F, Cl, CN, $-(C{=}O)OR^5$, $-O(C{=}O)R^5$ oder eine Alkyl-, Alkoxy- oder Thioalkoxygruppe mit 1 bis 10 C-Atomen, die mit einem oder mehreren Resten $R^5$ substituiert sein kann, wobei eine oder mehrere $CH_2$-Gruppen in den Alkyl-, Alkoxy- oder Thioalkoxygruppen durch $-R^5C{=}CR^5-$, -C≡C-, C=O, C=S, -C(=O)O-, -OC(=O)-, $Si(R^5)_2$, $NR^5$, -O- oder -S- ersetzt sein können;

$R^3$, $R^4$ sind bei jedem Auftreten gleich oder verschieden H, D, F, Cl, CN, oder eine Alkyl-, Alkoxy-, oder Thioalkoxygruppe mit 1 bis 10 C-Atomen, die mit einem oder mehreren Resten $R^5$ substituiert sein kann, wobei eine oder mehrere $CH_2$-Gruppen in den Alkyl-, Alkoxy- oder Thioalkoxygruppen durch $-R^5C{=}CR^5-$, -C≡C-, C=O, C=S, -C(=O)O-, -OC(=O)-, $Si(R^5)_2$, $NR^5$, -O- oder -S- ersetzt sein können;

$R^5$ ist bei jedem Auftreten gleich oder verschieden H, D, F, Cl, CN, $N(R^6)_2$, eine Alkyl-, Alkoxy- oder Thioalkoxygruppe mit 1 bis 10 C-Atomen oder eine Alkenyl- oder Alkinylgruppe mit 2 bis 20 C-Atomen, wobei die oben genannten Gruppen jeweils mit einem oder mehreren Resten $R^6$ substituiert sein können und wobei eine oder mehrere $CH_2$-Gruppen in den oben genannten Gruppen durch $-R^6C{=}CR^6-$, -C≡C-, C=O, C=S, -C(=O)O-, -O(C=O)-, $Si(R^6)_2$, $NR^6$, -O- oder -S- ersetzt sein können, oder eine Aryl- oder Heteroarylgruppe mit 5 bis 30 aromatischen Ringatomen, die jeweils mit einem oder mehreren Resten $R^6$ substituiert sein kann;

$R^6$ ist bei jedem Auftreten gleich oder verschieden H, F oder ein aliphatischer organischer Rest mit 1 bis 20 C-Atomen, in dem ein oder mehrere H-Atome durch F ersetzt sein können, oder eine Aryl- oder Heteroarylgruppe mit 5 bis 20 C-Atomen, in der ein oder mehrere H-Atome durch F ersetzt werden können;

i ist gleich 0, 1, 2, 3, 4 oder 5.

Wenn i größer als 1 ist, können die Gruppen innerhalb der Klammern gleich oder verschieden sein.

Wenn i gleich 0 ist, fällt die Gruppe innerhalb der Klammern weg, und die Gruppen $Ar^1$ und $Z^2$ sind direkt miteinander verbunden.

Unter der Formulierung "zwei, drei, vier oder fünf miteinander kombinierte Gruppen, gewählt aus den Gruppen..." ist im Sinne der vorliegenden Erfindung zu verstehen, dass die Gruppen aneinander gebunden vorliegen, bevorzugt in Form einer Kette, in der zwei, drei, vier oder fünf der Gruppen aneinander gebunden vorliegen. Bevorzugt ist eine Kombination von genau zwei oder drei Gruppen. Die Gruppen können allgemein gleich oder verschieden sein.

Eine Arylgruppe im Sinne dieser Erfindung enthält 6 bis 30 aromatische Ringatome; eine Heteroarylgruppe im Sinne dieser Erfindung enthält 5 bis 30 aromatische Ringatome, von denen mindestens eines ein Heteroatom darstellt. Die Heteroatome sind bevorzugt ausgewählt aus N, O und S. Dies stellt die grundlegende Definition dar. Werden in der Beschreibung der vorliegenden Erfindung andere Bevorzugungen angegeben, beispielsweise bezüglich der Zahl der aromatischen Ringatome oder der enthaltenen Heteroatome, so gelten diese.

Dabei wird unter einer Arylgruppe bzw. Heteroarylgruppe entweder ein einfacher aromatischer Cyclus, also Benzol, bzw. ein einfacher heteroaromatischer Cyclus, beispielsweise Pyridin, Pyrimidin oder Thiophen, oder ein kondensierter (annellierter) aromatischer bzw. heteroaromatischer Polycyclus, beispielsweise Naphthalin, Phenanthren, Chinolin oder Carbazol verstanden. Ein kondensierter (annellierter) aromatischer bzw. heteroaromatischer Polycyclus besteht im Sinne der vorliegenden Erfindung aus zwei oder mehr miteinander kondensierten einfachen aromatischen bzw. heteroaromatischen Cyclen. Ein solcher Polycyclus kann auch einzelne nichtkonjugierte Einheiten enthalten, wie es beispielsweise beim Fluoren-Grundkörper der Fall ist.

Unter einer Aryl- oder Heteroarylgruppe, die jeweils mit den oben genannten Resten substituiert sein kann und die über beliebige Positionen am Aromaten bzw. Heteroaromaten verknüpft sein kann, werden insbesondere Gruppen verstanden, welche abgeleitet sind von Benzol, Naphthalin, Anthracen, Phenanthren, Pyren, Dihydropyren, Chrysen, Perylen, Fluoranthen, Benzanthracen, Benzphenanthren, Tetracen, Pentacen, Benzpyren, Fluoren, Spirobifluoren, Furan, Benzofuran, Isobenzofuran, Dibenzofuran, Thiophen, Benzothiophen, Isobenzothiophen, Dibenzothiophen, Benzodithiophen, Cyclopentadithiophen, Thienothiophen, Indenothiophen, Dithienopyrrol, Silolodithiophen, Selenophen, Benzoselenophen, Dibenzoselenophen, Pyrrol, Indol, Isoindol, Carbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, Pyrazin, Phenazin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol.

Im Rahmen der vorliegenden Erfindung werden unter einer Alkylgruppe mit 1 bis 10 C-Atomen bzw. einer Alkenyl- oder Alkinylgruppe mit 2 bis 10 C-Atomen, in der auch einzelne H-Atome oder $CH_2$-Gruppen durch die oben

bei der Definition der Reste genannten Gruppen substituiert sein können, bevorzugt die Reste Methyl, Ethyl, n-Propyl, i-Propyl, n-Butyl, i-Butyl, s-Butyl, t-Butyl, 2-Methylbutyl, n-Pentyl, s-Pentyl, Cyclopentyl, neo-Pentyl, n-Hexyl, Cyclohexyl, neo-Hexyl, n-Heptyl, Cycloheptyl, n-Octyl, Cyclooctyl, 2-Ethylhexyl, Trifluormethyl, Pentafluorethyl, 2,2,2-Trifluorethyl, Ethenyl, Propenyl, Butenyl, Pentenyl, Cyclopentenyl, Hexenyl, Cyclohexenyl, Heptenyl, Cycloheptenyl, Octenyl, Cyclooctenyl, Ethinyl, Propinyl, Butinyl, Pentinyl, Hexinyl oder Octinyl verstanden.

Unter einer Alkoxy- oder Thioalkoxygruppe mit 1 bis 10 C-Atomen werden bevorzugt Methoxy, Trifluormethoxy, Ethoxy, n-Propoxy, i-Propoxy, n-Butoxy, i-Butoxy, s-Butoxy, t-Butoxy, n-Pentoxy, s-Pentoxy, 2-Methylbutoxy, n-Hexoxy, Cyclohexyloxy, n-Heptoxy, Cycloheptyloxy, n-Octyloxy, Cyclooctyloxy, 2-Ethylhexyloxy, Pentafluorethoxy, 2,2,2-Trifluorethoxy, Methylthio, Ethylthio, n-Propylthio, i-Propylthio, n-Butylthio, i-Butylthio, s-Butylthio, t-Butylthio, n-Pentylthio, s-Pentylthio, n-Hexylthio, Cyclohexylthio, n-Heptylthio, Cycloheptylthio, n-Octylthio, Cyclooctylthio, 2-Ethylhexylthio, Trifluormethylthio, Pentafluorethylthio, 2,2,2-Trifluorethylthio, Ethenylthio, Propenylthio, Butenylthio, Pentenylthio, Cyclopentenylthio, Hexenylthio, Cyclohexenylthio, Heptenylthio, Cycloheptenylthio, Octenylthio, Cyclooctenylthio, Ethinylthio, Propinylthio, Butinylthio, Pentinylthio, Hexinylthio, Heptinylthio oder Octinylthio verstanden.

Unter einem aliphatischen organischen Rest mit 1 bis 20 C-Atomen wird prinzipiell ein beliebiger organischer Rest verstanden, welcher nicht aromatisch bzw. heteroaromatisch ist. Bevorzugt werden darunter Alkylgruppen mit 1 bis 10 C-Atomen, Alkoxygruppen mit 1 bis 10 C-Atomen, Alkenyl- oder Alkinylgruppen mit 2 bis 10 C-Atomen, wie oben näher beschrieben, verstanden.

Bevorzugt steht höchstens eine Gruppe X in der Verbindung der Formel (I) oder Formel (II) für N. Es ist allgemein erfindungsgemäß bevorzugt, dass X für $CR^2$ steht.

Weiterhin ist es bevorzugt, dass Y für S steht.

Für $Z^2$ ist es bevorzugt, dass es bei jedem Auftreten gleich oder verschieden für eine Einfachbindung, $-C(R^3)_2C(R^3)_2-$, $-CR^3=CR^3-$, -C=C-, $-OC(R^3)_2-$ oder $-C(R^3)_2O-$ steht, besonders bevorzugt für eine Einfachbindung, $-CH_2CH_2-$, $-CF_2CF_2-$, -CH=CH-, -CF=CF-, -C=C-, $-OCH_2-$, $-OCF_2-$, $-CH_2O-$ oder $-CF_2O-$.

Für die in Anspruch 1 definierte Gruppe $Ar^1$ ist es bevorzugt, dass es bei jedem Auftreten gleich oder verschieden eine Arylgruppe mit 6 bis 15 C-Atomen oder eine Heteroarylgruppe mit 5 bis 15 C-Atomen darstellt, die mit einem oder mehreren Resten $R^4$ substituiert sein kann. Besonders bevorzugt ist die in Anspruch 1 definierte Gruppe $Ar^1$ bei jedem Auftreten gleich oder verschieden gewählt aus wahlweise mit Resten $R^4$ substituiertem Benzol, Fluoren, Naphthalin, Pyridin, Pyrimidin, Pyrazin, Triazin, Thiophen, Thiophen mit ankondensiertem 1,4-Dioxanring, Benzothiophen, Dibenzothiophen, Benzodithiophen, Cyclopentadithiophen, Thienothiophen, Indenothiophen, Dithienopyrrol, Silolodithiophen, Selenophen, Benzoselenophen, Dibenzoselenophen, Furan, Benzofuran, Dibenzofuran und Chinolin.

Die Gruppe $R^1$ ist bevorzugt bei jedem Auftreten gleich oder verschieden H, F, CN, $N(R^5)_2$, oder eine geradkettige Alkyl- oder Alkoxylgruppe mit 1 bis 10 C-Atomen, die mit einem oder mehreren Resten $R^5$ substituiert sein kann, oder eine verzweigte Alkyl- oder Alkoxygruppe mit 3 bis 10 C-Atomen, die mit einem oder mehreren Resten $R^5$ substituiert sein kann, oder eine cyclische Alkylgruppe mit 4 bis 8 C-Atomen, die mit einem oder mehreren Resten $R^5$ substituiert sein kann, wobei in den Alkyl- und Alkoxygruppen eine oder mehrere $CH_2$-Gruppen durch -O-, -S- oder - $R^5C=CR^5$- ersetzt sein können, oder eine Siloxanylgruppe mit 1 bis 10 Si-Atomen, die mit einem oder mehreren Resten $R^5$ substituiert sein kann.

Ganz besonders bevorzugt ist $R^1$ bei jedem Auftreten gleich oder verschieden H, F, oder eine geradkettige Alkyl- oder Alkoxylgruppe mit 3 bis 8 C-Atomen, die mit einem oder mehreren Resten $R^5$ substituiert sein kann, oder eine verzweigte Alkyl- oder Alkoxygruppe mit 3 bis 8 C-Atomen, die mit einem oder mehreren Resten $R^5$ substituiert sein kann, oder eine cyclische Alkylgruppe mit 6 C-Atomen, die mit einem oder mehreren Resten $R^5$ substituiert sein kann, wobei in den Alkyl- und Alkoxygruppen eine oder mehrere $CH_2$-Gruppen durch -O-, -S- oder -$R^5C=CR^5$- ersetzt sein können, oder eine Siloxanylgruppe mit 1 bis 6 Si-Atomen, die mit einem oder mehreren Resten $R^5$ substituiert sein kann.

Die Gruppe $R^2$ ist bevorzugt bei jedem Auftreten gleich oder verschieden H, F, Cl, CN, oder eine Alkyl- oder Alkoxylgruppe mit 1 bis 10 C-Atomen, die mit einem oder mehreren Resten $R^5$ substituiert sein kann. Ganz besonders bevorzugt ist $R^2$ bei jedem Auftreten gleich oder verschieden H, F, oder Cl. Am stärksten bevorzugt ist $R^2$ gleich H.

$R^3$ ist bevorzugt bei jedem Auftreten gleich oder verschieden H, F, oder eine Alkylgruppe mit 1 bis 10 C-Atomen, die mit einem oder mehreren Resten $R^5$ substituiert sein kann. Besonders bevorzugt ist $R^3$ bei jedem Auftreten gleich oder verschieden H oder F.

$R^4$ ist bevorzugt bei jedem Auftreten gleich oder verschieden H, D, F, CN, oder eine Alkyl- oder Alkoxygruppe mit 1 bis 10 C-Atomen, die mit einem oder mehreren Resten $R^5$ substituiert sein kann. Besonders bevorzugt ist $R^4$ bei jedem Auftreten gleich oder verschieden H oder F.

$R^5$ ist bei jedem Auftreten gleich oder verschieden H, F, CN, oder eine Alkyl- oder Alkoxygruppe mit 1 bis 10 C-Atomen, die mit einem oder mehreren Resten $R^6$ substituiert sein kann, oder eine Aryl- oder Heteroarylgruppe mit 5 bis 30 aromatischen Ringatomen, die jeweils mit einem oder mehreren Resten $R^6$ substituiert sein kann, oder eine Siloxanylgruppe mit 1 bis 6 Si-Atomen, die mit einem oder mehreren Resten $R^6$ substituiert sein kann.

Für den Index i gilt, dass er bevorzugt gleich 1,2, oder 3 ist, besonders bevorzugt gleich 1 oder 2, ganz besonders

bevorzugt gleich 1.

Bevorzugte Ausführungsformen der Formel (I) sind die folgenden Formeln. (I-1) und (I-2)

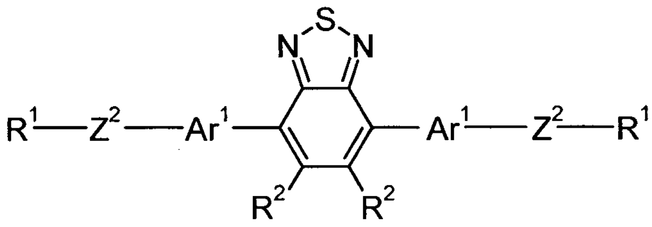

Formel (I-1)

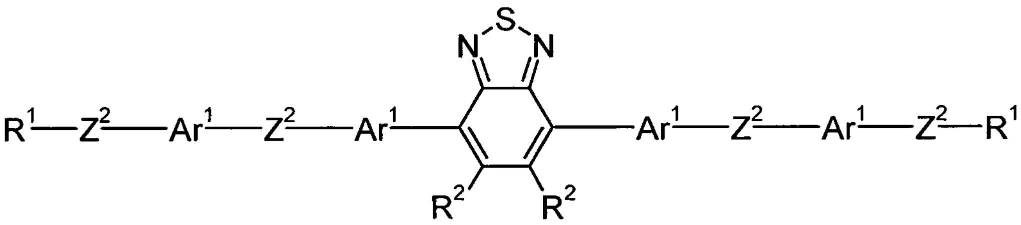

Formel (I-2),

wobei die auftretenden Gruppen wie oben definiert sind.

Bevorzugt gelten für Formeln (I-1) und (I-2) die oben angegebenen bevorzugten Ausführungsformen der Gruppen $Ar^1$, $Z^2$, $R^1$ und $R^2$.

Es ist für die Formeln (I-1) und (I-2) bevorzugt, dass mindestens ein direkt an Benzothiadiazol gebundendes $Ar^1$ für eine schwefelhaltige Heteroarylgruppe steht, besonders bevorzugt für Thiophen. Die Gruppe kann mit einem oder mehreren Resten $R^4$ substituiert sein. Derartige Verbindungen zeichnen sich durch eine besonders hohe Lichtstabilität aus.

Insbesondere ist es für Formeln (I-1) und (I-2) bevorzugt, dass $R^2$ für H, F, oder Cl steht, besonders bevorzugt für H.

Bevorzugte Ausführungsformen von Verbindungen der Formel (I-1) und (I-2) entsprechen den Formeln (I-1-1) bis (I-1-4) und (I-2-1) bis (I-2-4)

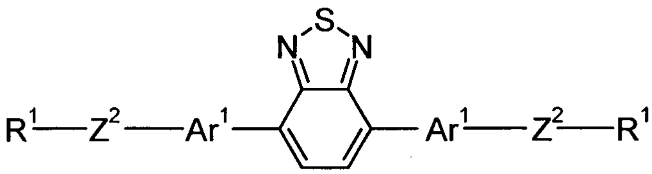

Formel (I-1-1)

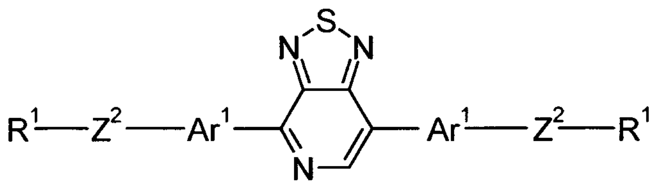

Formel (I-1-2)

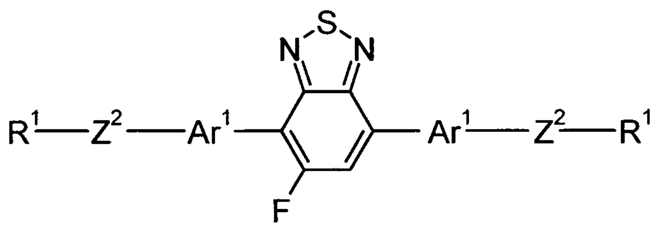

Formel (I-1-3)

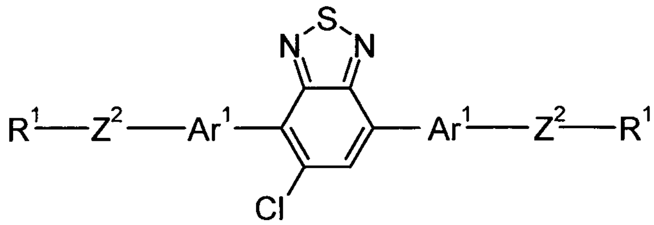

Formel (I-1-4)

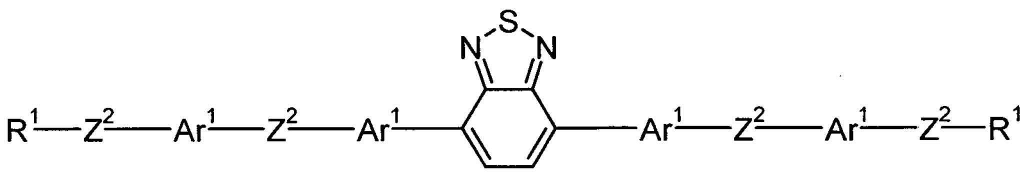

Formel (I-2-1)

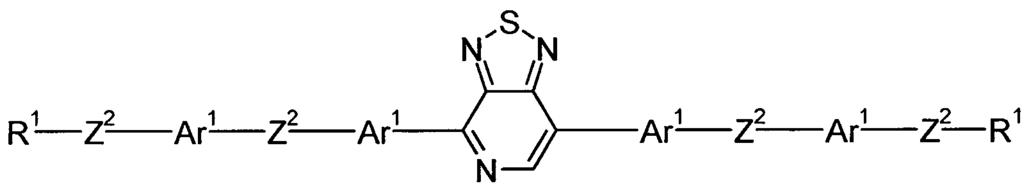

Formel (I-2-2)

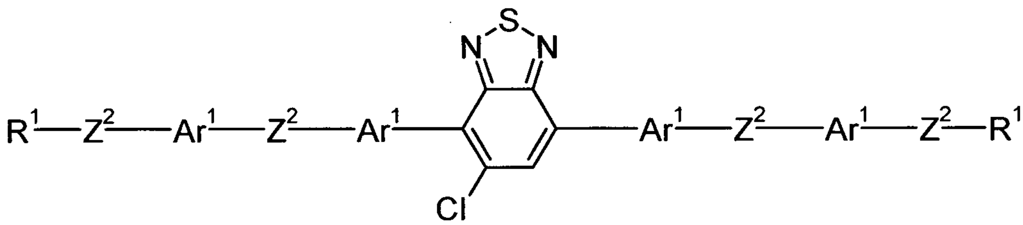

Formel (I-2-3)

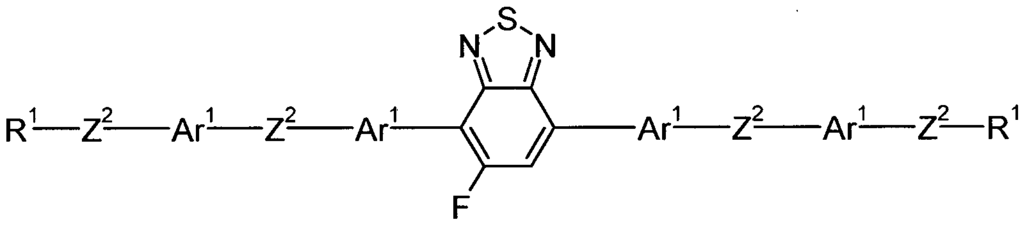

For

mel (I-2-4),

wobei die auftretenden Gruppen $Ar^1$, $Z^2$ und $R^1$ wie oben definiert sind.

Bevorzugt gelten für Formeln (I-1-1) bis (I-1-4) und (I-2-1) bis (I-2-4) die oben angegebenen bevorzugten Ausführungsformen der Gruppen $Ar^1$, $Z^2$, und $R^1$.

Unter den Formeln (I-1-1) bis (I-1-4) und (I-2-1) bis (I-2-4) sind die Formeln (I-1-1) und (I-2-1) besonders bevorzugt.

Bevorzugte Ausführungsformen von Verbindungen der Formel (I-1-1) und (I-2-1) sind die folgenden Formeln:

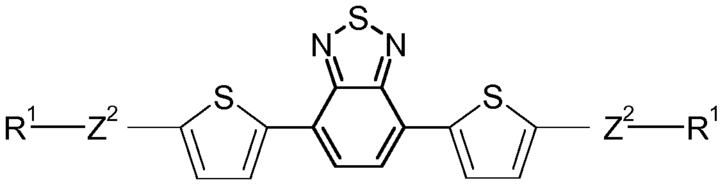

Formel (I-1-1-3)

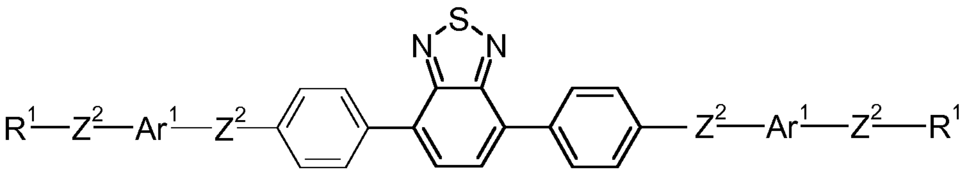

Formel (I-2-1-1)

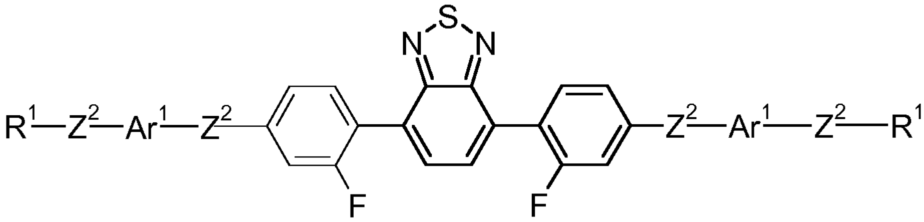

Formel (I-2-1-2)

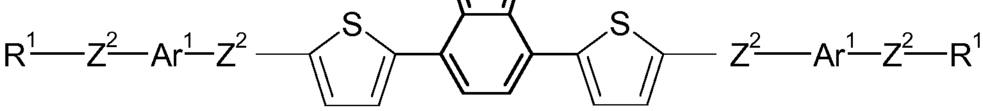

Formel (I-2-1-3)

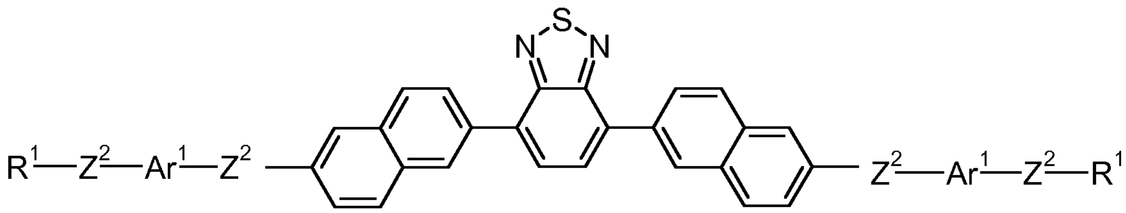

Formel (I-2-1-4),

wobei die auftretenden Gruppen $Ar^1$, $Z^2$ und $R^1$ definiert sind wie in Anspruch 1 und oben.

Bevorzugt gelten für Formeln (I-1-1-3) und (I-2-1-1) bis (I-2-1-4) die oben angegebenen bevorzugten Ausführungsformen der Gruppen $Ar^1$, $Z^2$, und $R^1$.

Betreffend die oben beschriebenen bevorzugten Ausführungsformen der variablen Gruppen von Formel (I) oder Formel (II) gilt bevorzugt, dass diese in Kombination miteinander auftreten.

Weiterer Gegenstand der vorliegenden Erfindung sind spezielle Verbindungen gemäß Formel (I), die den folgenden Formeln (Ia) und (Ib) entsprechen

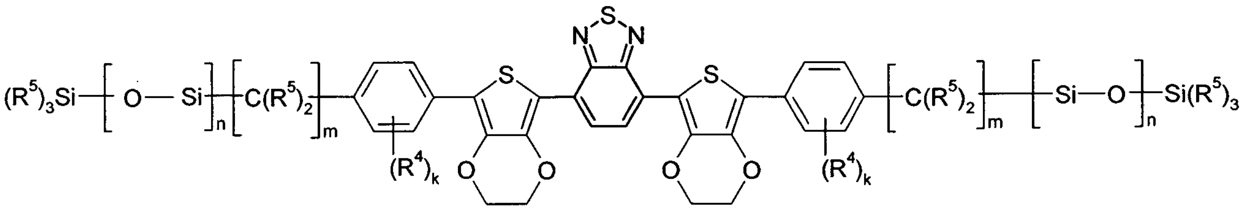

Formel (Ia)

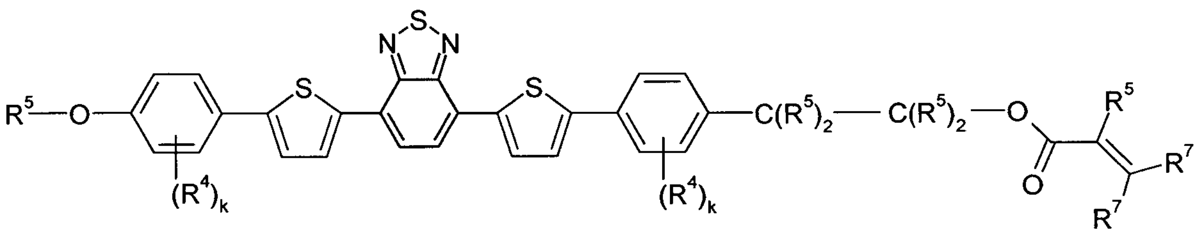

Formel (Ib),

wobei gilt:

die auftretenden Gruppen $R^4$ und $R^5$ sind definiert wie oben, $R^7$ ist definiert wie $R^5$ oben, und

k ist, gleich oder verschieden bei jedem Auftreten, 0, 1, 2, 3 oder 4;

m ist, gleich oder verschieden bei jedem Auftreten, 0, 1, 2, 3, 4, 5 oder 6;

n ist, gleich oder verschieden bei jedem Auftreten, 1, 2, 3, 4 oder 5.

In einer bevorzugten Ausführungsform ist der Index k in den oben genannten Formeln gleich oder verschieden 0 oder 1, besonders bevorzugt gleich 0.

In einer bevorzugten Ausführungsform ist der Index m in den oben genannten Formeln gleich oder verschieden 0, 1 oder 2, besonders bevorzugt gleich 1 oder 2.

In einer bevorzugten Ausführungsform ist der Index n in den oben genannten Formeln gleich oder verschieden 1,2 oder 3, besonders bevorzugt gleich 2.

Weiterhin bevorzugt ist $R^5$ in den oben genannten Formeln Wasserstoff oder eine Alkylgruppe mit 1 bis 5 C-Atomen, die mit einem oder mehreren Resten $R^6$ substituiert sein kann, besonders bevorzugt Methyl.

In einer bevorzugten Ausführungsform ist $R^7$ in der oben genannten Formel Wasserstoff oder eine Alkylgruppe

mit 1 bis 5 C-Atomen die mit einem oder mehreren Resten $R^6$ substituiert sein kann, besonders bevorzugt Wasserstoff.

Die Verbindungen der Formel (Ia) und (Ib) weisen besonders große Vorteile in Bezug auf die oben genannten Eigenschaften gute Löslichkeit im flüssigkristallinen Medium, gute Lichtstabilität, hohe Fluoreszenz und/oder hohe Anisotropie der Absorption bzw. dichroitisches Verhältnis auf.

Ferner beschrieben hierin sind spezielle Verbindungen gemäß Formel (II), die der folgenden Formel (IIa) entsprechen

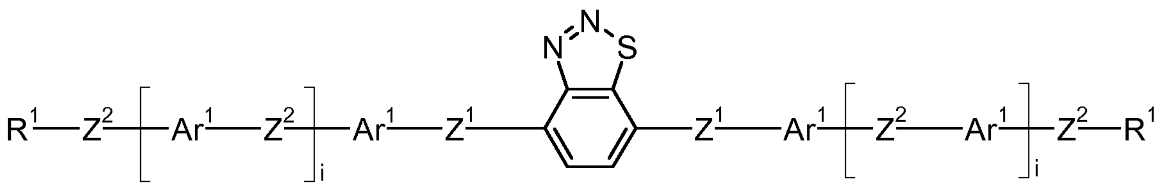

Formel (IIa),

wobei gilt:

die auftretenden Gruppen $R^1$, $Ar^1$, $Z^1$ und $Z^2$ sowie der Index i sind definiert wie oben.

Bevorzugt ist der Index i in der oben genannten Formel (IIa) gleich oder verschieden 0 ,1 oder 2, besonders bevorzugt gleich 1.

Weiterhin bevorzugt ist $Z^1$ in der oben genannten Formel (IIa) eine Einfachbindung.

Bevorzugt ist $Ar^1$ in der oben genannten Formel (IIa) ein mit $R^4$ substituierter Arylrest, besonders bevorzugt ein mit $R^4$ substituiertes Benzol, Fluoren, Naphthalin, Pyridin, Pyrimidin, Pyrazin, Triazin, Thiophen, Thiophen mit ankondensiertem 1,4-Dioxanring, Benzothiophen, Dibenzothiophen, Benzodithiophen, Cyclopentadithiophen, Thienothiophen, Indenothiophen, Dithienopyrrol, Silolodithiophen, Selenophen, Benzoselenophen, Dibenzoselenophen, Furan, Benzofuran, Dibenzofuran und Chinolin.

Folgende Verbindungen sind Beispiele für Verbindungen gemäß Formel (I) oder Formel (II), bzw. (Ia) oder (Ib):

| Tabelle 1 |
|---|
| 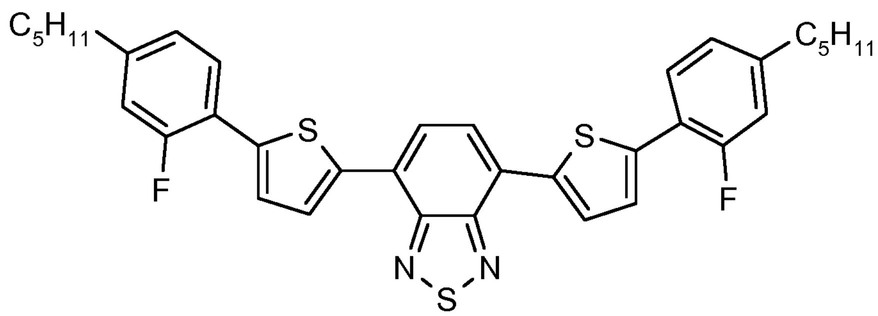 |
| (1) |
| 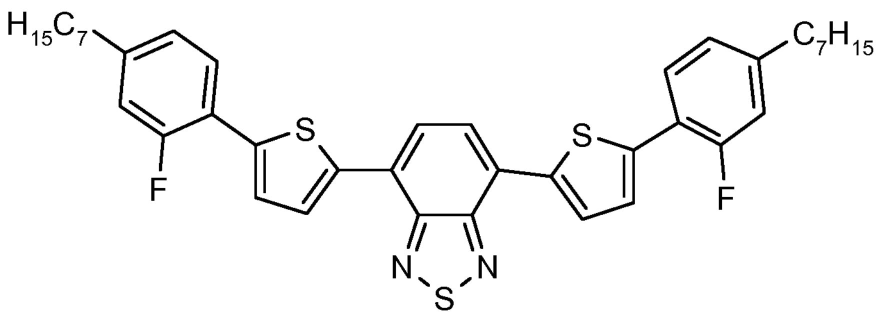 |
| (2) |

(fortgesetzt)

| Tabelle 1 |
| --- |
| 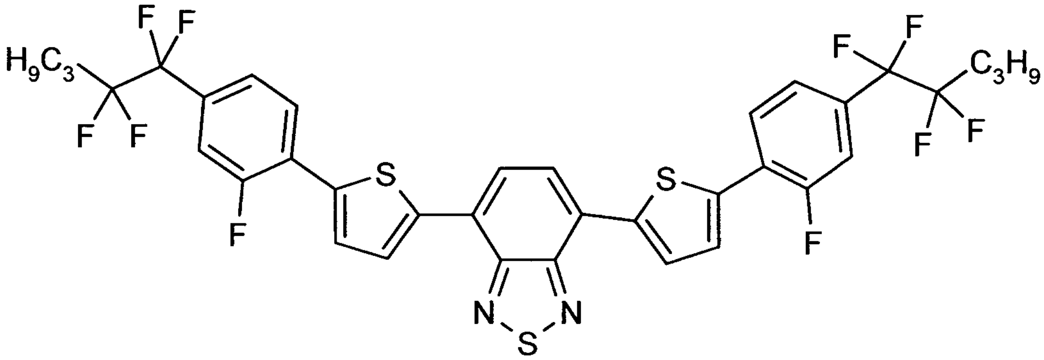 |
| (3) |
| 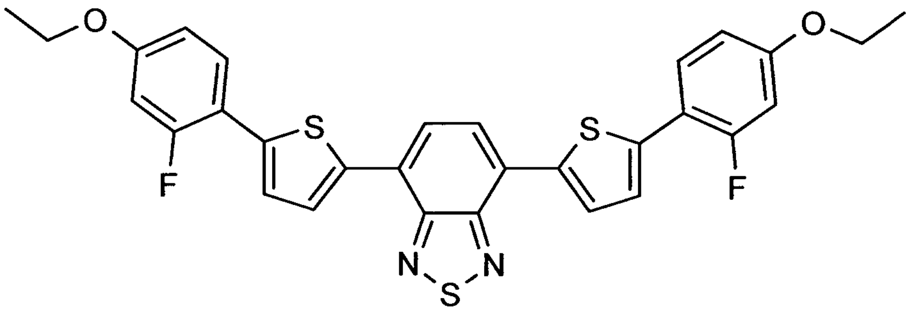 |
| (4) |
| 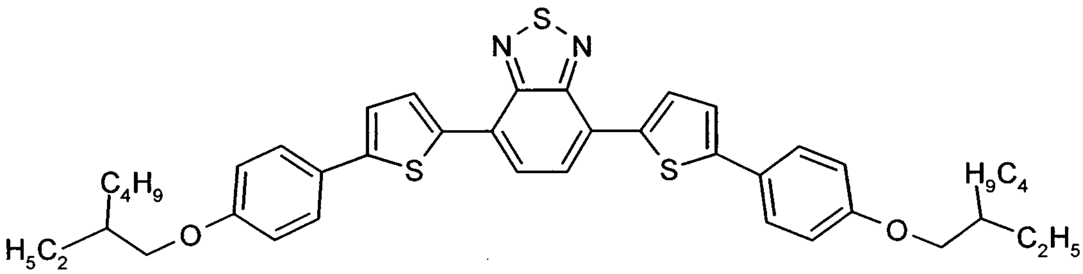 |
| (5) |
| 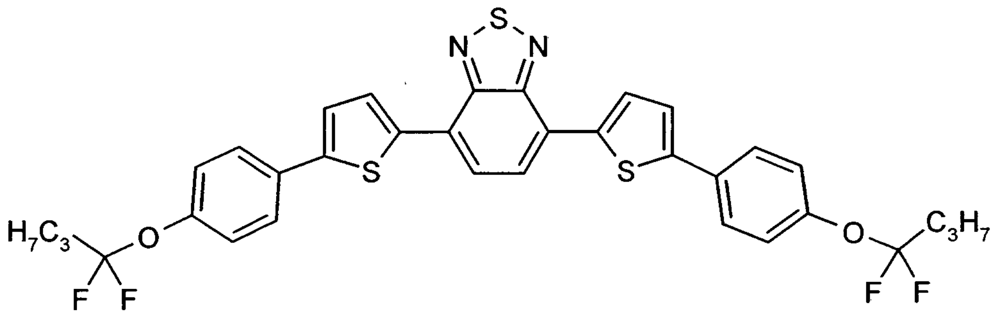 |
| (6) |

(fortgesetzt)

| Tabelle 1 |
| --- |
| 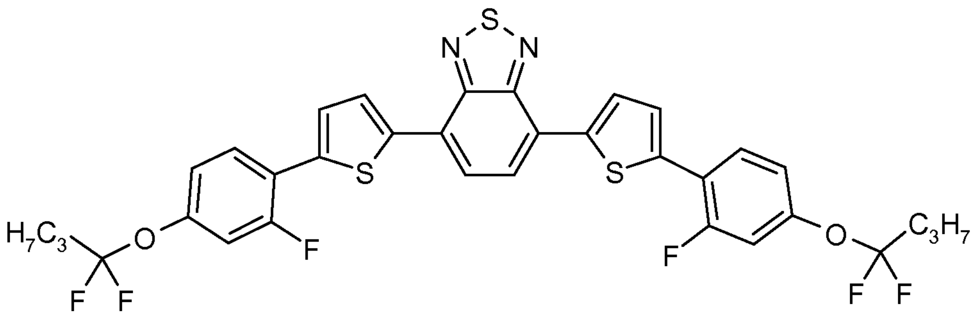 |
| (7) |
| 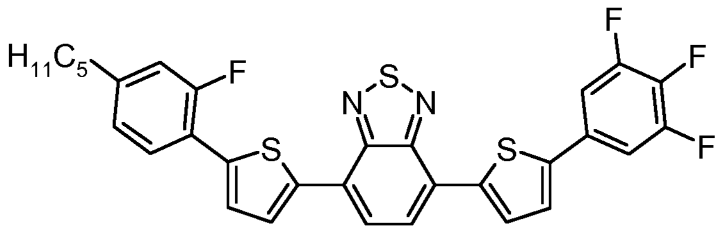 |
| (10) |
| 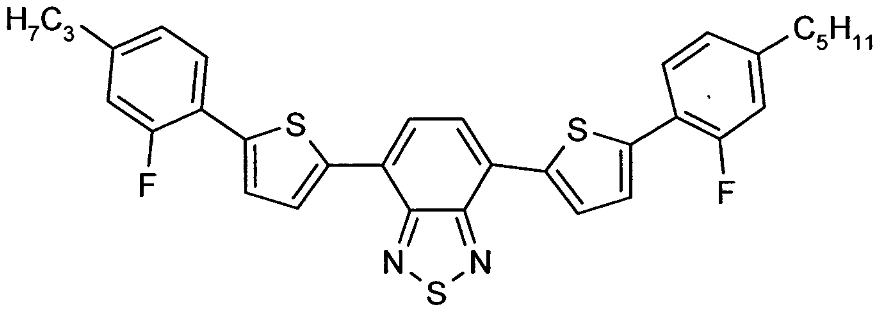 |
| (11) |
| 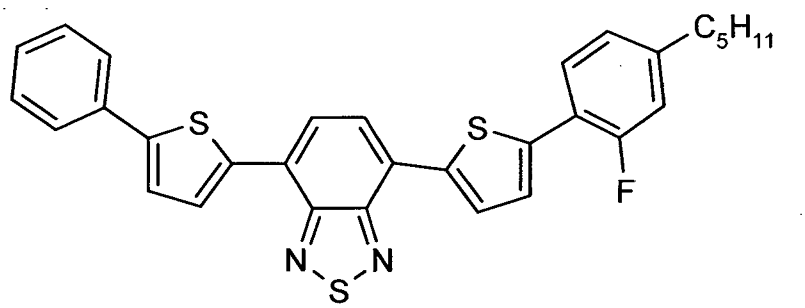 |
| (12) |

(fortgesetzt)

| Tabelle 1 |
|---|
| 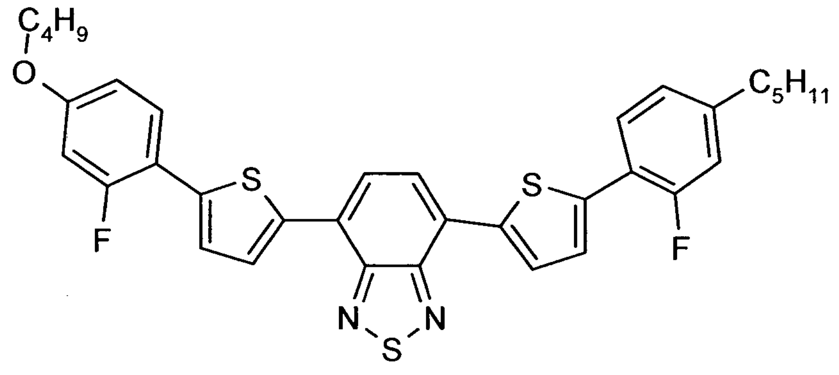 |
| (13) |
| 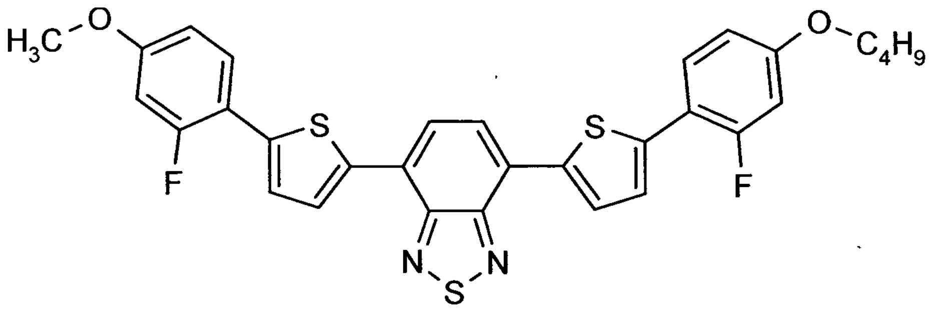 |
| (14) |
| 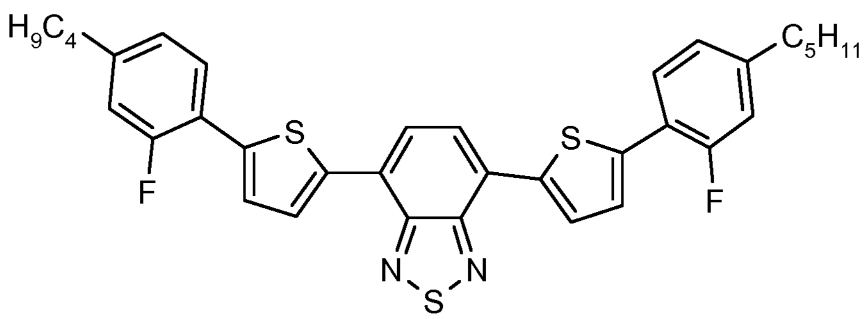 |
| (15) |
| 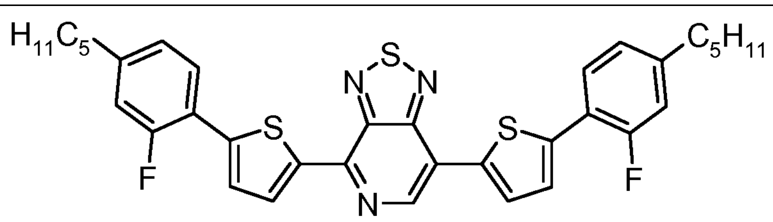 |
| (32) |

(fortgesetzt)

| Tabelle 1 |
|---|
| 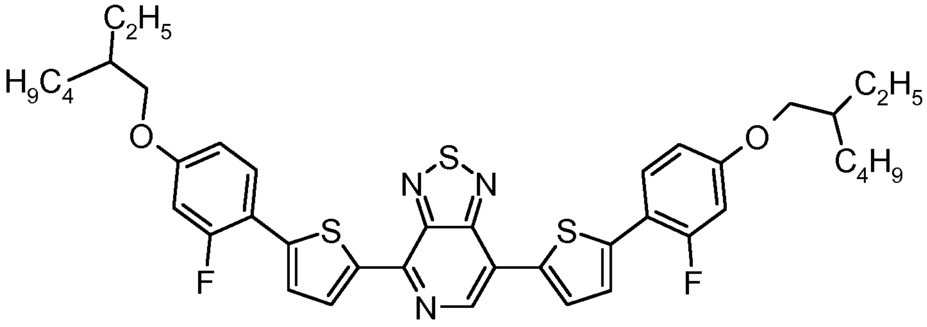 |
| (33) |
| 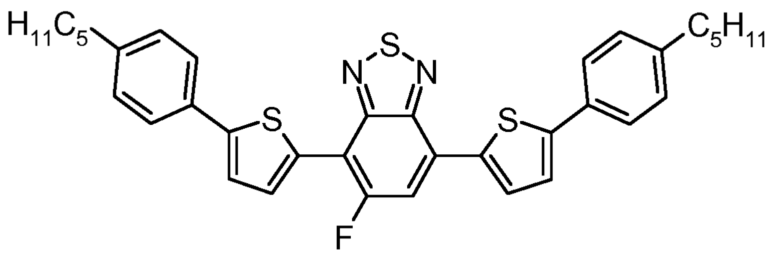 |
| (35) |
| 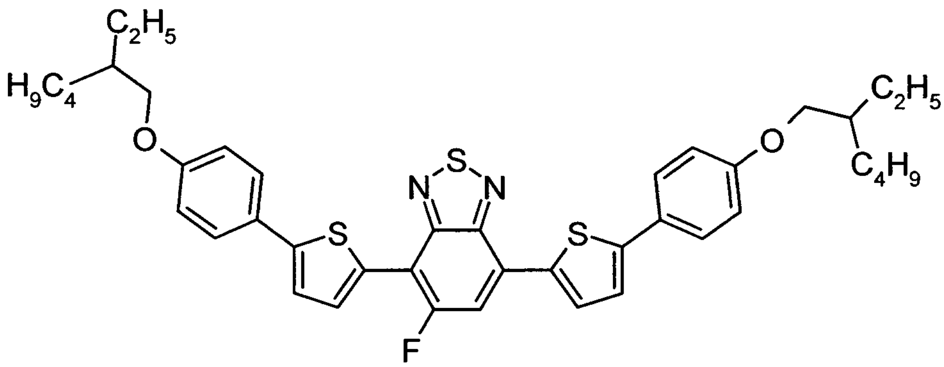 |
| (36) |
| 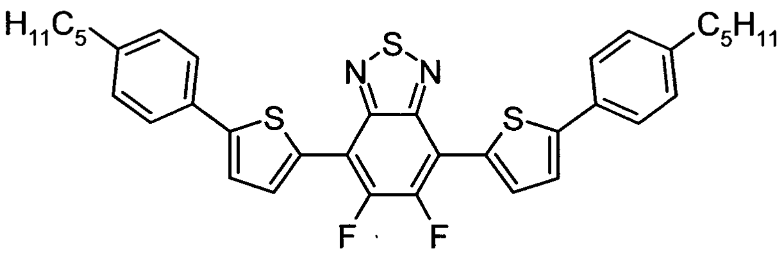 |
| (37) |

(fortgesetzt)

| Tabelle 1 |
|---|
| 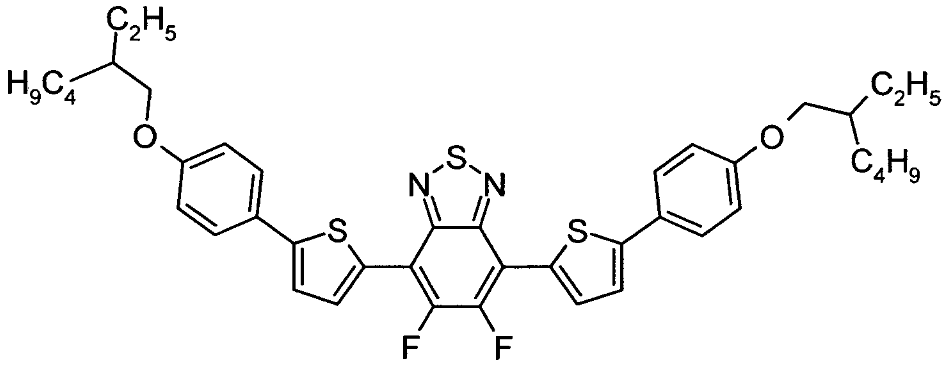 |
| (38) |
| 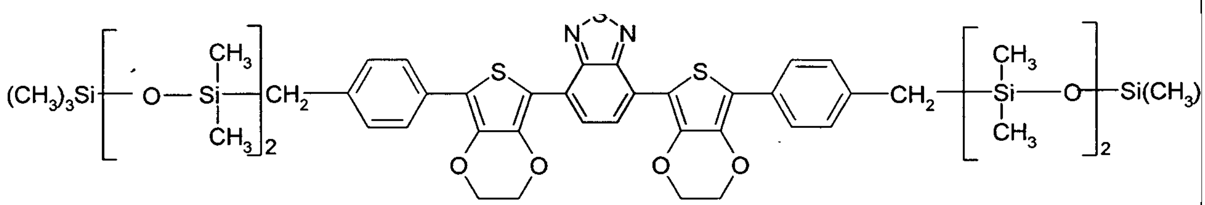 |
| (39) |
| 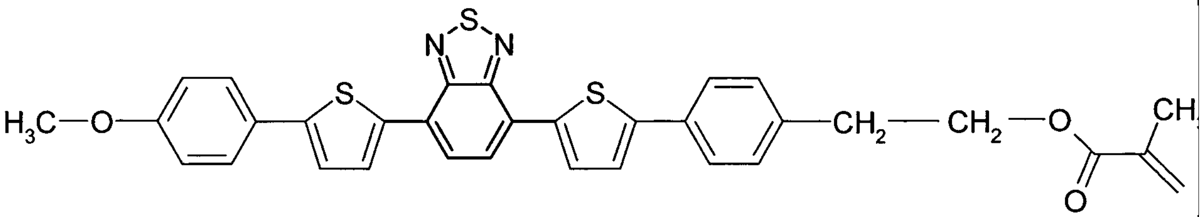 |
| (40) |
| 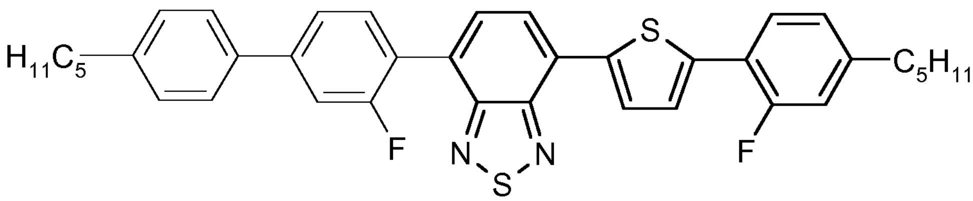 |
| (41) |
| 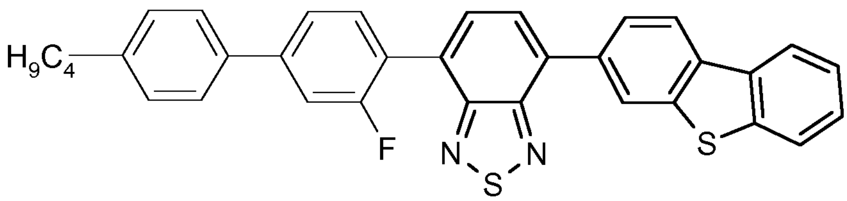 |
| (42) |

(fortgesetzt)

| Tabelle 1 |
|---|
| 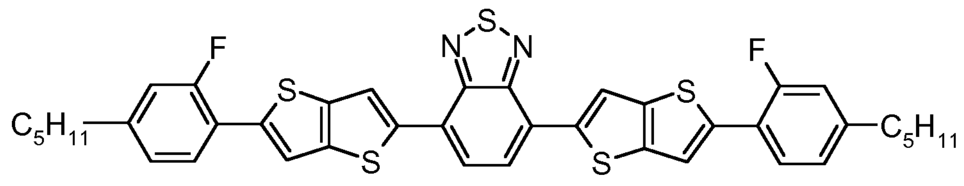 |
| (44) |
| 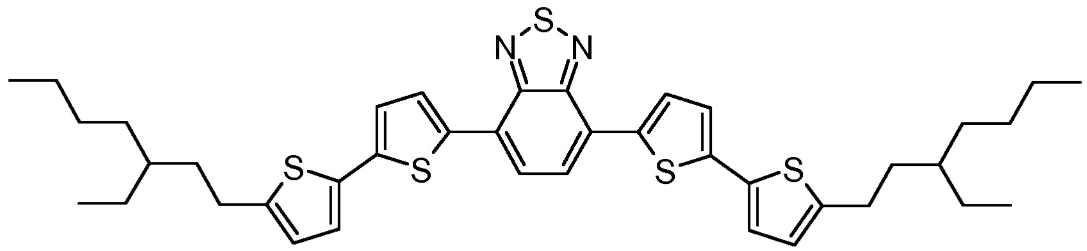 |
| (45) |
| 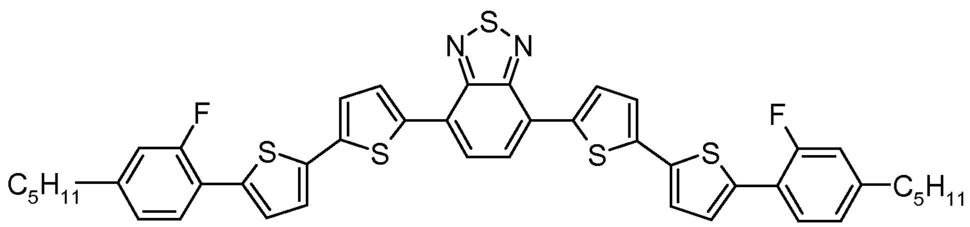 |
| (46) |
| 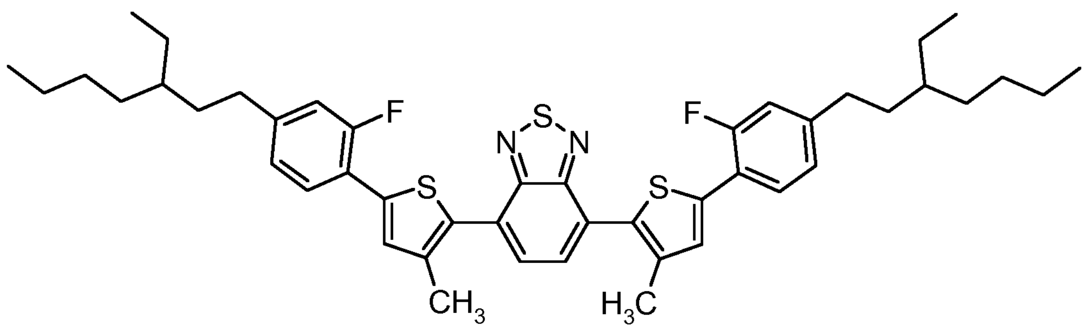 |
| (47) |
| 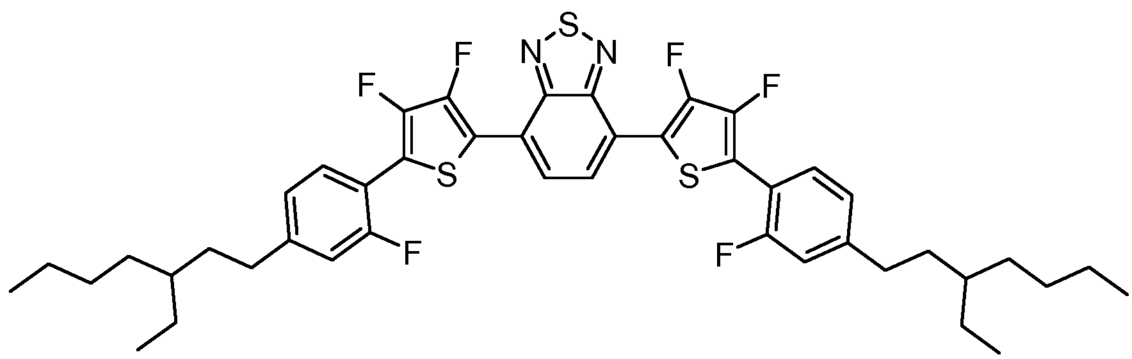 |
| (48) |

(fortgesetzt)

| Tabelle 1 |
| --- |
| 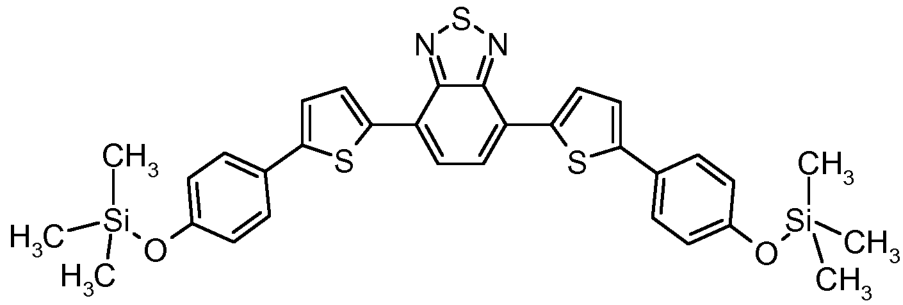 |
| (49) |
| 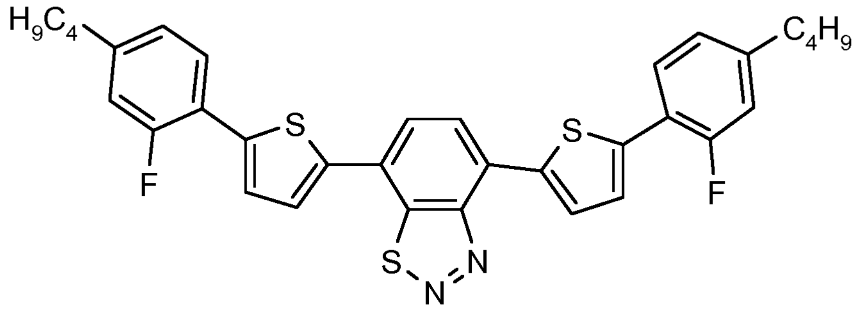 |
| (52) |

Die Verbindungen nach Formel (I) und Formel (II) können gemäß bekannten Verfahren der organischen Chemie hergestellt werden, insbesondere durch Suzuki-Kupplung zwischen organischen Bromiden und organischen Boronsäuren. Im Folgenden werden besonders geeignete Verfahren in allgemeiner Form genannt. Für konkrete Verfahren zur Herstellung von Verbindungen der Formel (I) und Formel (II) wird weiterhin auf die bekannte Literatur und auf die Ausführungsbeispiele verwiesen.

Ein mögliches, bevorzugtes Verfahren zur Herstellung von Verbindungen der Formel (I) und Formel (II) basiert auf einem Benzothiadiazol-Derivat, welches zwei Brom- oder Chlor-Substituenten trägt. Durch Suzuki-Kupplung mit geeigneten Boronsäure-Derivaten können daraus Verbindungen der Formel (I) und Formel (II) hergestellt werden, wie die folgenden Schemata 1 und 2 zeigen.

## Schema 1

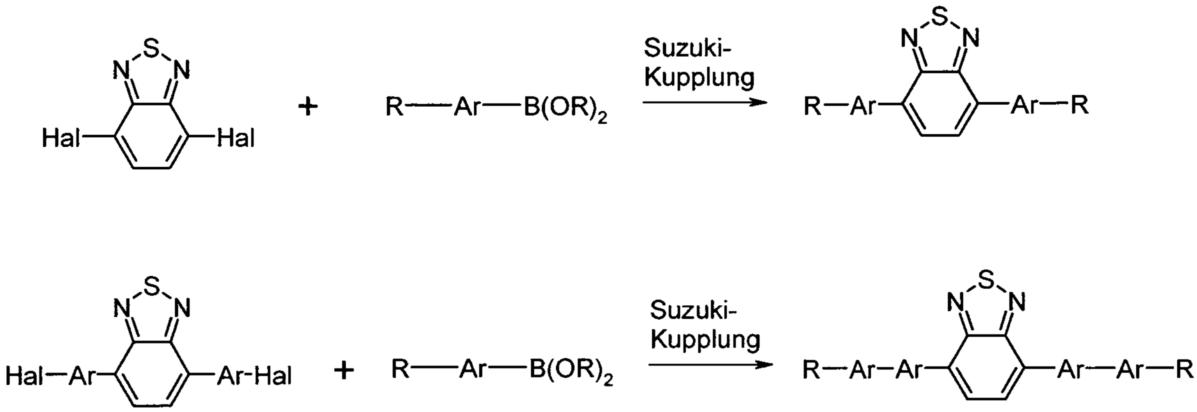

Schema 2

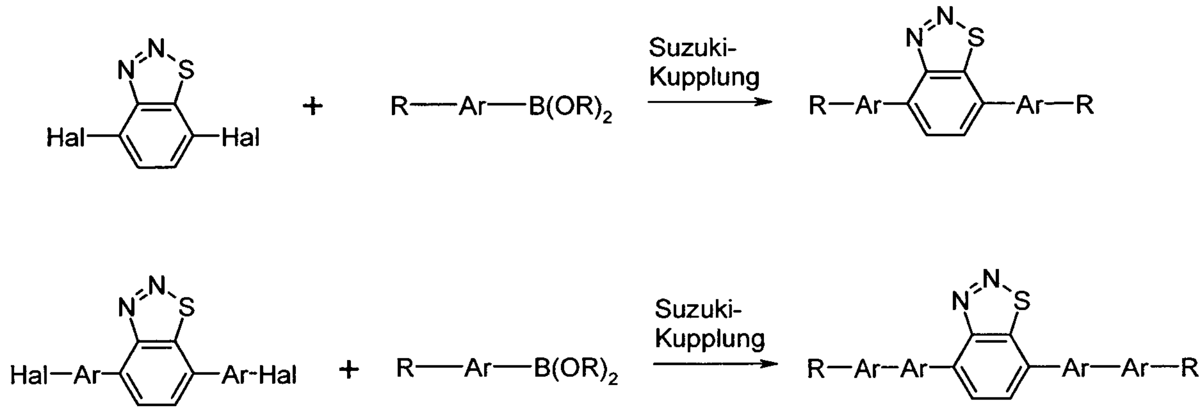


Die Verbindung der Formel (I) oder Formel (II) ist bevorzugt ein positiv dichroitischer Farbstoff, d. h. ein Farbstoff, welcher einen positiven Anisotropiegrad R, ermittelt wie in den Ausführungsbeispielen angegeben, aufweist. Besonders bevorzugt ist der Anisotropiegrad R größer als 0.4, ganz besonders bevorzugt größer als 0.6 und am stärksten bevorzugt größer als 0.7, wobei R ermittelt wird wie in den Ausführungsbeispielen angegeben.

Bevorzugt erreicht die Absorption ein Maximum, wenn die Polarisationsrichtung des Lichts parallel zur Richtung der längsten Ausdehnung des Moleküls gemäß Formel (I) oder Formel (II) ist, und sie erreicht ein Minimum, wenn die Polarisationsrichtung des Lichts senkrecht zur Richtung der längsten Ausdehnung des Moleküls gemäß Formel (I) oder Formel (II) ist.

Weiterhin bevorzugt ist die Verbindung der Formel (I) oder Formel (II) ein fluoreszierender Farbstoff. Unter Fluoreszenz wird dabei verstanden, dass eine Verbindung durch Absorption von Licht einer bestimmten Wellenlänge in einen elektronisch angeregten Zustand versetzt wird, wobei die Verbindung anschließend unter Emission von Licht in den Grundzustand übergeht. Bevorzugt hat das emittierte Licht längere Wellenlänge als das absorbierte Licht. Weiterhin bevorzugt ist der Übergang vom angeregten Zustand in den Grundzustand spin-erlaubt, erfolgt also ohne Änderung des Spins. Weiterhin bevorzugt ist die Lebensdauer des angeregten Zustands der fluoreszierenden Verbindung kürzer als $10^{-5}$ s, besonders bevorzugt kürzer als $10^{-6}$ s, ganz besonders bevorzugt zwischen $10^{-9}$ und $10^{-7}$ s.

Die dichroitischen Verbindungen der Formel (I) oder Formel (II) liegen bevorzugt in einem Anteil von 0.01 bis 10 Gew.-%, besonders bevorzugt 0.05 bis 7 Gew.-% und ganz besonders bevorzugt 0.1 bis 7 Gew.-% in der Schaltschicht vor.

Neben den Verbindungen der Formel (I) oder Formel (II) liegt in der Schaltschicht erfindungsgemäß ein flüssigkristallines Medium enthaltend eine oder mehrere verschiedene Verbindungen vor. Bevorzugt stellt das flüssigkristalline Medium die Hauptkomponente der Mischung der Schaltschicht der erfindungsgemäßen Vorrichtung dar. Die dichroitischen Verbindungen der Formel (I) oder Formel (II) liegen in der Schaltschicht bevorzugt in Lösung vor. Sie werden bevorzugt in ihrer Ausrichtung durch die Ausrichtung der Verbindungen des flüssigkristallinen Mediums beeinflusst.

Unter dem Begriff flüssigkristallines Medium wird im Rahmen der vorliegenden Erfindung ein Material verstanden, das unter bestimmten Bedingungen flüssigkristalline Eigenschaften aufweist. Bevorzugt hat das Material bei Raumtemperatur und in einer gewissen Temperaturspanne darüber und darunter flüssigkristalline Eigenschaften. Das flüssigkristalline Medium kann eine einzige Verbindung enthalten, oder es kann mehrere unterschiedliche Verbindungen enthalten. Typischerweise enthält das flüssigkristalline Medium gemäß der Erfindung mindestens eine Verbindung, deren Moleküle eine langgestreckte Form aufweisen, d.h. in einer Raumrichtung deutlich länger (Längsachse) sind als in den anderen beiden Raumrichtungen.

Weiterer Gegenstand der Erfindung ist die Verwendung der Mischung gemäß Anspruch 14, enthaltend ein flüssigkristallines Medium und mehrere Verbindungen der Formel (I) oder Formel (II), in einer Vorrichtung zur Regulierung des Energie-Durchtritts von einem Außenraum in einen Innenraum, wie in Anspruch 14 definiert.

Erfindungsgemäß hat das flüssigkristalline Medium der Schaltschicht einen Klärpunkt, bevorzugt einen Phasenübergang von einem nematisch flüssigkristallinen Zustand zu einem isotropen Zustand, im Temperaturbereich von 70 °C bis 170 °C, bevorzugt von 90 °C bis 160 °C, besonders bevorzugt von 95 °C bis 150 °C und ganz besonders bevorzugt von 105 °C bis 140 °C.

Bevorzugt ist weiterhin die dielektrische Anisotropie des flüssigkristallinen Mediums der Schaltschicht größer als 3, besonders bevorzugt größer als 7.

In einer weiteren bevorzugten Ausführungsform ist die dielektrische Anisotropie des flüssigkristallinen Mediums der Schaltschicht kleiner als null, besonders bevorzugt kleiner als -2.

Weiterhin bevorzugt hat das flüssigkristalline Medium der Schaltschicht eine optische Anisotropie ($\Delta n$) von 0.01 bis 0.3, besonders bevorzugt von 0.04 bis 0.27.

Weiterhin bevorzugt umfasst das flüssigkristalline Medium der Schaltschicht 3 bis 20 verschiedene flüssigkristalline Verbindungen, bevorzugt 8 bis 18, besonders bevorzugt 12 bis 16 verschiedene flüssigkristalline Verbindungen.

Verbindungen, welche als Bestandteile des flüssigkristallinen Mediums verwendet werden können, sind dem Fachmann bekannt und können beliebig gewählt werden.

Es ist bevorzugt, dass das flüssigkristalline Medium der Schaltschicht mindestens eine Verbindung enthält, welche Strukturelemente basierend auf 1,4-Phenylenen und 1,4-Cyclohexylenen aufweist, die mit einem oder mehreren Fluoratomen oder einer oder mehreren Nitrilgruppen substituiert sind. Besonders bevorzugt ist es, dass das flüssigkristalline Medium der Schaltschicht mindestens eine Verbindung enthält, welche 2, 3 oder 4, besonders bevorzugt 3 oder 4 Strukturelemente basierend auf 1,4-Phenylenen und 1,4-Cyclohexylenen aufweist.

Es ist weiterhin bevorzugt, dass das flüssigkristalline Medium der Schaltschicht einen oder mehrere chirale Dotierstoffe enthält. Bevorzugt liegen in diesem Fall in der Schaltschicht der Vorrichtung die Moleküle des flüssigkristallinen Medium gegeneinander verdrillt vor, besonders bevorzugt wie aus dem TN-Modus von Displays bekannt.

Chirale Dotierstoffe werden in dem flüssigkristallinen Medium der Schaltschicht bevorzugt in einer Gesamtkonzentration von 0.01 bis 3 Gew.-%, besonders bevorzugt von 0.05 bis 1 Gew.-% verwendet. Um hohe Werte für die Verdrillung zu erhalten, kann die Gesamtkonzentration der chiralen Dotierstoffe auch höher als 3 Gew.-% gewählt werden, bevorzugt bis maximal 10 Gew.-%.

Gemäß einer alternativen, ebenfalls bevorzugten Ausführungsform, enthält das flüssigkristalline Medium der Schaltschicht keine chiralen Dotierstoffe. Bevorzugt liegen in diesem Fall in der Schaltschicht der Vorrichtung die Moleküle des flüssigkristallinen Medium nicht gegeneinander verdrillt vor.

Die Anteile dieser Verbindungen und anderer Mindermengenkomponenten werden bei der Angabe der Anteile der flüssigkristallinen Verbindungen und der dichroitischen Farbstoffe vernachlässigt.

Das flüssigkristalline Medium der Schaltschicht enthält weiterhin bevorzugt einen oder mehrere Stabilisatoren. Die Gesamtkonzentration der Stabilisatoren liegt bevorzugt zwischen 0.00001 und 10 Gew.-%, besonders bevorzugt zwischen 0.0001 und 1 Gew.-% der Gesamtmischung. Die Anteile dieser Verbindungen und anderer Mindermengenkomponenten werden bei der Angabe der Anteile der flüssigkristallinen Verbindungen und der dichroitischen Farbstoffe vernachlässigt.

Die erfindungsgemäße Vorrichtung enthält bevorzugt in der Schaltschicht zusätzlich zu den mehreren Verbindungen der Formel (I) oder Formel (II) und dem flüssigkristallinen Medium noch weitere dichroitische Farbstoffe mit anderer Struktur als Formel (I) oder Formel (II). Besonders bevorzugt enthält es einen, zwei, drei oder vier weitere Farbstoffe, ganz besonders bevorzugt zwei oder drei weitere Farbstoffe und am stärksten bevorzugt drei weitere Farbstoffe mit anderer Struktur als Formel (I) oder Formel (II).

Bezüglich der Eigenschaft des Dichroismus gelten die für die Verbindungen der Formel (I) oder Formel (II) beschriebenen bevorzugten Eigenschaften auch für die optionalen weiteren dichroitischen Farbstoffe als bevorzugt.

Die Absorptionsspektren der dichroitischen Farbstoffe der Schaltschicht ergänzen sich bevorzugt derart, dass für das Auge der Eindruck von schwarzer Farbe entsteht. Bevorzugt decken die zwei oder mehr dichroitischen Farbstoffe des erfindungsgemäßen flüssigkristallinen Mediums einen großen Teil des sichtbaren Spektrums ab. Wie genau eine Mischung von Farbstoffen hergestellt werden kann, die für das Auge schwarz bzw. grau erscheint, ist dem Fachmann bekannt und beispielsweise in Manfred Richter, Einführung in die Farbmetrik, 2. Auflage, 1981, ISBN 3-11-008209-8, Verlag Walter de Gruyter & Co., beschrieben.

Die Einstellung des Farbortes einer Mischung aus Farbstoffen wird im Rahmen der Farbmetrik beschrieben. Hierzu werden die Spektren der Einzelfarbstoffe unter Berücksichtigung des Lambert-Beer'schen Gesetzes zu einem Gesamtspektrum berechnet und unter der zugehörigen Beleuchtung, z.B. für Tageslicht die Lichtart D65, gemäß den Regeln der Farbmetrik in die entsprechenden Farborte und Helligkeitswerte umgerechnet. Die Lage des Weißpunktes ist durch die jeweilige Lichtart, z.B. D65 festgelegt und in Tabellen (z.b. obenstehende Literaturstelle) aufgeführt. Durch Änderung der Anteile der verschiedenen Farbstoffe lassen sich verschiedene Farborte einstellen.

Gemäß einer bevorzugten Ausführungsform enthält die Schaltschicht einen oder mehrere dichroitische Farbstoffe, die Licht im roten und NIR-Bereich, d.h. von 600 bis 2000 nm Wellenlänge absorbieren, bevorzugt im Bereich von 650 bis 1800 nm, besonders bevorzugt im Bereich von 650 bis 1300 nm. In einer bevorzugten Ausführung sind diese dichroitischen Farbstoffe gewählt aus Azoverbindungen, Anthrachinonen, Methinverbindungen, Azomethinverbindungen, Merocyaninverbindungen, Naphthochinonen, Tetrazinen, Perylenen, Terrylenen, Quaterrylenen, höheren Rylenen, Pyrromethenen, Azofarbstoffen, Nickeldithiolenen, (Metall-)Phthalocyaninen, (Metall-) Naphthalocyaninen und (Metall- )Porphyrinen. Besonders bevorzugt sind darunter Perylene und Terrylene. Der Anteil aller dichroitischen Farbstoffe in der Mischung der Schaltschicht beträgt bevorzugt insgesamt 0.01 bis 10 Gew.-%, besonders bevorzugt 0.1 bis 7 Gew.-% und ganz besonders bevorzugt 0.2 bis 7 Gew.-%.

Die weiteren dichroitischen Farbstoffe der Schaltschicht mit anderer Struktur als Formel (I) oder Formel (II), sind bevorzugt gewählt aus den in B. Bahadur, Liquid Crystals - Applications and Uses, Vol. 3, 1992, World Scientific

Publishing, Abschnitt 11.2.1 angegebenen Farbstoffklassen und besonders bevorzugt aus den in der dort vorhandenen Tabelle aufgeführten expliziten Verbindungen.

Die genannten Farbstoffe gehören zu den dem Fachmann bekannten Klassen von dichroitischen Farbstoffen, die vielfach in der Literatur beschrieben sind. So sind z.B. Anthrachinonfarbstoffe beschrieben in EP 34832, EP 44893, EP 48583, EP 54217, EP 56492, EP 59036, GB 2065158, GB 2065695, GB 2081736, GB 2082196, GB 2094822, GB 2094825, JP-OS 55-123673, DE 3017877, DE 3040102, DE 3115147, DE 3115762, DE 3150803 und DE 3201120, Naphthochinonfarbstoffe beschrieben in DE 3126108 und DE 3202761, Azofarbstoffe in EP 43904, DE 3123519, WO 82/2054, GB 2079770, JP-OS 56-57850, JP-OS 56-104984, US 4308161, US 4308162, US 4340973, T. Uchida, C. Shishido, H. Seki und M. Wada: Mol. Cryst. Lig. Cryst. 39, 39-52 (1977) und H. Seki, C. Shishido, S. Yasui und T. Uchida: Jpn. J. Appl. Phys. 21, 191-192 (1982), und Perylene beschrieben in EP 60895, EP 68427 und WO 82/1191. Rylen-Farbstoffe sind beispielsweise beschrieben in EP 2166040, US 2011/0042651, EP 68427, EP 47027, EP 60895, DE 3110960 und EP 698649.

Gemäß einer bevorzugten Ausführungsform enthält die Schaltschicht der erfindungsgemäßen Vorrichtung neben Verbindungen der Formel (I) oder Formel (II) ausschließlich dichroitische Farbstoffe gewählt aus Rylen-Farbstoffen.

Beispiele für bevorzugte weitere dichroitische Farbstoffe, die in der Schaltschicht der erfindungsgemäßen Vorrichtung enthalten sein können, sind in der folgenden Tabelle abgebildet:

| Tabelle 2 |
|---|
| 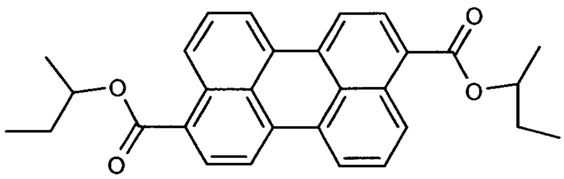 |
| |
| 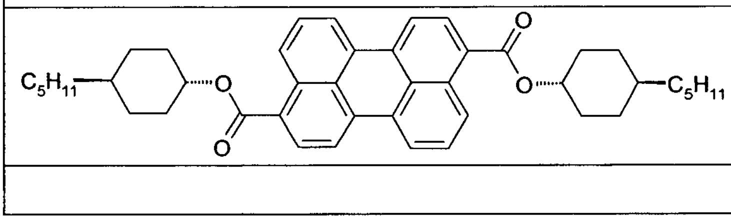 |
| |

| |
|---|
| 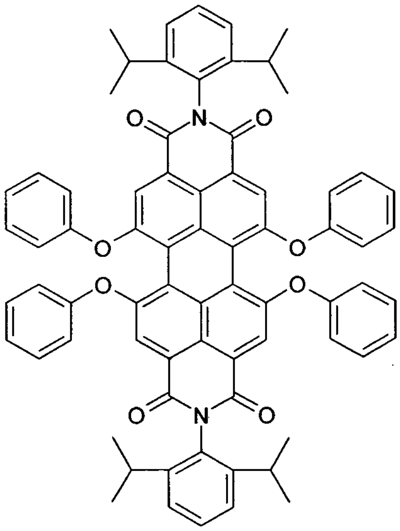 |
| |
| 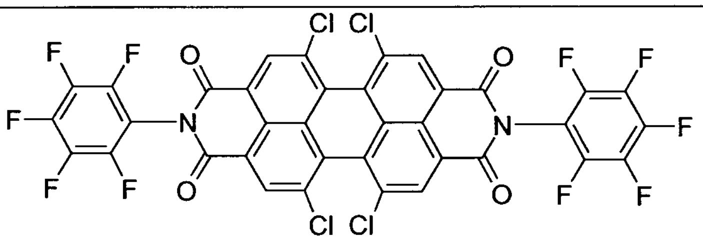 |
| |
| 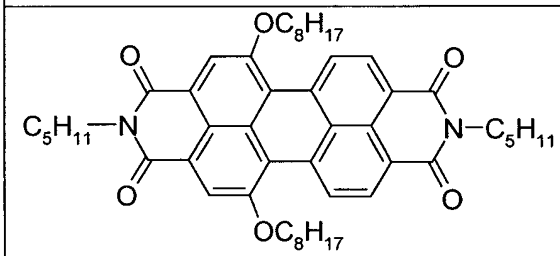 |
| |
| 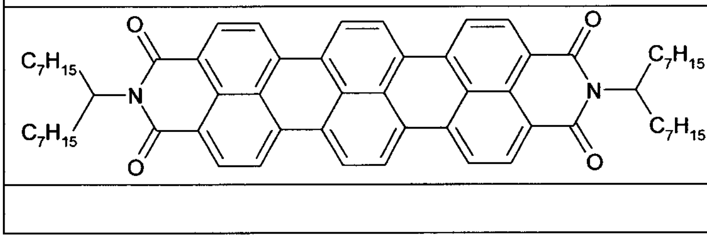 |
| |

| |
|---|
| 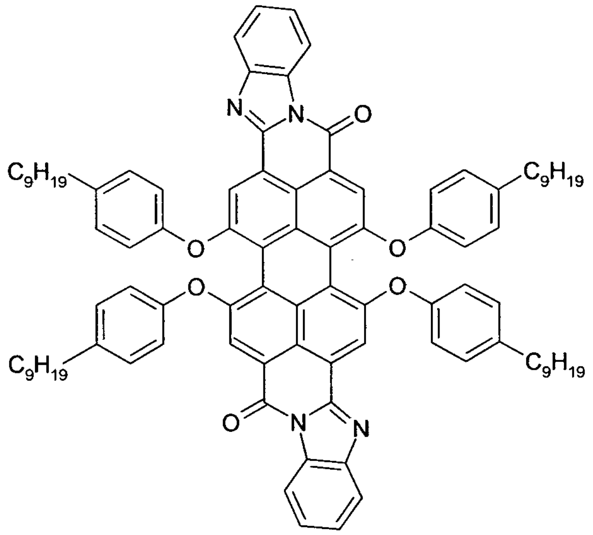 |
| |
| 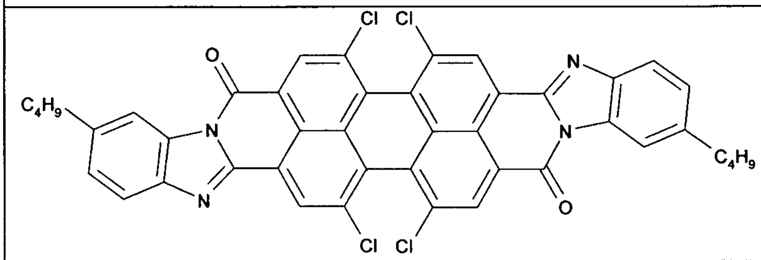 |
| |
| 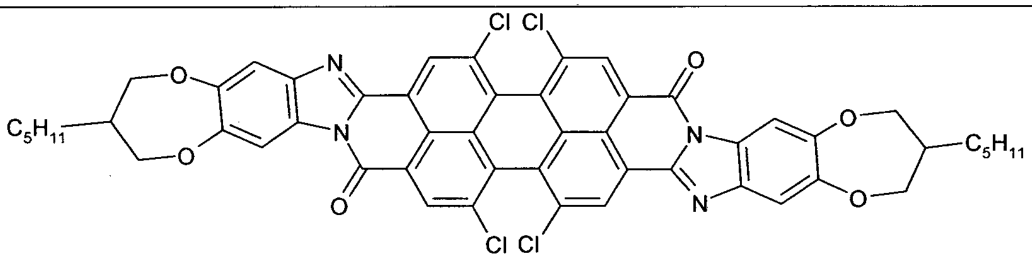 |
| |
| 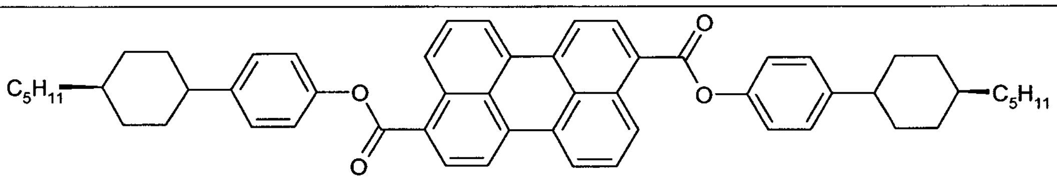 |
| |
| 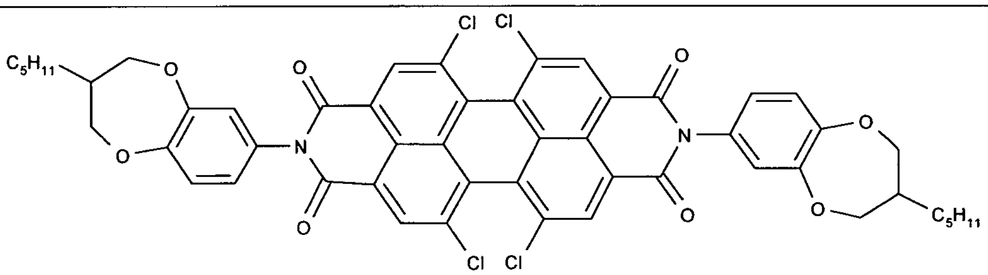 |

| |
|---|
| 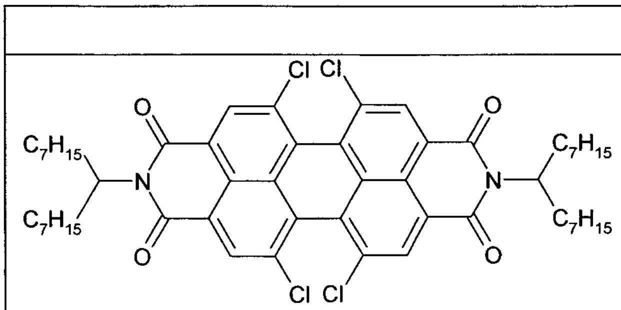 |
| |
| 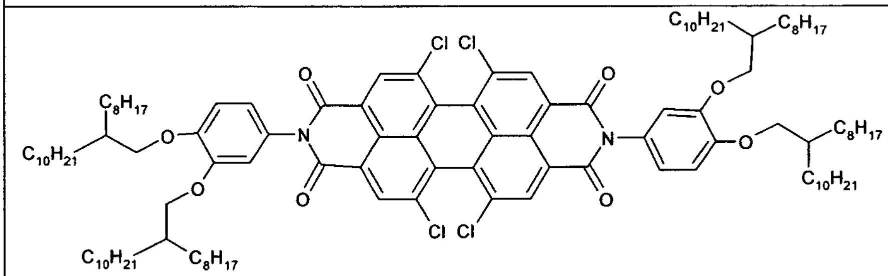 |
| |
| 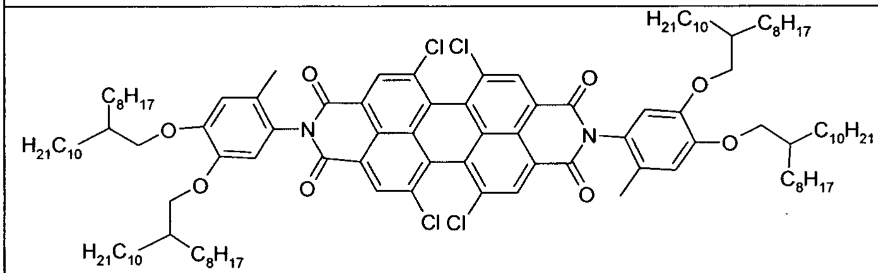 |
| |
| 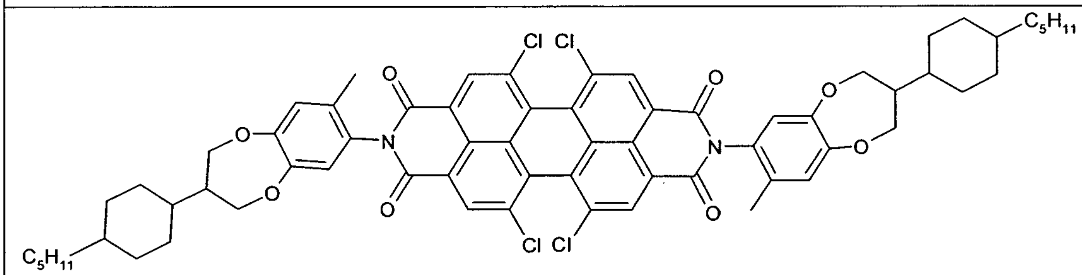 |
| |
| 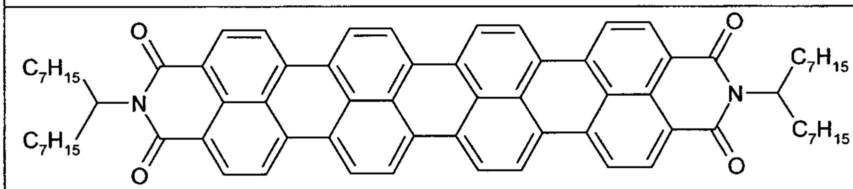 |
| |

| |
|---|
| 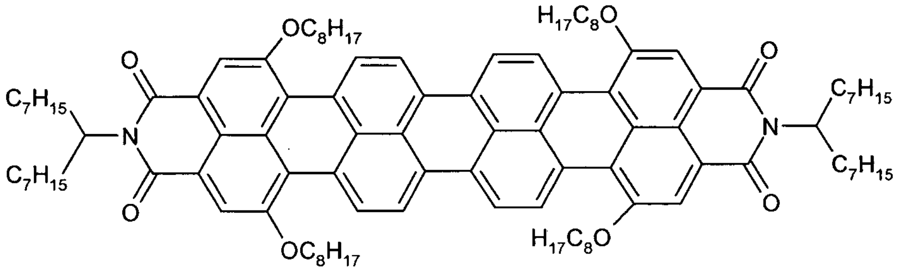 |
| |
| 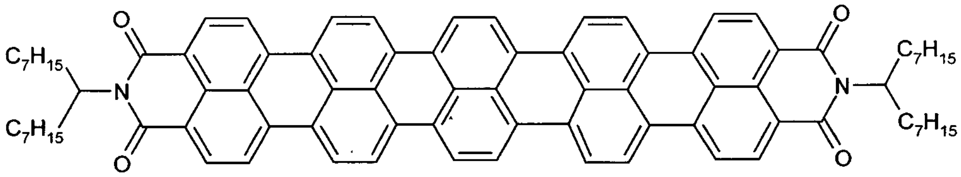 |
| |
| 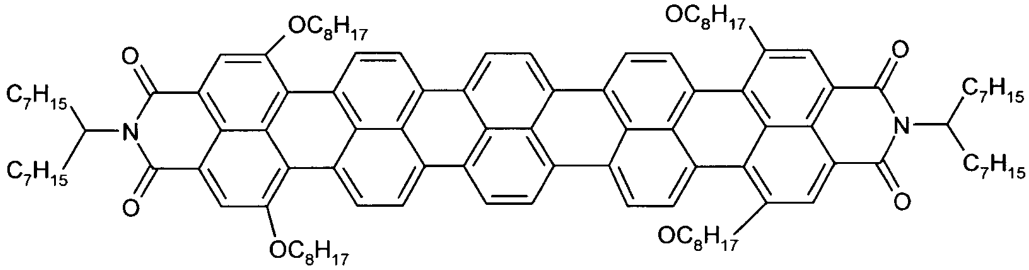 |
| |
| 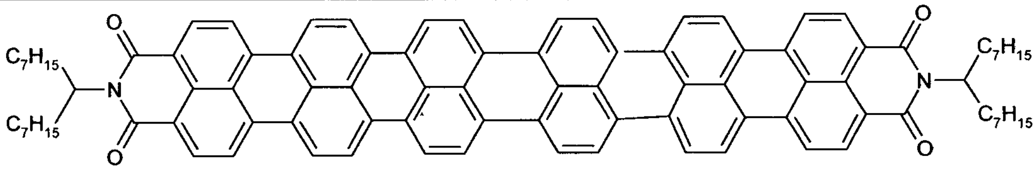 |
| |
| 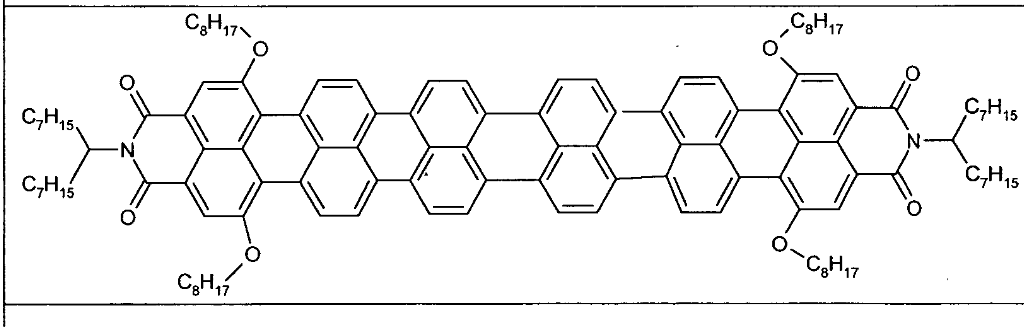 |
| |
| 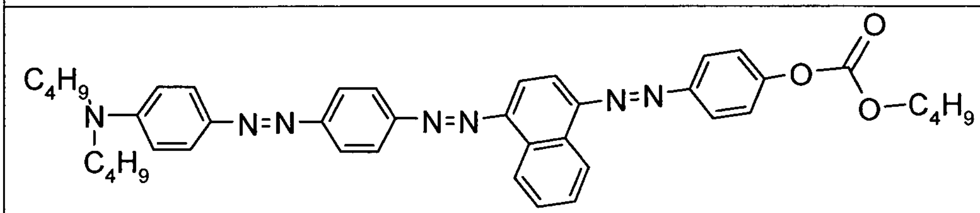 |
| |

| |
|---|
| 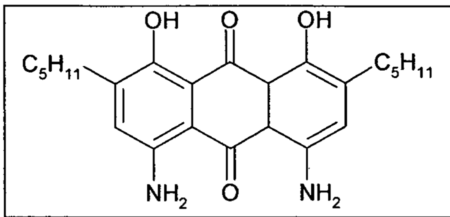 |
| |
| 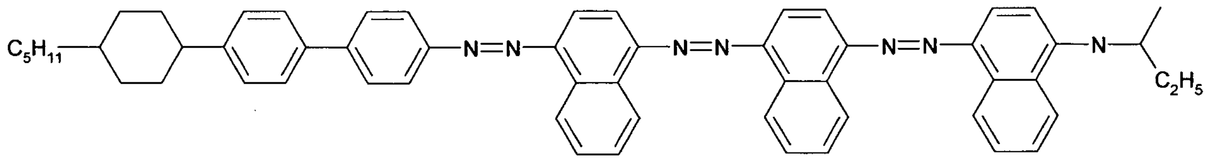 |
| |
| 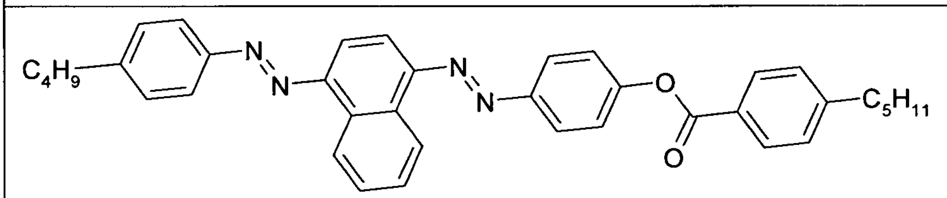 |
| |
| 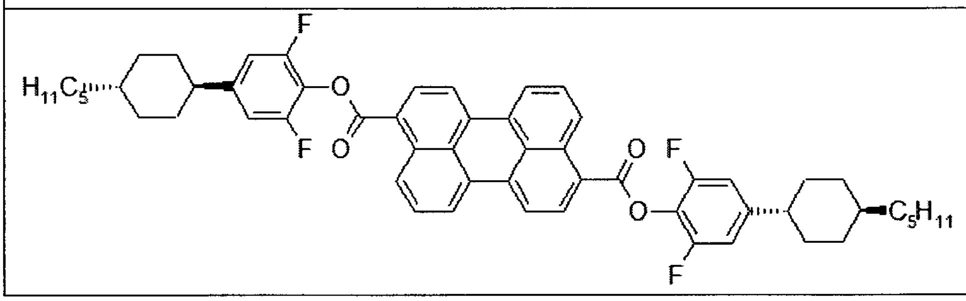 |

In einer bevorzugten Ausführungsform enthält die Schaltschicht der erfindungsgemäßen Vorrichtung eine oder mehrere Quencherverbindungen. Dies ist insbesondere dann bevorzugt, wenn die erfindungsgemäße Vorrichtung in ihrer Schaltschicht einen oder mehrere fluoreszierende Farbstoffe enthält.

Quencherverbindungen sind Verbindungen, welche die Fluoreszenz löschen. Die Quencherverbindungen können in der Schaltschicht die elektronische Anregungsenergie von Nachbarmolekülen, wie z.B. fluoreszierenden Farbstoffen, übernehmen und gehen dabei in einen elektronisch angeregten Zustand über. Der gequenchte fluoreszierende Farbstoff wechselt dadurch in den elektronischen Grundzustand und wird so an der Fluoreszenz oder einer Folgereaktion gehindert. Die Quencherverbindung selbst kehrt durch strahlungslose Desaktivierung oder durch Abgabe von Licht in den Grundzustand zurück und steht zum erneuten Quenchen wieder zur Verfügung.

In der Schaltschicht der erfindungsgemäßen Vorrichtung können der Quencherverbindung unterschiedliche Funktionen zukommen. Zum einen kann die Quencherverbindung zur Verlängerung der Lebensdauer eines Farbstoffsystems, durch Deaktivierung elektronischer Anregungsenergie, beitragen. Zum anderen eliminiert die Quencherverbindung ästhetisch ggfs. unerwünschte zusätzliche Farbeffekte, z.B. farbiges Abstrahlen in den Innenraum, die von den fluoreszierenden Farbstoffen in der Schaltschicht ausgehen.

Um ein effektives Quenchen zu erzielen, ist die Quencherverbindung dem jeweiligen Farbstoffsystem, insbesondere dem längstwelligst absorbierenden Farbstoff einer Farbstoffkombination, anzupassen. Wie dies zu tun ist, ist dem Fachmann bekannt.

Bevorzugte Quencherverbindungen sind beispielweise in Tabelle 8.1 auf Seite 279 in Joseph R. Lakowicz, Principles of Fluorescence Spectroscopy, 3rd Edition, 2010, ISBN 10: 0-387-31278-1, Verlag Springer Science+Business Media LLC, beschrieben. Weitere Molekülklassen sind beispielsweise unter den Stichworten Dark-Quencher oder Black Hole Quencher dem Fachmann geläufig. Beispiele sind Azofarbstoffe und Aminoanthrachinone. In der Schaltschicht der erfindungsgemäßen Vorrichtung können als Quencherverbindungen auch nicht-fluoreszierende oder nur im NIR fluoreszierende Farbstoffe zum Einsatz kommen.

In einer bevorzugten Ausführungsform der erfindungsgemäßen Schaltschicht sind gegebenenfalls enthaltene

Quencherverbindungen so gewählt, dass die Fluoreszenz im sichtbaren Teil des Spektrums unterdrückt wird.

Die erfindungsgemäße Vorrichtung ist zur Regulierung des Energie-Durchtritts in Form von von der Sonne ausgehendem Licht von der Umgebung in einen Innenraum geeignet. Dabei erfolgt der zu regulierende Energie-Durchtritt von der Umwelt (dem Außenraum) in einen Innenraum.

Der Innenraum kann dabei ein beliebiger weitgehend von der Umgebung abgeschlossener Raum sein, beispielsweise ein Gebäude, ein Fahrzeug, oder ein Container.

Erfindungsgemäß ist daher weiterhin die Verwendung der Vorrichtung zur Regulierung des Energie-Durchtritts von einem Außenraum in einen Innenraum.

Die Vorrichtung kann jedoch auch zur ästhetischen Raumgestaltung eingesetzt werden, beispielsweise für Licht- und Farbeffekte. Beispielsweise können Tür- und Wandelemente enthaltend die erfindungsgemäße Vorrichtung in grau oder in Farbe nach transparent geschaltet werden. Desweiteren kann die Vorrichtung auch eine weiße oder farbige flächige Hinterleuchtung , die in der Helligkeit moduliert wird, oder eine gelbe flächige Hinterleuchtung, die mit einer blauen Gast-Wirtsanzeige in ihrer Farbe moduliert wird, umfassen. Es können eine oder beide Glasseiten der erfindungsgemäßen Vorrichtung mit angerauhtem oder strukturiertem Glas versehen sein zur Lichtauskopplung und/oder zur Lichteffekterzeugung.

In einer nochmals alternativen Verwendung wird die Vorrichtung zur Regulierung des Lichteinfalls auf die Augen eingesetzt, beispielsweise in Schutzbrillen, Visieren oder Sonnenbrillen, wobei die Vorrichtung in einem Schaltzustand den Lichteinfall auf die Augen gering hält, und in einem anderen Schaltzustand den Lichteinfall weniger stark verringert.

Die erfindungsgemäße Vorrichtung ist bevorzugt in einer Öffnung einer größeren flächigen Struktur angeordnet, wobei die flächige Struktur selbst nicht oder nur geringfügig Energie-Durchtritt zulässt, und wobei die Öffnung relativ gesehen energiedurchlässiger ist. Bevorzugt ist die flächige Struktur eine Wand oder eine sonstige Begrenzung eines Innenraums nach außen. Weiterhin bevorzugt bedeckt die flächige Struktur eine mindestens genauso große Fläche, besonders bevorzugt eine mindestens doppelt so große Fäche wie die Öffnung in ihr, in der die erfindungsgemäße Vorrichtung angeordnet ist.

Bevorzugt ist die Vorrichtung dadurch gekennzeichnet, dass sie eine Flächenausdehnung von mindestens 0.05 $m^2$ aufweist, bevorzugt mindestens 0.1 $m^2$, besonders bevorzugt mindestens 0.5 $m^2$ und ganz besonders bevorzugt mindestens 0.8 $m^2$.

Die Vorrichtung ist bevorzugt in einer wie oben beschriebenen, relativ gesehen energiedurchlässigeren Öffnung eines Gebäudes, eines Containers, eines Fahrzeugs oder eines sonstigen weitgehend geschlossenen Raums aufgebracht. Die Vorrichtung kann allgemein für beliebige Innenräume verwendet werden, besonders wenn diese nur begrenzten Luftaustausch mit der Umgebung aufweisen und lichtdurchlässige Begrenzungsflächen aufweisen, durch die EnergieEintrag von außen in Form von Lichtenergie stattfinden kann. Besonders relevant ist die Verwendung der Vorrichtung für Innenräume, welche starker Sonneneinstrahlung durch lichtdurchlässige Flächen, beispielsweise durch Fensterflächen, ausgesetzt sind.

Die erfindungsgemäße Vorrichtung ist schaltbar. Unter Schaltung wird dabei eine Änderung des Energie-Durchtritts durch die Vorrichtung verstanden. Die erfindungsgemäße Vorrichtung ist bevorzugt elektrisch schaltbar, wie beispielsweise in WO 2009/141295 und in der Anmeldung EP 12008320.9 beschrieben wird.

Sie kann aber auch thermisch schaltbar sein, wie beispielsweise in WO 2010/118422 beschrieben. In diesem Fall erfolgt das Schalten bevorzugt durch einen Übergang von einem nematischen zu einem isotropen Zustand durch Änderung der Temperatur der Schaltschicht enthaltend die Verbindungen der Formel (I) oder Formel (II) und ein flüssigkristallines Medium. Im nematischen Zustand liegen die Moleküle des flüssigkristallinen Mediums geordnet vor und damit auch die Verbindungen der Formel (I) oder Formel (II), beispielsweise ausgerichtet parallel zur Oberfläche der Vorrichtung durch die Wirkung einer Orientierungsschicht. Im isotropen Zustand liegen die Moleküle ungeordnet vor, und damit auch die Verbindungen der Formel (I) oder Formel (II). Der Unterschied zwischen geordnetem und ungeordnetem Vorliegen der dichroitischen Verbindungen der Formel (I) oder Formel (II) bewirkt einen Unterschied in der Lichtdurchlässigkeit der Schaltschicht der erfindungsgemäßen Vorrichtung, gemäß dem Prinzip, dass dichroitische Verbindungen je nach Orientierung in Bezug auf die Schwingungsebene des Lichts einen höheren oder einen niedrigeren Absorptionskoeffizienten aufweisen.

Ist die Vorrichtung elektrisch schaltbar, umfasst sie bevorzugt zwei oder mehr Elektroden, die zu beiden Seiten der Schaltschicht angebracht sind. Die Elektroden bestehen bevorzugt aus ITO oder aus einer dünnen, bevorzugt transparenten Metall- und/oder Metalloxidschicht, beispielsweise aus Silber oder FTO (Fluor-dotiertes Zinnoxid) oder einem alternativen, dem Fachmann für diese Verwendung bekannten Material. Die Elektroden sind bevorzugt mit elektrischen Anschlüssen versehen. Die Spannung wird bevorzugt durch eine Batterie, einen Akkumulator, oder durch externe Stromversorgung bereitgestellt.

Der Schaltvorgang erfolgt im Fall von elektrischer Schaltung durch eine Ausrichtung der Moleküle des flüssigkristallinen Mediums durch das Anlegen von Spannung.

In einer bevorzugten Ausführungsform wird dabei die Vorrichtung von einem Zustand mit hoher Absorption, d. h. geringer Lichtdurchlässigkeit, der ohne Spannung vorliegt, in einen Zustand mit geringerer Absorption, d. h. höherer

Lichtdurchlässigkeit, überführt. Bevorzugt ist das flüssigkristalline Medium der Schaltschicht in beiden Zuständen nematisch. Der spannungslose Zustand ist bevorzugt dadurch gekennzeichnet, dass die Moleküle des flüssigkristallinen Mediums, und damit die Moleküle der Verbindungen der Formel (I) oder Formel (II), parallel zur Ebene der Schaltschicht ausgerichtet vorliegen. Dies wird bevorzugt durch eine entsprechend gewählte Orientierungsschicht erreicht. Der Zustand unter Spannung ist bevorzugt dadurch gekennzeichnet, dass die Moleküle des flüssigkristallinen Mediums, und damit die Moleküle der Verbindungen der Formel (I) oder Formel (II), senkrecht zur Ebene der Schaltschicht vorliegen.

In einer zur oben genannten Ausführungsform alternativen Ausführungsform wird die Vorrichtung von einem Zustand mit geringer Absorption, d. h. hoher Lichtdurchlässigkeit, der ohne Spannung vorliegt, in einen Zustand mit höherer Absorption, d. h. geringerer Lichtdurchlässigkeit, überführt. Bevorzugt ist das flüssigkristalline Medium der Schaltschicht in beiden Zuständen nematisch. Der spannungslose Zustand ist bevorzugt dadurch gekennzeichnet, dass die Moleküle des flüssigkristallinen Mediums der Schaltschicht, und damit die Moleküle der Verbindungen der Formel (I) oder Formel (II), senkrecht zur Ebene der Schaltschicht ausgerichtet vorliegen. Dies wird bevorzugt durch eine entsprechend gewählte Orientierungsschicht erreicht. Der Zustand unter Spannung ist bevorzugt dadurch gekennzeichnet, dass die Moleküle des flüssigkristallinen Mediums der Schaltschicht, und damit die Moleküle der Verbindungen der Formel (I) oder Formel (II), parallel zur Ebene der Schaltschicht vorliegen.

Gemäß einer bevorzugten Ausführungsform der Erfindung kann die Vorrichtung ohne externe Stromversorgung betrieben werden, indem die nötige Energie durch eine Solarzelle oder eine sonstige Vorrichtung zur Umwandlung von Licht und/oder Wärmeenergie in elektrische Energie bereitgestellt wird, die mit der Vorrichtung verbunden ist. Die Bereitstellung der Energie durch die Solarzelle kann direkt oder indirekt, d. h. über eine dazwischengeschaltete Batterie oder Akkumulator oder sonstige Einheit zur Speicherung von Energie, erfolgen. Bevorzugt ist die Solarzelle außen an der Vorrichtung angebracht oder sie ist innerer Bestandteil der Vorrichtung, wie beispielsweise in WO 2009/141295 offenbart. Bevorzugt sind dabei insbesondere Solarzellen, welche bei diffusem Licht besonders effizient sind, sowie transparente Solarzellen.

Die erfindungsgemäße Vorrichtung weist bevorzugt die folgende Schichtenabfolge auf, wobei zusätzlich weitere Schichten vorhanden sein können. Bevorzugt grenzen die im Folgenden angegebenen Schichten in der Vorrichtung direkt aneinander.

- Substratschicht, bevorzugt aus Glas oder Polymer
- elektrisch leitfähige transparente Schicht, bevorzugt aus ITO
- Orientierungsschicht
- Schaltschicht, enthaltend mehrere Verbindungen der Formel (I) oder Formel (II)
- Orientierungsschicht
- elektrisch leitfähige transparente Schicht, bevorzugt aus ITO
- Substratschicht, bevorzugt aus Glas oder Polymer

Die bevorzugten Ausführungsformen der einzelnen Schichten werden im Folgenden dargestellt.

Bevorzugt umfasst die erfindungsgemäße Vorrichtung eine oder mehrere, besonders bevorzugt zwei Orientierungsschichten. Die Orientierungsschichten liegen bevorzugt direkt angrenzend an die beiden Seiten der Schaltschicht enthaltend die Verbindungen der Formel (I) oder Formel (II) vor.

Als Orientierungsschichten der erfindungsgemäßen Vorrichtung können beliebige dem Fachmann hierzu bekannte Schichten verwendet werden. Bevorzugt sind Polyimid-Schichten, besonders bevorzugt Schichten aus geriebenem Polyimid. Auf bestimmte, dem Fachmann bekannte Art geriebenes Polyimid führt zu einer Ausrichtung der Moleküle des flüssigkristallinen Mediums in Reiberichtung, wenn die Moleküle parallel zur Orientierungsschicht vorliegen (planare Ausrichtung). Dabei ist es bevorzugt, dass die Moleküle des flüssigkristallinen Mediums nicht völlig planar auf der Orientierungsschicht vorliegen, sondern einen leichten Aufstellwinkel aufweisen (pretilt). Zum Erreichen von vertikaler Ausrichtung der Verbindungen des flüssigkristallinen Mediums zur Oberfläche der Orientierungsschicht (homeotrope Ausrichtung) wird bevorzugt auf bestimmte Art behandeltes Polyimid als Material für die Orientierungsschicht eingesetzt (Polyimid für sehr hohe Pretiltwinkel). Weiterhin können Polymere, die durch einen Belichtungsvorgang mit polarisiertem Licht erhalten wurden, als Orientierungsschicht zum Erreichen einer Ausrichtung der Verbindungen des flüssigkristallinen Mediums gemäß einer Orientierungsachse verwendet werden (photoalignment).

Weiterhin bevorzugt ist in der erfindungsgemäßen Vorrichtung die Schaltschicht zwischen zwei Substratschichten angeordnet bzw. von diesen eingeschlossen. Die Substratschichten können beispielsweise aus Glas oder einem Polymer, bevorzugt einem lichtdurchlässigen Polymer, bestehen.

Bevorzugt ist die Vorrichtung dadurch gekennzeichnet, dass sie keinen Polarisator auf Polymerbasis, besonders bevorzugt keinen in fester Materiephase vorliegenden Polarisator und ganz besonders bevorzugt überhaupt keinen Polarisator umfasst.

Die Vorrichtung kann jedoch gemäß einer alternativen Ausführungsform auch einen oder mehrere Polarisatoren aufweisen. Bevorzugt sind die Polarisatoren in diesem Fall Linearpolarisatoren.

Wenn genau ein Polarisator vorhanden ist, so ist dessen Absorptionsrichtung bevorzugt senkrecht stehend zur Orientierungsachse der Verbindungen des flüssigkristallinen Mediums der erfindungsgemäßen Vorrichtung auf derjenigen Seite der Schaltschicht, auf der sich der Polarisator befindet.

In der erfindungsgemäßen Vorrichtung können sowohl absorptive als auch reflektive Polarisatoren eingesetzt werden. Bevorzugt werden Polarisatoren verwendet, die als dünne optische Filme vorliegen. Beispiele für reflektive Polarisatoren, die in der erfindungsgemäßen Vorrichtung verwendet werden können, sind DRPF- (diffusive reflective polariser film, 3M), DBEF- (dual brightness enhanced film, 3M), DBR- (layered-polymer distributed Bragg reflectors, wie in US 7,038,745 und US 6,099,758 beschrieben) und APF-Filme (advanced polariser film, 3M, vgl. Technical Digest SID 2006, 45.1, US 2011/0043732 und US 7,023,602). Weiterhin können Polarisatoren basierend auf Drahtgittern (WGP, wire-gridpolarisers), welche Infrarotlicht reflektieren, eingesetzt werden. Beispiele für absorptive Polarisatoren, welche in den erfindungsgemäßen Vorrichtungen eingesetzt werden können, sind der Itos XP38-Polarisatorfilm und der Nitto Denko GU-1220DUN-Polarisatorfilm. Ein Beispiel für einen circularen Polarisator, welcher erfindungsgemäß verwendet werden kann, ist der Polarisator APNCP37-035-STD (American Polarizers). Ein weiteres Beispiel ist der Polarisator CP42 (ITOS).

Weiterhin bevorzugt enthält erfindungsgemäße Vorrichtung ein Lichtleitsystem, das Licht zu einer Solarzelle oder einer sonstigen Vorrichtung zur Umwandlung von Licht und/oder Wärmeenergie in elektrische Energie leitet, bevorzugt wie in WO 2009/141295 beschrieben. Das Lichtleitsystem sammelt und konzentriert Licht, das auf die Vorrichtung trifft. Bevorzugt sammelt und konzentriert es Licht, das von fluoreszierenden dichroitischen Farbstoffen in der Schaltschicht emittiert wird. Das Lichtleitsystem steht mit einer Vorrichtung zur Umwandlung von Lichtenergie in elektrische Energie, bevorzugt einer Solarzelle, in Kontakt, so dass das gesammelte Licht konzentriert auf diese trifft. In einer bevorzugten Ausführungsform der Erfindung ist die Vorrichtung zur Umwandlung von Lichtenergie in elektrische Energie am Rand der Vorrichtung angebracht, in diese integriert und elektrisch mit Mitteln zur elektrischen Schaltung der erfindungsgemäßen Vorrichtung verbunden.

In einer bevorzugten Ausführungsform ist die erfindungsgemäße Vorrichtung Bestandteil eines Fensters, besonders bevorzugt eines Fensters enthaltend mindestens eine Glasfläche, ganz besonders bevorzugt eines Fensters, welche Mehrscheiben-Isolierglas enthält.

Unter Fenster wird dabei insbesondere eine Struktur in einem Gebäude verstanden, welche einen Rahmen und mindestens eine von diesem Rahmen umfasste Glasscheibe enthält. Bevorzugt enthält sie einen wärmeisolierenden Rahmen und zwei oder mehr Glasscheiben (Mehrscheiben-Isolierglas).

Gemäß einer bevorzugten Ausführungsform ist die erfindungsgemäße Vorrichtung direkt auf einer Glasfläche eines Fensters aufgebracht, besonders bevorzugt im Zwischenraum zwischen zwei Glasscheiben von Mehrscheiben-Isolierglas.

Ein Fenster enthaltend eine erfindungsgemäße Vorrichtung, bevorzugt mit den oben angegebenen bevorzugten Merkmalen, ist weiterer Gegenstand der Erfindung.

## Ausführungsbeispiele

Die folgenden Beispiele sollen die vorliegende Erfindung verdeutlichen und sind nicht beschränkend auszulegen.

In der vorliegenden Anmeldung werden Strukturen von flüssigkristallinen Verbindungen durch Abkürzungen wiedergegeben (Akronyme). Diese Abkürzungen sind in WO 2012/052100 explizit vorgestellt und erklärt (S. 63 - 89).

Alle physikalischen Eigenschaften werden nach "Merck Liquid Crystals, Physical Properties of Liquid Crystals", Status Nov. 1997, Merck KGaA, Deutschland bestimmt und gelten für eine Temperatur von 20°C. Der Wert von $\Delta n$ wird bei 589 nm bestimmt und der Wert von $\Delta\varepsilon$ wird bei 1 kHz bestimmt, sofern nicht jeweils explizit anders angegeben. $n_e$ und $n_o$ sind jeweils die Brechungsindices des außerordentlichen und des ordentlichen Lichtstrahls unter den oben angegebenen Bedingungen.

Der Anisotropiegrad R wird ermittelt aus dem Wert für den Extinktionskoeffizienten E(p) (Extinktionskoeffizient der Mischung bei paralleler Ausrichtung der Moleküle zur Polarisationsrichtung des Lichts) und dem Wert für den Extinktionskoeffizienten der Mischung E(s) (Extinktionskoeffizient der Mischung bei senkrechter Ausrichtung der Moleküle zur Polarisationsrichtung des Lichts), jeweils bei der Wellenlänge des Maximums der Absorptionsbande des betreffenden Farbstoffs. Hat der Farbstoff mehrere Absorptionsbanden, so wird die intensivste Absorptionsbande ausgewählt. Die Ausrichtung der Moleküle der Mischung wird durch eine Orientierungsschicht erreicht, wie sie dem Fachmann auf dem Gebiet der LC-Displaytechnik bekannt ist. Zur Eliminierung von Einflüssen durch flüssigkristallines Medium, sonstigen Absorptionen und/oder Reflexionen wird jeweils gegen eine identisch vorliegende Mischung, die keinen Farbstoff enthält, gemessen und der erhaltene Wert subtrahiert.

Die Messung erfolgt mit linear polarisiertem Licht, dessen Schwingungsrichtung entweder parallel zur Orientierungsrichtung ist (Bestimmung von E(p)) oder senkrecht zur Orientierungsrichtung ist (Bestimmung von E(s)). Dies kann durch einen Linearpolarisator erreicht werden, wobei der Polarisator gegen die Vorrichtung gedreht wird, um die

beiden unterschiedlichen Schwingungsrichtungen zu realisieren. Die Messung von E(p) und E(s) erfolgt also über die Drehung der Schwingungsrichtung des eingestrahlten polarisierten Lichts.

Der Anisotropiegrad R wird aus den erhaltenen Werten für E(s) und E(p) nach der Formel

$$R=[E(p)-E(s)] / [E(p) + 2*E(s)]$$

berechnet, wie unter anderem in "Polarized Light in Optics and Spectroscopy", D. S. Kliger et al., Academic Press, 1990, angegeben. Eine detaillierte Beschreibung des Verfahrens zur Bestimmung des Anisotropiegrads von flüssigkristallinen Medien enthaltend einen dichroitischen Farbstoff findet sich auch in B. Bahadur, Liquid Crystals - Applications and Uses, Vol. 3, 1992, World Scientific Publishing, Abschnitt 11.4.2.

**A) Herstellung der Farbstoffe**

A-1) Synthese der Referenzverbindung BT-1

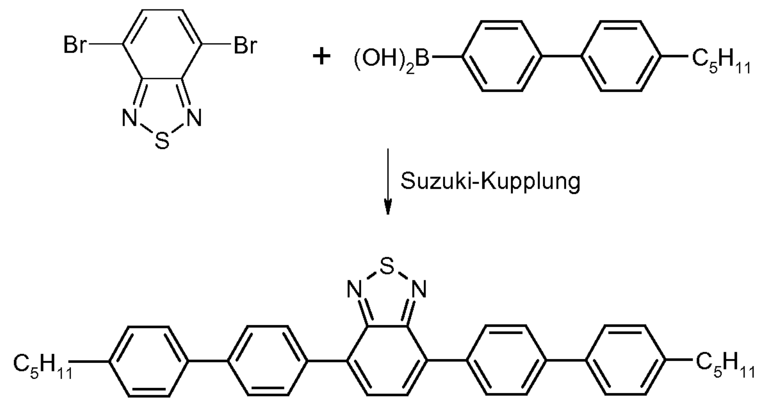


**BT-1**

Das Dibromid (5,0 mmol) und die Boronsäure (10.0 mmol) werden unter Stickstoff in 40 mL Toluol vorgelegt. Anschließend werden 20.1 mL einer 2.0 mol/L Lösung von Natriumcarbonat zugegeben. Anschließend wird Tris(dibenzyliden-aceton)dipalladium (0.05 mmol) und Tris-(o)-tolylphosphin (0.2 mmol) zugegeben, und die Mischung wird über Nacht unter Rückfluss gerührt. Anschließend lässt man den Ansatz auf 40 °C abkühlen und trennt die wäßrige Phase ab. Die wäßrige Phase wird mit warmem Toluol extrahiert. Die vereinigten organischen Phasen werden aufkonzentriert und mit Toluol über Kieselgel eluiert. Anschließend wird aus Isopropanol/Toluol 1:4 umkristallisiert. Dabei wird das Produkt in einer Ausbeute von 54% d.Th. erhalten. Die Identität des Produkts wird durch Massenspektroskopie (m/z = 580) bestätigt.

A-2) Synthese der Referenzverbindung BT-2

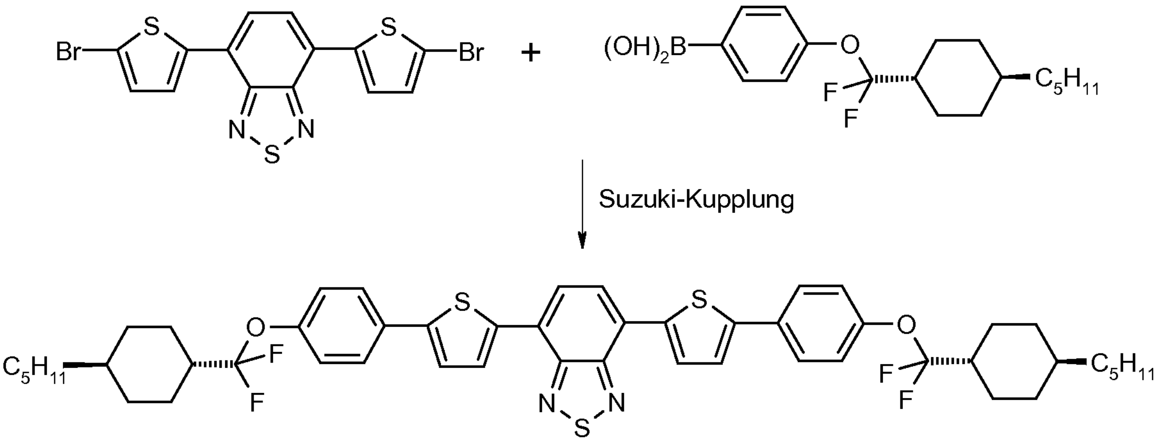


**BT-2**

Zur Synthese der Verbindung wird wie bei A-1) angegeben vorgegangen, mit dem Unterschied, dass lediglich 1.5 h unter Rückfluss erhitzt wird, bevor die Mischung aufgearbeitet wird. Die Umkristallisation erfolgt aus Toluol. Das Produkt wird als Pulver in einer Ausbeute von 29.5% d. Th. erhalten, die Reinheit laut HPLC ist 100 %. Die Identität des Produkts wird durch Massenspektroskopie bestätigt (m/z = 888).

A-3) Synthese der Referenzverbindung BT-3

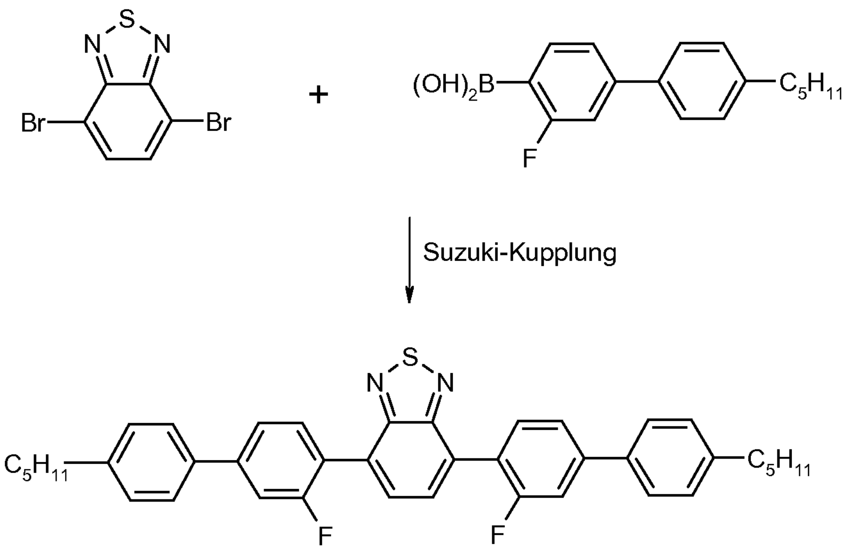


**BT-3**

Zur Synthese der Verbindung wird wie bei A-1) angegeben vorgegangen. Die Umkristallisation erfolgt aus Isopropanol/Toluol 1:1. Das Produkt wird als Kristalle in einer Ausbeute von 45.1 % d. Th. erhalten, die Reinheit laut HPLC ist 99.4 %. Die Identität des Produkts wird durch Massenspektroskopie bestätigt (m/z = 616).

A-4) Herstellung von Verbindung BT-4

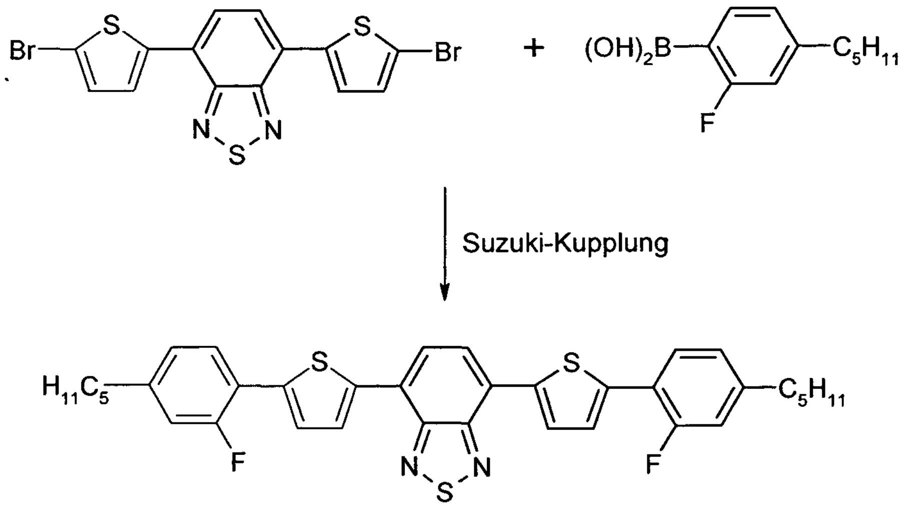


**BT-4**

Zur Synthese der Verbindung wird wie bei A-1) angegeben vorgegangen, mit dem Unterschied, dass 0.3 Äq. Aliquat 336 zusammen mit der Carbonatlösung zugegeben werden und aus Toluol umkristallisiert wird. Das Produkt wird in einer Ausbeute von 68.5 % d. Th. erhalten, die Reinheit laut HPLC ist 99.6 %. Die Identität des Produkts wird durch Massenspektroskopie bestätigt (m/z = 628).

A-5) Herstellung von Verbindung BT-5

Schritt 1 und 2:

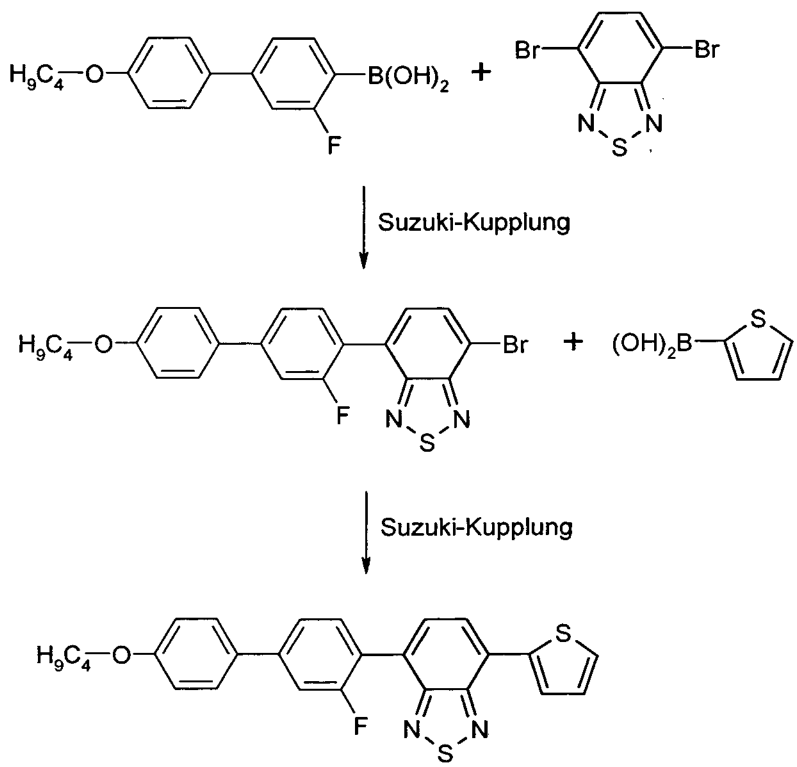

Schritt 3 und 4:

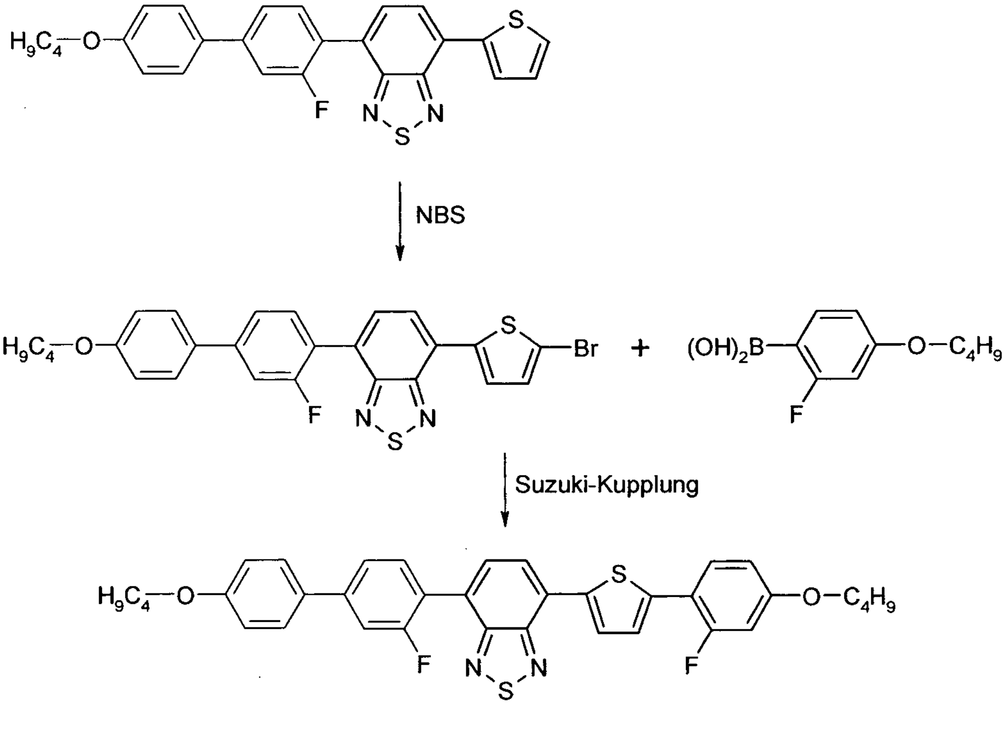


**BT-5**

Schritt 1:

Das Dibromid (33.8 mmol) und die Boronsäure (33.8 mmol) werden unter Stickstoff in 40 mL Toluol vorgelegt. Anschließend werden 67.7 mL einer 2.0 mol/L Lösung von Natriumcarbonat zugegeben. Anschließend wird Tris(dibenzyliden-aceton)dipalladium (0.34 mmol) und Tris-(o)-tolylphosphin (1.35 mmol) zugegeben, und die Mischung wird über Nacht unter Rückfluss gerührt. Anschließend lässt man den Ansatz abkühlen und trennt die wäßrige Phase ab. Die wäßrige Phase wird mit warmem Toluol extrahiert. Die vereinigten organischen Phasen werden zur Trockne eingeengt. Es wird mit 1-Chlorbutan über Kieselgel eluiert. Dann wird das Produkt aus Chlorbutan umkristallisiert. Das Produkt wird in einer Ausbeute von 38.7 % d. Th. und Reinheit von 99.8 % (HPLC) erhalten. Die Identität des Produkts wird durch Massenspektroskopie (m/z = 620) bestätigt.

Schritt 2:

Es wird Natrium-Metaborat-Tetrahydrat (45.8 mmol) in 30 mL Wasser vorgelegt. Dann werden Bis-Triphenylphosphin-palladium(II)chlorid (0.5 mmol), Hydraziniumhydroxid (0.89 mmol) und die Boronsäure (14.4 mmol) und THF (80 mL) zugegeben und 5 min gerührt. Dann wird das Bromid (13.1 mmol) zugegeben und 3 h unter Rückfluss erhitzt. Anschließend wird die Mischung mit Wasser und Methyl-tert-Butyl-Ether versetzt und wäßrig aufgearbeitet. Nach Einengen der organischen Phasen zur Trockne wird das Produkt in einer Ausbeute von 90.9 % d. Th. und einer Reinheit von 97.9 % (HPLC) erhalten. Die Identität des Produkts wird durch Massenspektroskopie (m/z = 460) bestätigt.

Schritt 3:

Das Thiophen-Derivat (11.9 mmol) wird in 60 mL 1,2-Dichlorbenzol unter $N_2$ gelöst. Es wird N-Bromsuccinimid (12.5 mmol) unter Rühren zugegeben, und es wird auf 70 °C erhitzt. Anschließend lässt man 4 h bei dieser Temperatur rühren. Es wird abkühlen gelassen, dann wird mit 0.5 M NaOH-Lösung versetzt und 30 min gerührt. Der ausgefallene Feststoff wird isoliert und getrocknet. Das Produkt wird in einer Ausbeute von 87.8 % d. Th. und Reinheit von 97.4 % (HPLC) erhalten.

Schritt 4:

Das Bromid (4.5 mmol) und die Boronsäure (4.9 mmol) werden unter Stickstoff in 50 mL Toluol vorgelegt. Anschließend werden 9.0 mL einer 2.0 mol/L Lösung von Natriumcarbonat zugegeben. Anschließend wird Tris(dibenzyliden-aceton)dipalladium (0.045 mmol) und Tris-(o)-tolylphosphin (0.18 mmol) zugegeben, und die Mischung wird über Nacht unter Rückfluss gerührt. Anschließend lässt man den Ansatz auf 40 °C abkühlen und trennt die wäßrige Phase ab. Die wäßrige Phase wird mit warmem Toluol extrahiert. Die vereinigten organischen Phasen werden aufkonzentriert und mit Chlorbutan über Kieselgel eluiert. Anschließend wird aus Chlorbutan umkristallisiert. Dabei wird das Produkt in einer Ausbeute von 61% d. Th. erhalten.

A-6) Herstellung von Verbindungen BT-6 bis BT-17

Die Verbindungen BT-6 bis BT-17 können entsprechend hergestellt werden. Ihre Strukturen sind in der folgenden Tabelle 3 gezeigt.

A-7) Herstellung von Referenzverbindung BT-18

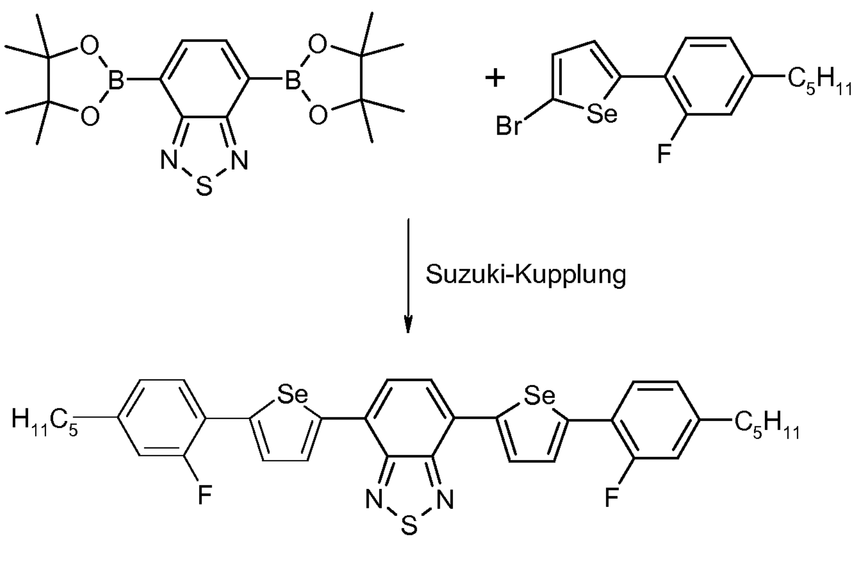


**BT-18**

Der Diboronester (11,9 mmol) wurde in 150 ml Toluol unter Stickstoff vorgelegt und auf 60° erwärmt. Dazu wurde das entsprechende Bromid (29,7 mmol) gelöst in 50 ml Toluol auf ebenfalls 60° erwärmt und zugegeben. Nun wurde die zuvor auf 40° erwärmte 2 molare Natriumcarbonatlösung (94,8 mmol) rasch zugegeben. 0,95 mmol Tris(dibenzyliden-aceton-)dipalladium und 0,24 mmol Tris-(o)-tolylphoshin wurde danach hinzugefügt, rasch auf Rückflußtemperatur gebracht. Über Nacht wurde am Rückfluß gekocht. Es wurde auf 40° abgekühlt, die wässrige Phase abgetrennnt, die org.Phase 1x mit gesättigter NaCl-Lösung gewaschen. Der org.Extrakt wurde nun aufkonzentriert und mit einem Gemisch aus Toluol/Heptan (1:1) über Kieselgel chromatographiert. Anschließend wurden die gesammelten Fraktionen zum Rückstand eingeengt und mehrmals aus Toluol umkristallisiert.
Dabei konnten 379 mg Produkt (4,2% der Theorie) isoliert werden. Die Identität wurde durch Massenspektroskopie (M:722,65) und NMR bestätigt.

A-8) Herstellung von Verbindung BT-19

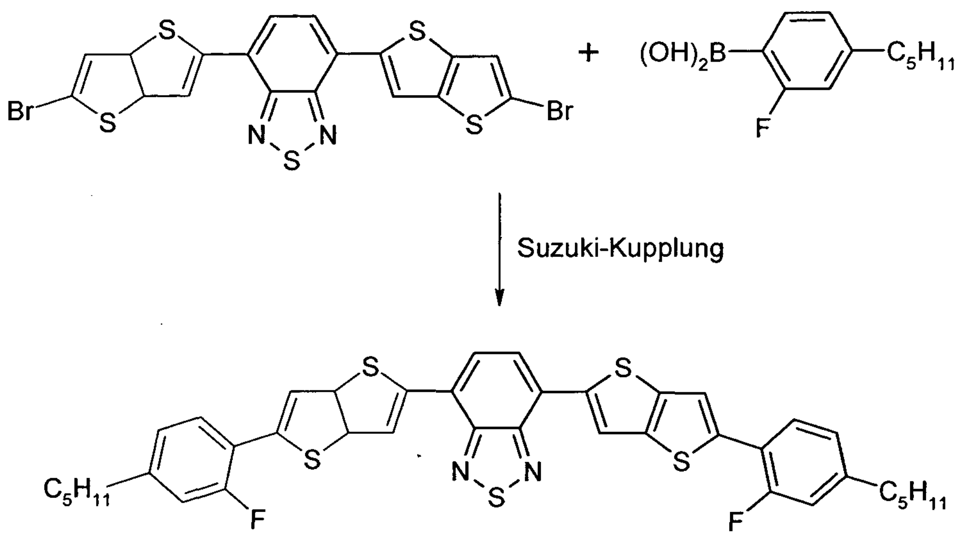


**BT-19**

Zur Synthese der Verbindung BT-19 wird wie bei A-1) angegeben vorgegangen, Die Identität des Produkts wird durch Massenspektroskopie bestätigt (m/z = 741).

A-9) Herstellung von Verbindung BT-20

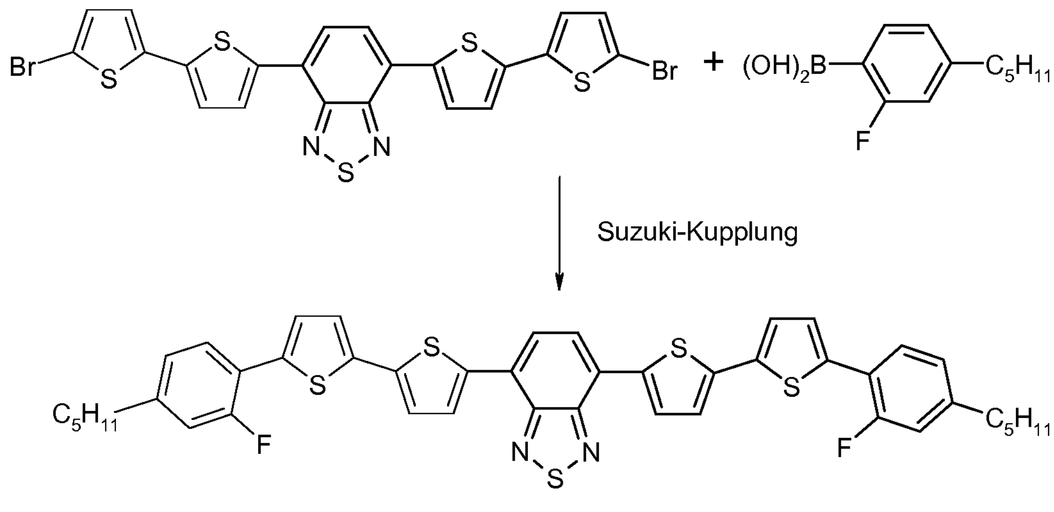


**BT-20**

Zur Synthese der Verbindung BT-20 wird wie bei A-1) angegeben vorgegangen, Die Identität des Produkts wird durch Massenspektroskopie bestätigt (m/z = 793).

A-10) Herstellung von Referenzverbindung BT-21

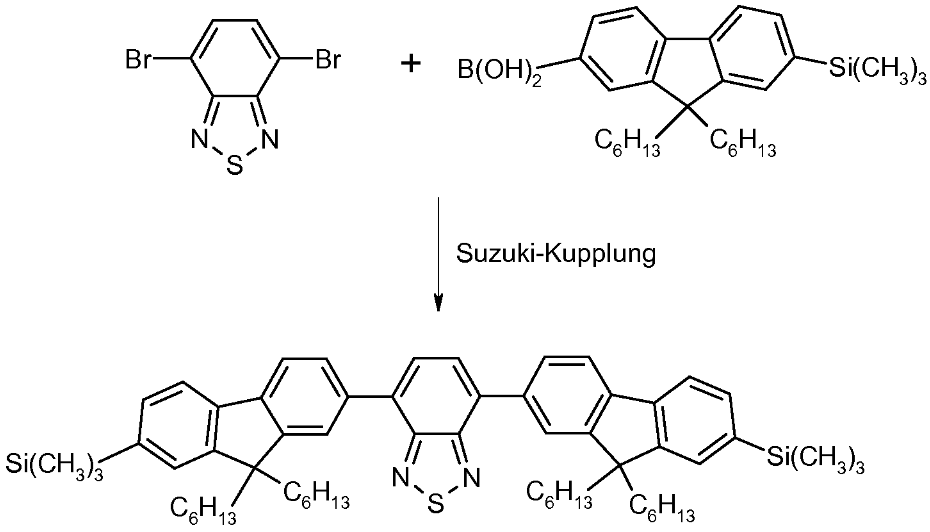


**BT-21**

Zur Synthese der Verbindung BT-21 wird wie bei A-1) angegeben vorgegangen, Die Identität des Produkts wird durch Massenspektroskopie bestätigt (m/z = 945).

A-11) Herstellung von Referenzverbindung BT-22

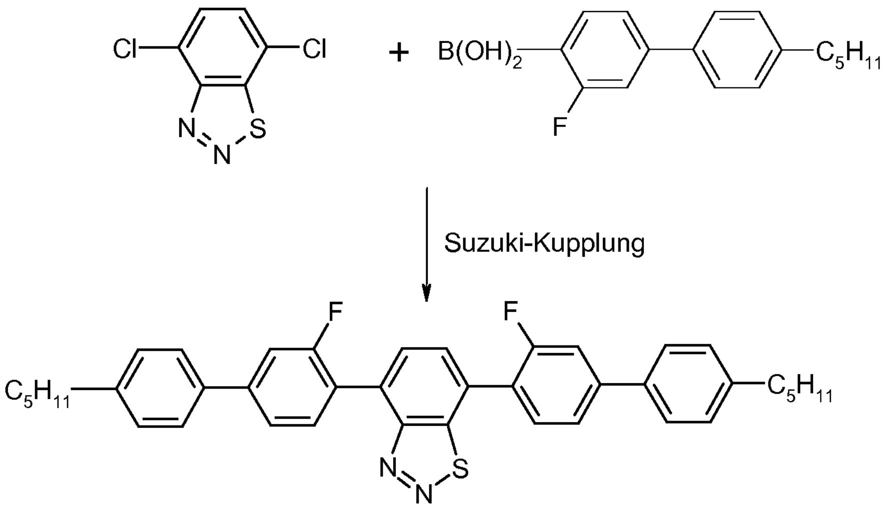


**BT-22**

Das Dichlorid (5,0 mmol) und die Boronsäure (10.0 mmol) werden unter Stickstoff in 40 mL Toluol vorgelegt. Anschließend werden 20.1 mL einer 2.0 mol/L Lösung von Natrium-tert-butylat zugegeben. Anschließend wird Tris(dibenzyliden-aceton)dipalladium (0.05 mmol) und Tricyclohexylphosphin (0.2 mmol) zugegeben, und die Mischung wird über Nacht unter Rückfluss gerührt. Anschließend lässt man den Ansatz auf 40 °C abkühlen und arbeitet wäßrig auf. Die vereinigten organischen Phasen werden aufkonzentriert und mit Toluol über Kieselgel eluiert. Anschließend wird aus Isopropanol/Toluol 1:4 umkristallisiert. Dabei wird das Produkt in einer Ausbeute von 4,5% d.Th. erhalten. Die Identität des Produkts wird durch Massenspektroskopie (m/z = 616) bestätigt.

| Tabelle 3 | |
|---|---|
| BT-6 (Ref.) | 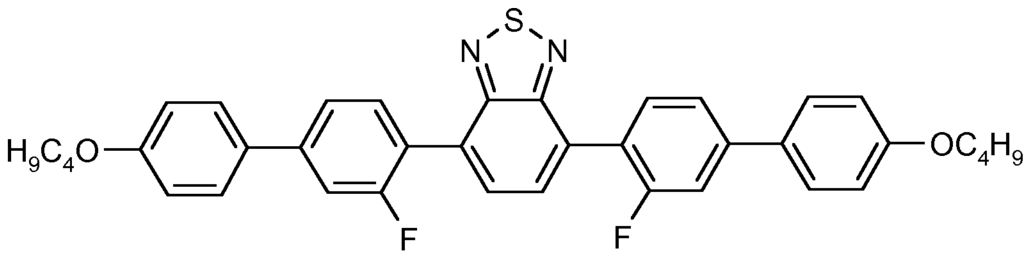 |
| BT-7 | 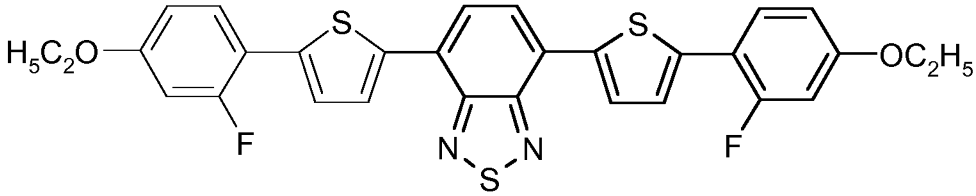 |
| BT-8 | 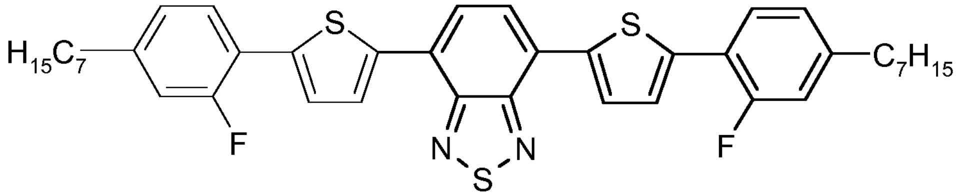 |
| BT-9 | 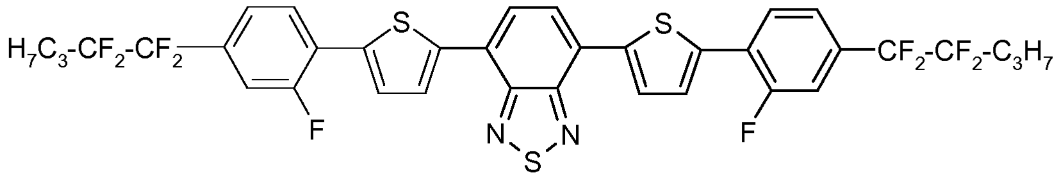 |
| BT-10 | 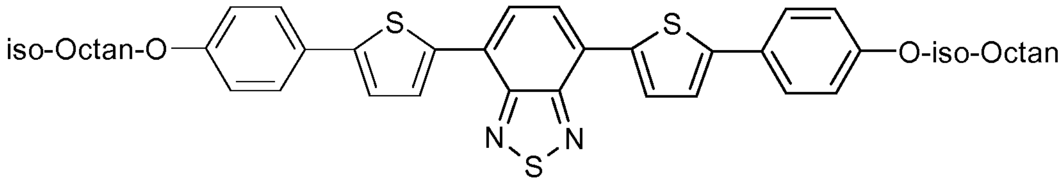 |
| BT-11 | 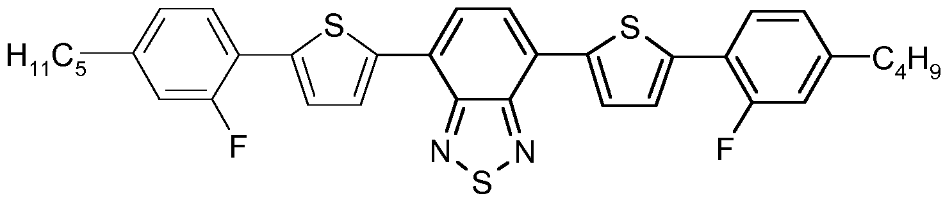 |
| BT-12 | 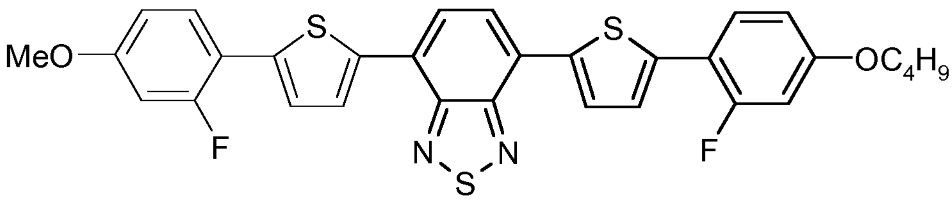 |

(fortgesetzt)

| Tabelle 3 | |
|---|---|
| BT-13 | 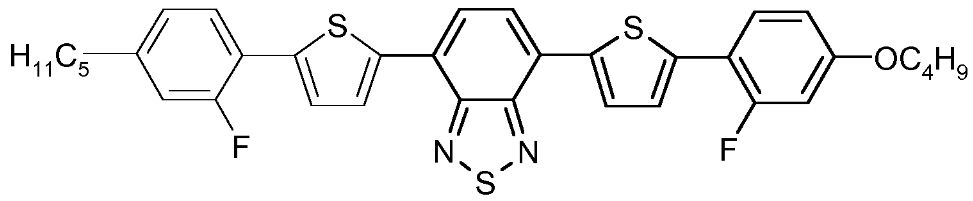 |
| BT-14 | 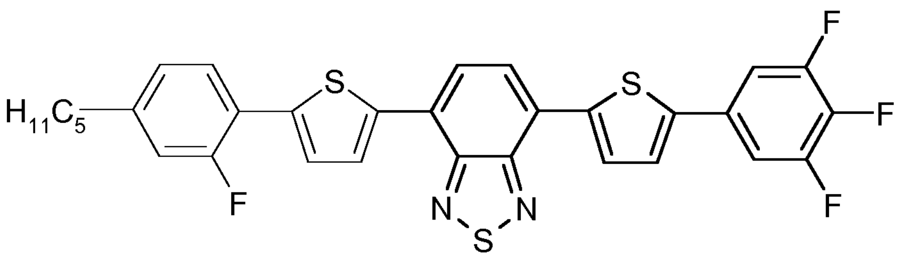 |
| BT-15 | 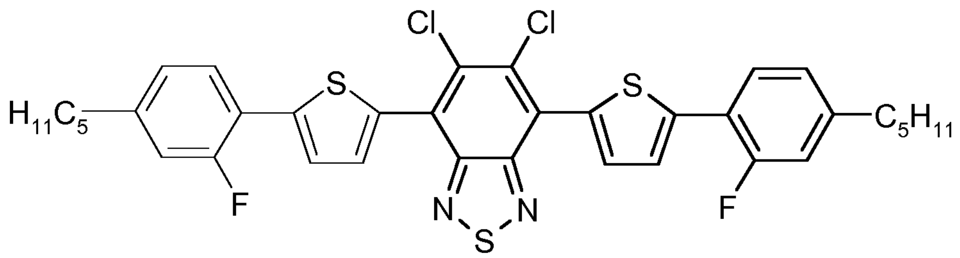 |
| BT-16 | 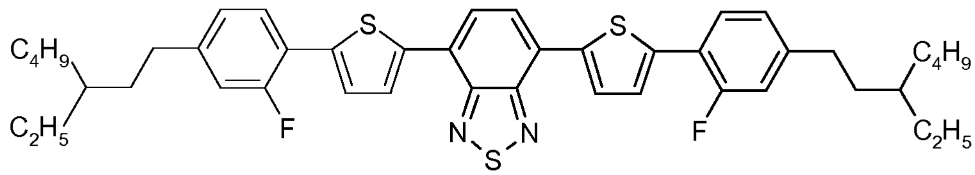 |
| BT-17 | 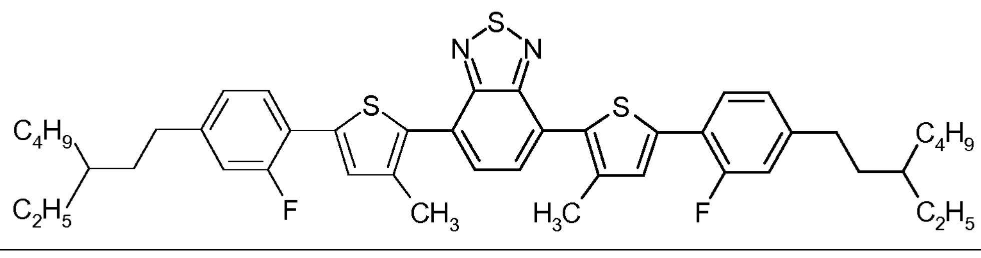 |
| BT-18 (Ref.) | 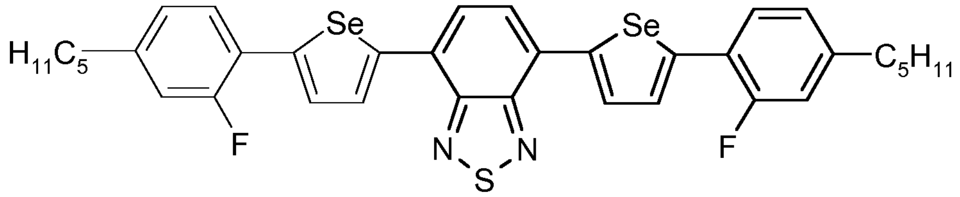 |
| BT-19 | 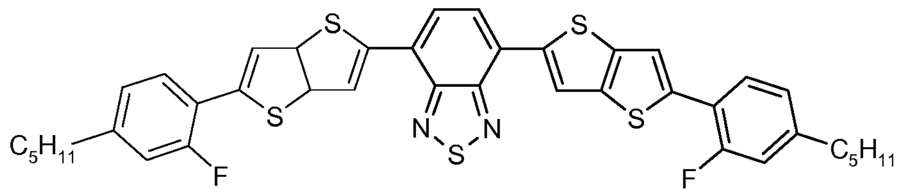 |

(fortgesetzt)

| Tabelle 3 | |
|---|---|
| BT-20 | 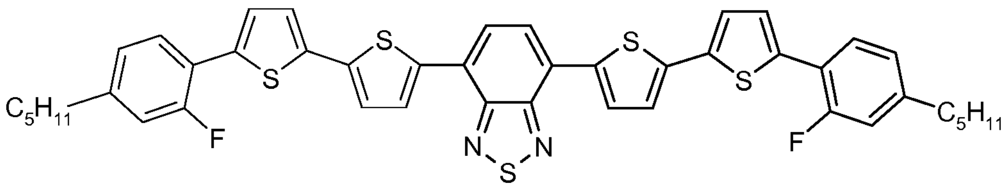 |
| BT-21 (Ref.) | 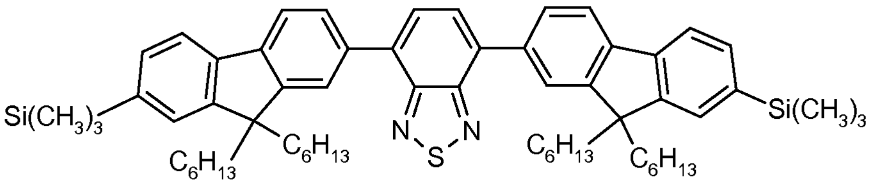 |
| BT-22 (Ref.) | 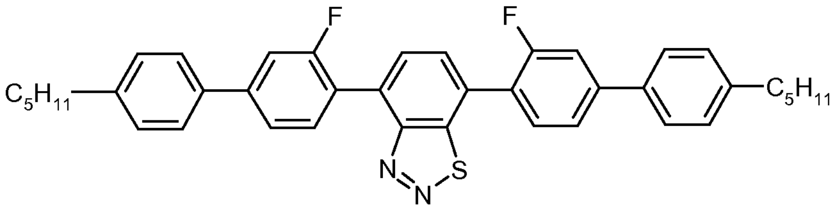 |

**B) Bestimmung der Eigenschaften der Farbstoffe**

Die hergestellten Farbstoffe werden auf ihre physikalischen Eigenschaften hin untersucht, um ihre Eignung zur Verwendung in Vorrichtungen zur Regulierung der Energie-Transmission festzustellen. Zum Vergleich werden die entsprechenden Eigenschaften für die Verbindung D-3 (Struktur siehe unten) angegeben.

Die Lichtbeständigkeit wird wie folgt bestimmt: Es wird ein UV-VIS-Spektrum in einer versiegelten Messzelle zu Beginn der Belastung registriert. Dann wird die Probe belastet, indem sie intensivem Licht (Ausschluss von UV-Licht durch 400 nm cut-off Filter) in einem Suntest CPS+ der Firma MTS-Atlas ausgesetzt wird. In regelmäßigen Abständen wird ein erneutes Spektrum aufgenommen. Die Zeit, nach der der Extinktionswert der langwelligsten und vorzugsweise intensivsten Absorption auf unter 80% der Ausgangswertes abgesunken ist, wird zur Kennzeichnung der Lichtbeständigkeit angegeben.

Als Stokes-Shift wird die Differenz zwischen der Wellenlänge des kurzwelligsten Emissionsmaximums und der Wellenlänge des langwelligsten Absorptionsmaximums angegeben.

Tabelle 4

| Name | Absorptions -Maximum / nm | Anisotropiegrad R | Stokes-Shift / nm | Löslichkeit in Gew.-% in M-1 | Lichtbeständigkeit |
|---|---|---|---|---|---|
| BT-1 | 413 | 0.80 | n. best. | >0.50 | 2 Wochen |
| BT-2 | 502 | 0.74 | 115 nm | 0.30 | 32 Wochen |
| BT-3 | 390 | 0.80 | n. best. | >0.50 | 1 Woche |
| BT-4 | 505 | 0.71 | 125 | 0.75 | 39 Wochen |
| BT-5 | 459 | 0.77 | n. best. | 3.90 | 12 Wochen |
| BT-6 | 398 | 0.81 | n. best. | < 0.25 | < 1 Woche |
| BT-7 | 517 | 0.73 | n. best. | 0.30 | 22 Wochen |
| BT-8 | 507 | 0.71 | n. best. | 0.40 | 27 Wochen |
| BT-9 | 491 | 0.73 | n. best. | 0.30 | 9 Wochen |
| BT-10 | 524 | 0.72 | n. best. | 0.30 | 18 Wochen |

(fortgesetzt)

| Tabelle 4 | | | | | |
|---|---|---|---|---|---|
| Name | Absorptions -Maximum / nm | Anisotropiegrad R | Stokes-Shift / nm | Löslichkeit in Gew.-% in M-1 | Lichtbeständigkeit |
| BT-11 | 505 | 0.73 | 120 | 0.25 | 21 Wochen |
| BT-12 | 515 | 0.74 | 125 | 0.25 | 25 Wochen |
| BT-13 | 510 | 0.76 | 125 | 1.50 | 17 Wochen |
| BT-14 | 495 | 0.69 | 120 | 1.60 | 13 Wochen |
| BT-15 | 475 | 0.74 | n. best. | 3.80 | 8 Wochen |
| BT-16 | 505 | 0.72 | n. best. | n. best. | n. best. |
| BT-17 | 463 | 0.67 | n. best. | n. best. | n. best. |
| BT-18 | 525 | 0.77 | n. best. | n. best. | n. best. |
| D-3 | 590 | 0.68 | 50 | 0.50 | > 15 Wochen |

Die Messungen zeigen, dass die erfindungsgemäßen Benzothiadiazol-Verbindungen hervorragende Eigenschaften in Bezug auf Anisotropiegrad, Löslichkeit in flüssigkristallinen Medien und Stokes-Shift aufweisen. Desweiteren weisen die erfindungsgemäßen Verbindungen, mit mindestens einem Schwefel enthaltenen Heterozyklus direkt an den Benzothiadiazolrest gebunden, eine besonders hohe Lichtbeständigkeit auf. Weiterhin haben sie abhängig vom Substitutionsmuster unterschiedliche Absorptionsfarben, so dass eine Mischung enthaltend zwei oder mehr der erfindungsgemäßen Farbstoffe bereits einen großen Teil des sichtbaren Spektrums abdeckt. Wird ein als weiterer Farbstoff ein rot absorbierender Farbstoff hinzugefügt, kann eine schwarze Mischung erhalten werden.

Der hohe Stokes-Shift der Verbindungen macht es möglich, eine hohe Fluoreszenzausbeute zu erreichen, da nur geringe Re-Absorption der Fluoreszenz stattfindet. Dies ist ein großer Vorteil bei Verwendung der Verbindungen in Vorrichtungen, bei denen die Fluoreszenzstrahlung zur Gewinnung von Energie (autonome Vorrichtungen zur Regulierung der Energietransmission in Fenstern mit Solarzellen, vgl. WO 2009/141295) genutzt wird.

**C) Herstellung von flüssigkristallinen Medien enthaltend die Farbstoffe**

C-1) Herstellung der Mischung LC-1

Es werden die folgenden Farbstoffe in den angegebenen Anteilen zur Grundmischung M-1 (s. u.) hinzugegeben und eine Lösung hergestellt:

| Tabelle 5 | |
|---|---|
| Farbstoff | Anteil |
| BT-4 | 0.20 Gew.-% |
| D-1 | 0.49 Gew.-% |
| D-2 | 0.35 Gew.-% |
| D-3 | 0.35 Gew.-% |

Zusammensetzung der Grundmischung M-1:

| Tabelle 6 | |
|---|---|
| | M-1 |
| Klärpunkt | 114.5 °C |

(fortgesetzt)

| Tabelle 6 | | |
|---|---|---|
| | M-1 | |
| Delta-n | 0.1342 | |
| $n_e$ | 1.6293 | |
| $n_o$ | 1.4951 | |
| Zusammensetzung | Verbindung | Gew.-% |
| | CPG-3-F | 5 |
| | CPG-5-F | 5 |
| | CPU-3-F | 15 |
| | CPU-5-F | 15 |
| | CP-3-N | 16 |
| | CP-5-N | 16 |
| | CCGU-3-F | 7 |
| | CGPC-3-3 | 4 |
| | CGPC-5-3 | 4 |
| | CGPC-5-5 | 4 |
| | CCZPC-3-3 | 3 |
| | CCZPC-3-4 | 3 |
| | CCZPC-3-5 | 3 |

Strukturen der weiteren verwendeten Farbstoffe:

| Tabelle 7 |
|---|
| 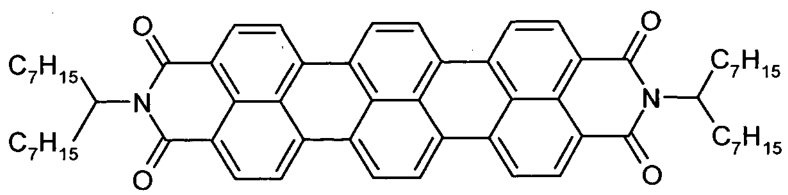 |
| D-1 |
| 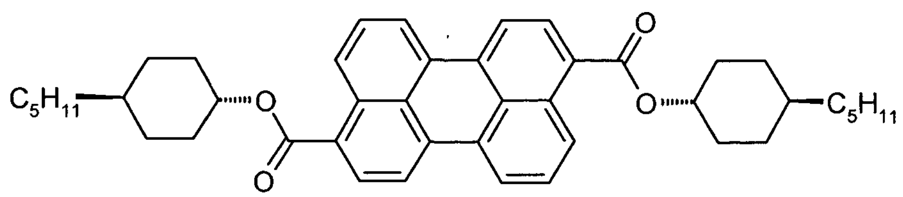 |
| D-2 |

(fortgesetzt)

| Tabelle 7 |
| --- |
| 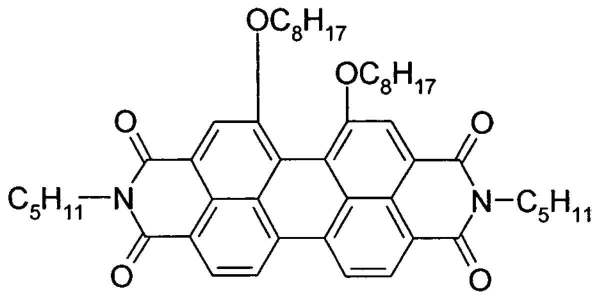 |
| D-3 |

**D) Verwendung von flüssigkristallinen Medien enthaltend die Farbstoffe in Vorrichtungen zur Regulierung des Energie-Durchtritts**

Um die Vorrichtung herzustellen, wird die Flüssigkristallmischung enthaltend die Farbstoffe (LC-1) in den Zwischenraum der folgenden Schichtenanordnung gefüllt:

- Substratschicht
- ITO-Schicht
- Polyimid-Orientierungsschicht
- mit Spacern offengehaltener Zwischenraum
- Polyimid-Orientierungsschicht
- ITO-Schicht
- Substratschicht

Die Flüssigkristallschicht ist in dieser Anordnung planar mit antiparallelem Pretiltwinkel orientiert. Diese Orientierung wird durch zwei antiparallel zueinander geriebene Polyimidschichten erreicht. Die Dicke der flüssigkristallinen Schicht wird durch Spacer definiert und beträgt üblicherweise 25 $\mu$m.

Es werden Werte für den Lichttransmissionsgrad $\tau_V$ jeweils für den dunklen und den hellen Schaltzustand der Vorrichtung ermittelt und im Folgenden aufgeführt. Der helle Schaltzustand wird durch Anlegen einer Spannung erreicht, während der dunkle Schaltzustand ohne Spannung vorliegt. Weiterhin wird der Farbort der Vorrichtung (in CIE-Koordinaten) im Dunkel- und im Hellzustand ermittelt.

Die Messung erfolgt mit der Vorrichtung enthaltend das flüssigkristalline Medium mit Farbstoffen im Messstrahl und einer baugleichen Vorrichtung, entsprechend ohne die Farbstoffe im Referenzstrahl. Hierdurch werden Reflexions- und Absorptionsverluste der Zelle eliminiert.

Der Wert $\tau_V$ und die CIE-Koordinaten (x,y) sind wie folgt definiert:

$\tau_V$= Lichttransmissionsgrad, bestimmt nach DIN EN410

Der Farbort (für weiß, grau schwarz) der hier zugrundegelegten Normlichtart D65 liegt bei x=0.3127 und y=0.3290 (Manfred Richter, Einführung in die Farbmetrik, zweite Auflage 1991, ISBN 3-11-008209-8). Die angegebenen Farborte (x,y) beziehen sich alle auf die Normlichtart D65 und den 2° Normalbeobachter nach CIE 1931.

Erhaltene Messwerte für die Vorrichtung:

- Dunkelzustand: x=0.312; y= 0.324; $\tau_V$ =33%

- Hellzustand: x=0.319; y=0.336; $\tau_V$ =60%

Im Beispiel zeigt sich eine gute Stabilität des flüssigkristallinen Mediums und eine genügende Löslichkeit der Farbstoffe im flüssigkristallinen Medium.

Weiterhin zeigt das Beispiel, dass sich die Vorrichtung durch Anlegen einer Spannung von einem Dunkelzustand mit deutlich geringerer Lichttransmission zu einem Hellzustand mit deutlich erhöhter Lichttransmission schalten lässt.

**E) Herstellung von Testvorrichtungen und Bestimmung der relativen Fluoreszenz aus Wellenleitung**

Zur Messung der relativen Fluoreszenz aus Wellenleitung wird eine Mischung des jeweiligen Farbstoffs in der flüssigkristallinen Grundmischung M-1 hergestellt. Dabei wird die Konzentration in allen Fällen so gewählt, dass eine Transmission von 35% durch die Mischung erhalten wird. Die Mischung wird in eine Zelle mit antiparallelen Orientierungsschichten gefüllt. (Schichtdicke 25 Mikrometer). Die Zelle wird mit einer Halogenlampe (300W) belichtet. Es wird die durch Wellenleitung weitergeleitete Lichtmenge an Fluoreszenzemission an den Kanten parallel und senkrecht zur Orientierungsachse einzeln gemessen und anschließend gemittelt. Hierzu wird das Licht in eine Ulbrichtkugel geleitet und mit einem Spektrometer gemessen. Es wird die Fläche unter dem Emissionsspektrum gebildet und mit dem der Referenzsubstanz D-3 verglichen. Der Vergleich wird als Relativwert in Prozent angegeben, der sich aus den Quotienten der Flächen Messung vs. Referenz ergibt.

Dabei werden für die unten genannten erfindungsgemäßen Verbindungen die folgenden Werte erhalten:

Tabelle 8

| Name | relative Fluoreszenz aus Wellenleitung |
|---|---|
| BT-4 | 108% |
| BT-8 | 106% |
| BT-9 | 110% |
| BT-11 | 111% |
| BT-12 | 113% |
| BT-13 | 113% |
| BT-14 | 111% |

Die erfindungsgemäßen Verbindungen zeigen bei Verwendung in einer Vorrichtung zur Regulierung des Energie-Durchtritts, bei der die absorbierte Lichtenergie über Fluoreszenz wieder emittiert wird, eine hervorragende Fluoreszenzlicht-Ausbeute an den Kanten der Vorrichtung. Damit kann an diesen Stellen das Fluoreszenzlicht aufgenommen und in elektrische Energie umgewandelt werden, beispielsweise mittels Solarzellen. Auf diese Weise können Vorrichtungen zur Regulierung des Energie-Durchtritts erhalten werden, welche autonom, d.h. unabhängig von äußerer Energiezufuhr, geschaltet werden können, da sie absorbierte Lichtenergie über Fluoreszenzemission und anschließende Umwandlung des Fluoreszenzlichts in elektrische Energie zur eigenen Energieversorgung nutzen können.

## Patentansprüche

1. Vorrichtung zur Regulierung des Durchtritts von von der Sonne ausgehendem Licht von einem Außenraum in einen Innenraum hinein, wobei die Vorrichtung eine Schaltschicht umfasst, welche ein flüssigkristallines Medium enthaltend eine oder mehrere verschiedene Verbindungen und mehrere dichroitische Farbstoffe einer Formel (I)

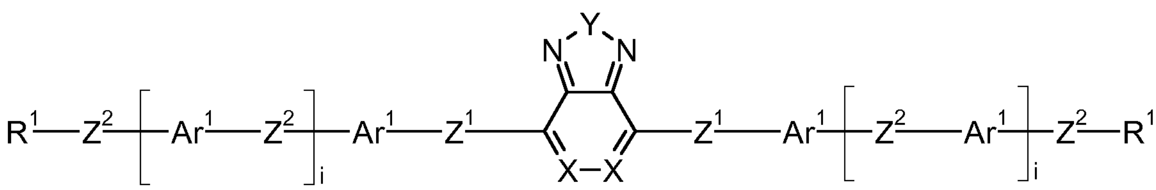

Formel (I),

oder einer Formel (II)

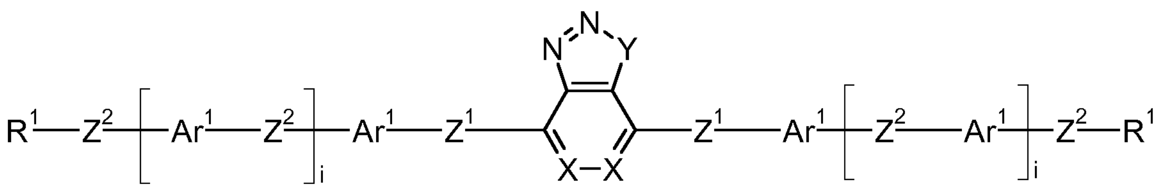

Formel (II),

enthält, wobei gilt:

X ist bei jedem Auftreten gleich oder verschieden $CR^2$ oder N;
Y ist gleich S oder Se;
$Z^1$ ist eine Einfachbindung;
$Z^2$ ist bei jedem Auftreten gleich oder verschieden eine Einfachbindung, O, S, $C(R^3)_2$, $-CR^3=CR^3-$ oder -C≡C-; oder zwei, drei, vier oder fünf miteinander kombinierte Gruppen, gewählt aus den Gruppen O, S, $C(R^3)_2$, $-CR^3=CR^3-$ und -C≡C-;
$Ar^1$ ist bei jedem Auftreten gleich oder verschieden eine Aryl- oder Heteroarylgruppe mit 5 bis 30 aromatischen Ringatomen, die mit einem oder mehreren Resten $R^4$ substituiert sein kann, wobei jeweils mindestens ein $Ar^1$ gewählt ist aus einer schwefelhaltigen Heteroarylgruppe, die mit einem oder mehreren Resten $R^4$ substituiert sein kann;
$R^1$ ist bei jedem Auftreten gleich oder verschieden H, D, F, CN, $N(R^5)_2$, oder eine Alkyl-, Alkoxy- oder Thioalkoxygruppe mit 1 bis 10 C-Atomen, die mit einem oder mehreren Resten $R^5$ substituiert sein kann, wobei eine oder mehrere $CH_2$-Gruppen in den Alkyl-, Alkoxy- oder Thioalkoxygruppen durch $-R^5C=CR^5-$, -C≡C-, C=O, C=S, -C(=O)O-, -OC(=O)-, $Si(R^5)_2$, $NR^5$, -O- oder -S- ersetzt sein können;
$R^2$ ist bei jedem Auftreten gleich oder verschieden H, D, F, Cl, CN, $-(C=O)OR^5$, $-O(C=O)R^5$ oder eine Alkyl-, Alkoxy- oder Thioalkoxygruppe mit 1 bis 10 C-Atomen, die mit einem oder mehreren Resten $R^5$ substituiert sein kann, wobei eine oder mehrere $CH_2$-Gruppen in den Alkyl-, Alkoxy- oder Thioalkoxygruppen durch $-R^5C=CR^5-$, -C≡C-, C=O, C=S, -C(=O)O-, -OC(=O)-, $Si(R^5)_2$, $NR^5$, -O- oder -S- ersetzt sein können;
$R^3$, $R^4$ sind bei jedem Auftreten gleich oder verschieden H, D, F, Cl, CN, oder eine Alkyl-, Alkoxy-, oder Thioalkoxygruppe mit 1 bis 10 C-Atomen, die mit einem oder mehreren Resten $R^5$ substituiert sein kann, wobei eine oder mehrere $CH_2$-Gruppen in den Alkyl-, Alkoxy- oder Thioalkoxygruppen durch $-R^5C=CR^5-$, -C≡C-, C=O, C=S, -C(=O)O-, -OC(=O)-, $Si(R^5)_2$, $NR^5$, -O- oder -S- ersetzt sein können;
$R^5$ ist bei jedem Auftreten gleich oder verschieden H, D, F, Cl, CN, $N(R^6)_2$, eine Alkyl-, Alkoxy- oder Thioalkoxygruppe mit 1 bis 10 C-Atomen oder eine Alkenyl- oder Alkinylgruppe mit 2 bis 20 C-Atomen, wobei die oben genannten Gruppen jeweils mit einem oder mehreren Resten $R^6$ substituiert sein können und wobei eine oder mehrere $CH_2$-Gruppen in den oben genannten Gruppen durch $-R^6C=CR^6-$, -C≡C-, C=O, C=S, -C(=O)O-, -O(C=O)-, $Si(R^6)_2$, $NR^6$, -O- oder -S- ersetzt sein können, oder eine Aryl- oder Heteroarylgruppe mit 5 bis 30 aromatischen Ringatomen, die jeweils mit einem oder mehreren Resten $R^6$ substituiert sein kann;
$R^6$ ist bei jedem Auftreten gleich oder verschieden H, F oder ein aliphatischer organischer Rest mit 1 bis 20 C-Atomen, in dem ein oder mehrere H-Atome durch F ersetzt sein können, oder eine Aryl- oder Heteroarylgruppe mit 5 bis 20 C-Atomen, in der ein oder mehrere H-Atome durch F ersetzt werden können;
i ist gleich 0, 1, 2, 3, 4 oder 5,

wobei das flüssigkristalline Medium der Schaltschicht einen Klärpunkt im Temperaturbereich von 70 °C bis 170 °C hat.

2. Vorrichtung nach Anspruch 1, **dadurch gekennzeichnet, dass** X gleich $CR^2$ ist.

3. Vorrichtung nach Anspruch 1 oder 2, **dadurch gekennzeichnet, dass** Y gleich S ist.

4. Vorrichtung nach einem oder mehreren der Ansprüche 1 bis 3, **dadurch gekennzeichnet, dass** $Z^2$ bei jedem Auftreten gleich oder verschieden für eine Einfachbindung, $-C(R^3)_2C(R^3)_2-$, $-CR^3=CR^3-$, -C≡C-, $-OC(R^3)_2-$ oder $-C(R^3)_2O-$ steht.

5. Vorrichtung nach einem oder mehreren der Ansprüche 1 bis 4, **dadurch gekennzeichnet, dass** die in Anspruch 1 definierte Gruppe $Ar^1$ bei jedem Auftreten gleich oder verschieden eine Arylgruppe mit 6 bis 15 C-Atomen oder eine Heteroarylgruppe mit 5 bis 15 C-Atomen darstellt, die mit einem oder mehreren Resten $R^4$ substituiert sein kann.

6. Vorrichtung nach einem oder mehreren der Ansprüche 1 bis 5, **dadurch gekennzeichnet, dass** die in Anspruch 1 oder 5 definierte Gruppe $Ar^1$ bei jedem Auftreten gleich oder verschieden gewählt ist aus wahlweise mit Resten $R^4$ substituiertem Benzol, Fluoren, Naphthalin, Pyridin, Pyrimidin, Pyrazin, Triazin, Thiophen, Thiophen mit ankondensiertem 1,4-Dioxanring, Benzothiophen, Dibenzothiophen, Benzodithiophen, Cyclopentadithiophen, Thienothiophen, Indenothiophen, Dithienopyrrol, Silolodithiophen, Selenophen, Benzoselenophen, Dibenzoselenophen, Furan, Benzofuran, Dibenzofuran und Chinolin.

7. Vorrichtung nach einem oder mehreren der Ansprüche 1 bis 6, **dadurch gekennzeichnet, dass** $R^1$ bei jedem Auftreten gleich oder verschieden ist H, F, oder eine geradkettige Alkyl- oder Alkoxylgruppe mit 3 bis 8 C-Atomen, die mit einem oder mehreren Resten $R^5$ substituiert sein kann, oder eine verzweigte Alkyl- oder Alkoxygruppe mit 3 bis 8 C-Atomen, die mit einem oder mehreren Resten $R^5$ substituiert sein kann, oder eine cyclische Alkylgruppe mit 6 C-Atomen, die mit einem oder mehreren Resten $R^5$ substituiert sein kann, wobei in den Alkyl- und Alkoxygruppen eine oder mehrere $CH_2$-Gruppen durch -O-, -S- oder $-R^5C=CR^{5-}$ ersetzt sein können, oder eine Siloxanylgruppe mit 1 bis 6 Si-Atomen, die mit einem oder mehreren Resten $R^5$ substituiert sein kann.

8. Vorrichtung nach einem oder mehreren der Ansprüche 1 bis 7, **dadurch gekennzeichnet, dass** der Index i gleich 1 oder 2 ist.

9. Vorrichtung nach einem oder mehreren der Ansprüche 1 bis 8, **dadurch gekennzeichnet, dass** der Anisotropiegrad R der Verbindung der Formel (I) oder Formel (II), ermittelt wie in den Ausführungsbeispielen angegeben, größer ist als 0.4.

10. Vorrichtung nach einem oder mehreren der Ansprüche 1 bis 9, **dadurch gekennzeichnet, dass** sie elektrisch schaltbar ist.

11. Vorrichtung nach einem oder mehreren der Ansprüche 1 bis 10, **dadurch gekennzeichnet, dass** sie mit einer Solarzelle oder einer sonstigen Vorrichtung zur Umwandlung von Licht und/oder Wärmeenergie in elektrische Energie verbunden ist.

12. Fenster, enthaltend eine Vorrichtung nach einem oder mehreren der Ansprüche 1 bis 11.

13. Verbindung gemäß Formel (Ia) oder (Ib)

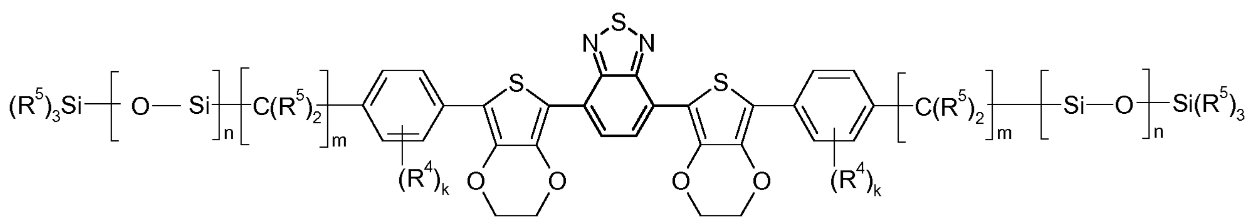

Formel (Ia)

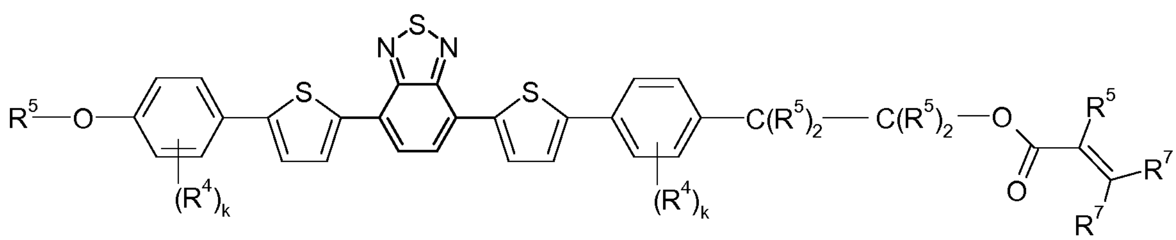

Formel (Ib),

wobei gilt:
die auftretenden Gruppen $R^4$ und $R^5$ sind definiert wie für Formel (I) in Anspruch 1, und

$R^7$ ist bei jedem Auftreten gleich oder verschieden Wasserstoff oder eine Alkylgruppe mit 1 bis 5 C-Atomen,

die mit einem oder mehreren Resten $R^6$ substituiert sein kann;
k ist, gleich oder verschieden bei jedem Auftreten, 0, 1, 2, 3 oder 4;
m ist, gleich oder verschieden bei jedem Auftreten, 0, 1, 2, 3, 4, 5 oder 6;
n ist, gleich oder verschieden bei jedem Auftreten, 1, 2, 3, 4 oder 5.

**14.** Verwendung einer Mischung, welche ein flüssigkristallines Medium und mehrere Verbindungen der wie in Anspruch 1 angegebenen Formel (I) oder Formel (II) enthält, in einer Vorrichtung zur Regulierung des Energie-Durchtritts in Form von von der Sonne ausgehendem Licht von einem Außenraum in einen Innenraum, wobei das flüssigkristalline Medium einen Klärpunkt im Temperaturbereich von 70 °C bis 170 °C hat.

## Claims

**1.** Device for regulating the passage of light emanating from the sun from an outside space into an inside space, where the device includes a switching layer which comprises a liquid-crystalline medium comprising one or more different compounds and a plurality of dichroic dyes of a formula (I)

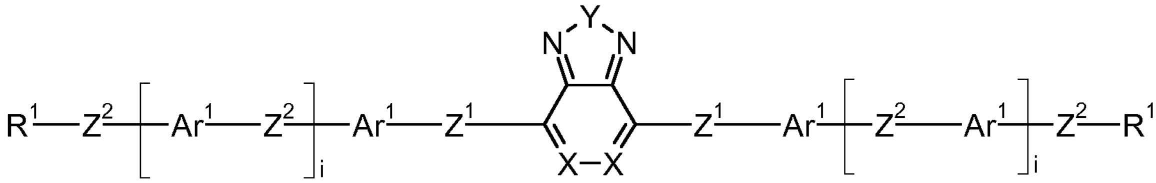

formula (I),

or a formula (II)

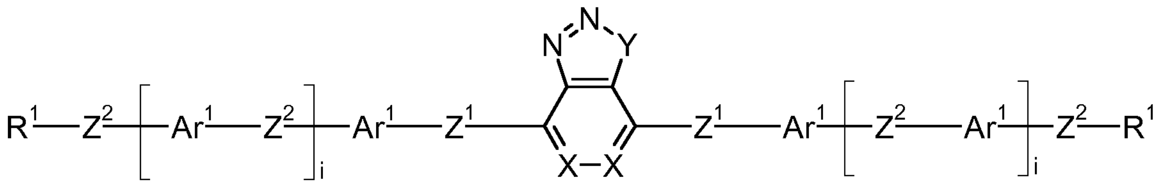

formula (II),

where:

X is on each occurrence, identically or differently, $CR^2$ or N;
Y is equal to S or Se;
$Z^1$ is a single bond;
$Z^2$ is on each occurrence, identically or differently, a single bond, O, S, $C(R^3)_2$, $-CR^3=CR^3-$ or -C≡C-; or two, three, four or five groups selected from the groups O, S, $C(R^3)_2$, $-CR^3=CR^3-$ and -C≡C- combined with one another;
$Ar^1$ is on each occurrence, identically or differently, an aryl or heteroaryl group having 5 to 30 aromatic ring atoms, which may be substituted by one or more radicals $R^4$, where in each case at least one $Ar^1$ is selected from a sulfurcontaining heteroaryl group, which may be substituted by one or more radicals $R^4$
$R^1$ is on each occurrence, identically or differently, H, D, F, CN, $N(R^5)_2$, or an alkyl, alkoxy or thioalkoxy group having 1 to 10 C atoms, which may be substituted by one or more radicals $R^5$, where one or more $CH_2$ groups in the alkyl, alkoxy or thioalkoxy groups may be replaced by $-R^5C=CR^5-$, -C≡C-, C=O, C=S, -C(=O)O-, -OC(=O)-, $Si(R^5)_2$, $NR^5$, -O- or -S-;
$R^2$ is on each occurrence, identically or differently, H, D, F, Cl, CN, $-(C=O)OR^5$, $-O(C=O)R^5$, or an alkyl, alkoxy or thioalkoxy group having 1 to 10 C atoms, which may be substituted by one or more radicals $R^5$, where one or more $CH_2$ groups in the alkyl, alkoxy or thioalkoxy groups may be replaced by $-R^5C=CR^5-$, -C≡C-, C=O, C=S, -C(=O)O-, -OC(=O)-, $Si(R^5)_2$, $NR^5$, -O- or -S-;

$R^3$, $R^4$ are on each occurrence, identically or differently, H, D, F, Cl, CN, or an alkyl, alkoxy or thioalkoxy group having 1 to 10 C atoms, which may be substituted by one or more radicals $R^5$, where one or more $CH_2$ groups in the alkyl, alkoxy or thioalkoxy groups may be replaced by $-R^5C=CR^5-$, -C≡C-, C=O, C=S, -C(=O)O-, -OC(=O)-, $Si(R^5)_2$, $NR^5$, -O- or -S-;
$R^5$ is on each occurrence, identically or differently, H, D, F, Cl, CN, $N(R^6)_2$, an alkyl, alkoxy or thioalkoxy group having 1 to 10 C atoms or an alkenyl or alkynyl group having 2 to 20 C atoms, where the above-mentioned groups may in each case be substituted by one or more radicals $R^6$ and where one or more $CH_2$ groups in the above-mentioned groups may be replaced by $-R^6C=CR^6-$, -C≡C-, C=O, C=S, -C(=O)O-, -O(C=O)-, $Si(R^6)_2$, $NR^6$, -O- or -S-, or an aryl or heteroaryl group having 5 to 30 aromatic ring atoms, which may in each case be substituted by one or more radicals $R^6$;
$R^6$ is on each occurrence, identically or differently, H, F or an aliphatic organic radical having 1 to 20 C atoms, in which one or more H atoms may be replaced by F, or an aryl or heteroaryl group having 5 to 20 C atoms, in which one or more H atoms may be replaced by F;
i is equal to 0, 1, 2, 3, 4 or 5,

where the liquid-crystalline medium of the switching layer has a clearing point in the temperature range from 70°C to 170°C.

**2.** Device according to Claim 1, **characterised in that** X is equal to $CR^2$.

**3.** Device according to Claim 1 or 2, **characterised in that** Y is equal to S.

**4.** Device according to one or more of Claims 1 to 3, **characterised in that** $Z^2$ stands on each occurrence, identically or differently, for a single bond, $-C(R^3)_2C(R^3)_2-$, $-CR^3=CR^3-$, -C=C-, $-OC(R^3)2-$ or $-C(R^3)_2O-$.

**5.** Device according to one or more of Claims 1 to 4, **characterised in that** the group $Ar^1$ defined in Claim 1 represents on each occurrence, identically or differently, an aryl group having 6 to 15 C atoms or a heteroaryl group having 5 to 15 C atoms, which may be substituted by one or more radicals $R^4$.

**6.** Device according to one or more of Claims 1 to 5, **characterised in that** the group $Ar^1$ defined in Claim 1 or 5 is selected on each occurrence, identically or differently, from benzene, fluorene, naphthalene, pyridine, pyrimidine, pyrazine, triazine, thiophene, thiophene with condensed-on 1,4-dioxane ring, benzothiophene, dibenzothiophene, benzodithiophene, cyclopentadithiophene, thienothiophene, indenothiophene, dithienopyrrole, silolodithiophene, selenophene, benzoselenophene, dibenzoselenophene, furan, benzofuran, dibenzofuran and quinoline, each of which is optionally substituted by radicals $R^4$.

**7.** Device according to one or more of Claims 1 to 6, **characterised in that** $R^1$ is on each occurrence, identically or differently, H, F, or a straight-chain alkyl or alkoxy group having 3 to 8 C atoms, which may be substituted by one or more radicals $R^5$, or a branched alkyl or alkoxy group having 3 to 8 C atoms, which may be substituted by one or more radicals $R^5$, or a cyclic alkyl group having 6 C atoms, which may be substituted by one or more radicals $R^5$, where one or more $CH_2$ groups in the alkyl and alkoxy groups may be replaced by -O-, -S- or $-R^5C=CR^5-$, or a siloxanyl group having 1 to 6 Si atoms, which may be substituted by one or more radicals $R^5$.

**8.** Device according to one or more of Claims 1 to 7, **characterised in that** the index i is equal to 1 or 2.

**9.** Device according to one or more of Claims 1 to 8, **characterised in that** the degree of anisotropy R of the compound of the formula (I) or formula (II), determined as indicated in the working examples, is greater than 0.4.

**10.** Device according to one or more of Claims 1 to 9, **characterised in that** it is electrically switchable.

**11.** Device according to one or more of Claims 1 to 10, **characterised in that** it is connected to a solar cell or another device for the conversion of light and/or heat energy into electrical energy.

**12.** Window containing a device according to one or more of Claims 1 to 11.

**13.** Compound of the formula (Ia) or (Ib)

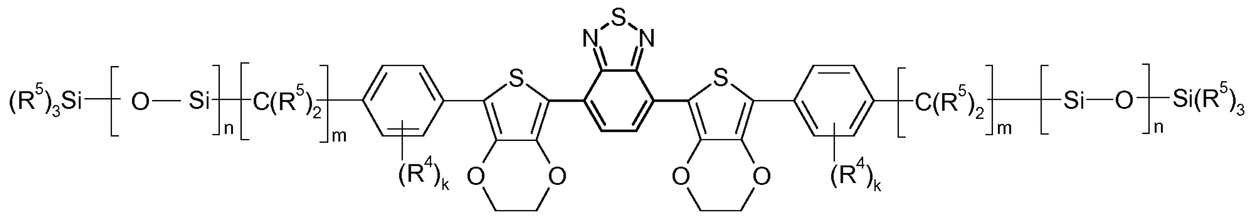

formula (Ia)

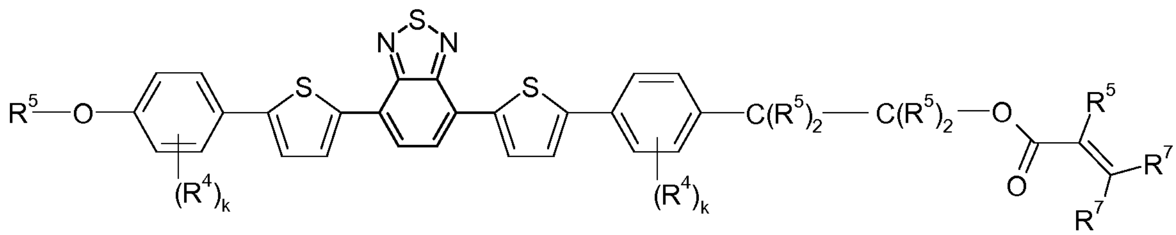

formula (Ib),

where:
the groups $R^4$ and $R^5$ occurring are defined as for formula (I) in Claim 1, and

$R^7$ is on each occurrence, identically or differently, hydrogen or an alkyl group having 1 to 5 C atoms, which may be substituted by one or more radicals $R^6$;
k is, identically or differently on each occurrence, 0, 1, 2, 3 or 4;
m is, identically or differently on each occurrence, 0, 1, 2, 3, 4, 5 or 6;
n is, identically or differently on each occurrence, 1, 2, 3, 4 or 5.

**14.** Use of a mixture which comprises a liquid-crystalline medium and a plurality of compounds of the formula (I) or formula (II) as indicated in Claim 1, in a device for regulating the passage of energy in the form of light emanating from the sun from an outside space into an inside space, where the liquid-crystalline medium has a clearing point in the temperature range from 70°C to 170°C.

**Revendications**

**1.** Dispositif pour réguler le passage de la lumière qui émane du soleil depuis un espace extérieur à l'intérieur d'un espace intérieur, dans lequel le dispositif inclut une couche de commutation qui comprend un milieu cristallin liquide qui comprend un ou plusieurs composé(s) différent(s) et une pluralité de colorants dichroïques d'une formule (I) :

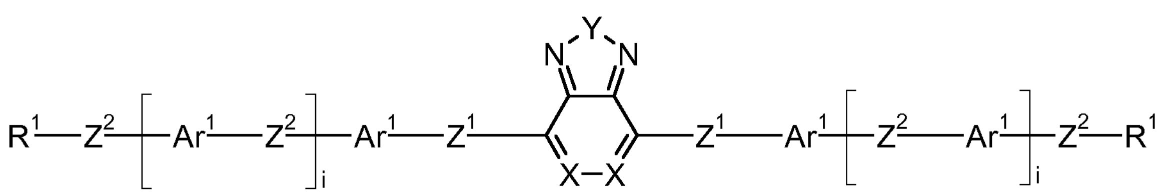

formule (I),

ou d'une formule (II) :

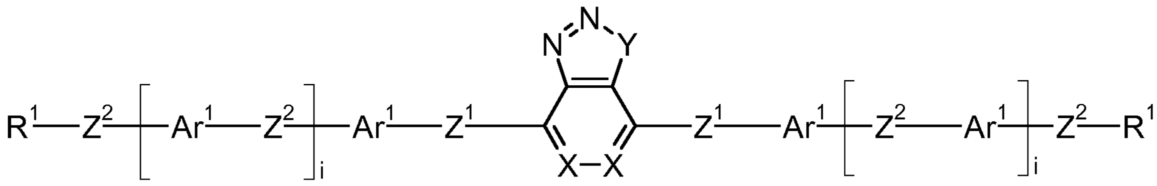

formule (II),

dans lesquelles :

X est pour chaque occurrence, de manière identique ou différente, $CR^2$ ou N ;
Y est égal à S ou Se ;
$Z^1$ est une liaison simple ;
$Z^2$ est pour chaque occurrence, de manière identique ou différente, une liaison simple, O, S, $C(R^3)_2$, $-CR^3=CR^3-$ ou -C≡C- ; ou deux, trois, quatre ou cinq groupes qui sont sélectionnés parmi les groupes O, S, $C(R^3)_2$, $-CR^3=CR^3-$ et -C≡C- et qui sont combinés l'un avec l'autre ou les uns avec les autres ;
$Ar^1$ est pour chaque occurrence, de manière identique ou différente, un groupe aryle ou hétéroaryle qui comporte de 5 à 30 atomes de cycle aromatique, lequel peut être substitué par un radical ou par plusieurs radicaux $R^4$, où, dans chaque cas, au moins un $Ar^1$ est sélectionné parmi un groupe hétéroaryle contenant du soufre, lequel peut être substitué par un radical ou par plusieurs radicaux $R^4$ ;
$R^1$ est pour chaque occurrence, de manière identique ou différente, H, D, F, CN, $N(R^5)_2$, ou un groupe alkyle, alcoxy ou thioalcoxy qui comporte de 1 à 10 atome(s) de C, lequel peut être substitué par un radical ou par plusieurs radicaux $R^5$, où un ou plusieurs groupe(s) $CH_2$ dans les groupes alkyle, alcoxy ou thioalcoxy peut/peuvent être remplacé(s) par $-R^5C=CR^5-$, -C≡C-, C=O, C=S, -C(=O)O-, -OC(=O)-, $Si(R^5)_2$, $NR^5$, -O- ou -S- ;
$R^2$ est pour chaque occurrence, de manière identique ou différente, H, D, F, Cl, CN, $-(C=O)OR^5$, $-O(C=O)R^5$, ou un groupe alkyle, alcoxy ou thioalcoxy qui comporte de 1 à 10 atome(s) de C, lequel peut être substitué par un radical ou par plusieurs radicaux $R^5$, où un ou plusieurs groupe(s) $CH_2$ dans les groupes alkyle, alcoxy ou thioalcoxy peut/peuvent être remplacé(s) par $-R^5C=CR^5-$, -C≡C-, C=O, C=S, -C(=O)O-, -OC(=O)-, $Si(R^5)_2$, $NR^5$, -O- ou -S- ;
$R^3$, $R^4$ sont pour chaque occurrence, de manière identique ou différente, H, D, F, Cl, CN, ou un groupe alkyle, alcoxy ou thioalcoxy qui comporte de 1 à 10 atome(s) de C, lequel peut être substitué par un radical ou par plusieurs radicaux $R^5$, où un ou plusieurs groupe(s) $CH_2$ dans les groupes alkyle, alcoxy ou thioalcoxy peut/peuvent être remplacé(s) par $-R^5C=CR^5-$, -C≡C-, C=O, C=S, -C(=O)O-, -OC(=O)-, $Si(R^5)_2$, $NR^5$, -O- ou -S-;
$R^5$ est pour chaque occurrence, de manière identique ou différente, H, D, F, Cl, CN, $N(R^6)_2$, un groupe alkyle, alcoxy ou thioalcoxy qui comporte de 1 à 10 atome(s) de C ou un groupe alkényle ou alkynyle qui comporte de 2 à 20 atomes de C, où les groupes qui ont été mentionnés ci-avant peuvent dans chaque cas être substitués par un radical ou par plusieurs radicaux $R^6$ et où un ou plusieurs groupe(s) $CH_2$ dans les groupes qui ont été mentionnés ci-avant peut/peuvent être remplacé(s) par $-R^6C=CR^6-$, -C≡C-, C=O, C=S, -C(=O)O-, -O(C=O)-, $Si(R^6)_2$, $NR^6$, -O- ou -S-, ou un groupe aryle ou hétéroaryle qui comporte de 5 à 30 atomes de cycle aromatique, lequel peut dans chaque cas être substitué par un radical ou par plusieurs radicaux $R^6$ ;
$R^6$ est pour chaque occurrence, de manière identique ou différente, H, F ou un radical organique aliphatique qui comporte de 1 à 20 atome(s) de C, où un ou plusieurs atome(s) de H peut/peuvent être remplacé(s) par F, ou un groupe aryle ou hétéroaryle qui comporte de 5 à 20 atomes de C, dans lequel un ou plusieurs atome(s) de H peut/peuvent être remplacé(s) par F ;
i est égal à 0, 1, 2, 3, 4 ou 5 ;

où le milieu cristallin liquide de la couche de commutation présente un point de clarification dans la plage de températures qui va de 70° C à 170° C.

**2.** Dispositif selon la revendication 1, **caractérisé en ce que** X est égal à $CR^2$.

**3.** Dispositif selon la revendication 1 ou 2, **caractérisé en ce que** Y est égal à S.

**4.** Dispositif selon une ou plusieurs des revendications 1 à 3, **caractérisé en ce que** $Z^2$ représente pour chaque occurrence, de manière identique ou différente, une liaison simple, $-C(R^3)_2C(R^3)_2-$, $-CR^3=CR^3-$, -C=C-, $-OC(R^3)_2-$ ou $-C(R^3)_2O-$.

**5.** Dispositif selon une ou plusieurs des revendications 1 à 4, **caractérisé en ce que** le groupe $Ar^1$ qui est défini selon la revendication 1 représente pour chaque occurrence, de manière identique ou différente, un groupe aryle qui comporte de 6 à 15 atomes de C ou un groupe hétéroaryle qui comporte de 5 à 15 atomes de C, lequel peut être substitué par un radical ou par plusieurs radicaux $R^4$.

**6.** Dispositif selon une ou plusieurs des revendications 1 à 5, **caractérisé en ce que** le groupe $Ar^1$ qui est défini selon la revendication 1 ou 5 est sélectionné pour chaque occurrence, de manière identique ou différente, parmi benzène, fluorène, naphtalène, pyridine, pyrimidine, pyrazine, triazine, thiophène, thiophène avec un cycle 1,4-dioxane condensé dessus, benzothiophène, dibenzothiophène, benzodithiophène, cyclopentadithiophène, thiénothiophène, indénothiophène, dithiénopyrrole, silolodithiophène, sélénophène, benzosélénophène, dibenzosélénophène, furane, benzofurane, dibenzofurane et quinoline, dont chacun est en option substitué par des radicaux $R^4$.

**7.** Dispositif selon une ou plusieurs des revendications 1 à 6, **caractérisé en ce que** $R^1$ est pour chaque occurrence, de manière identique ou différente, H, F, ou un groupe alkyle ou alcoxy en chaîne droite qui comporte de 3 à 8 atomes de C, lequel peut être substitué par un radical ou par plusieurs radicaux $R^5$, ou un groupe alkyle ou alcoxy ramifié qui comporte de 3 à 8 atomes de C, lequel peut être substitué par un radical ou par plusieurs radicaux $R^5$, ou un groupe alkyle cyclique qui comporte 6 atomes de C, lequel peut être substitué par un radical ou par plusieurs radicaux $R^5$, où un ou plusieurs groupe(s) $CH_2$ dans les groupes alkyle et alcoxy peut/peuvent être remplacé(s) par -O-, -S- ou $-R^5C=CR^5-$, ou un groupe siloxanyle qui comporte de 1 à 6 atome(s) de Si, lequel peut être substitué par un radical ou par plusieurs radicaux $R^5$.

**8.** Dispositif selon une ou plusieurs des revendications 1 à 7, **caractérisé en ce que** l'indice i est égal à 1 ou 2.

**9.** Dispositif selon une ou plusieurs des revendications 1 à 8, **caractérisé en ce que** le degré d'anisotropie R du composé de la formule (I) ou de la formule (II), déterminé comme indiqué au niveau d'exemples de travail, est supérieur à 0,4.

**10.** Dispositif selon une ou plusieurs des revendications 1 à 9, **caractérisé en ce qu'**il peut être commuté électriquement.

**11.** Dispositif selon une ou plusieurs des revendications 1 à 10, **caractérisé en ce qu'**il est connecté à une cellule solaire ou à un autre dispositif pour la conversion de l'énergie lumineuse et/ou thermique en énergie électrique.

**12.** Fenêtre contenant un dispositif selon une ou plusieurs des revendications 1 à 11.

**13.** Composé de la formule (Ia) ou (Ib) :

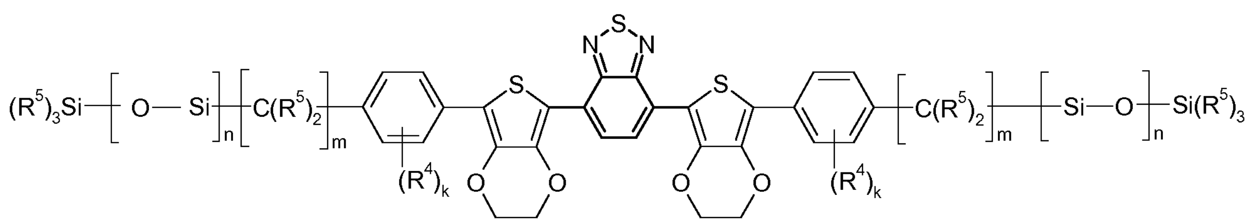

formule (Ia)

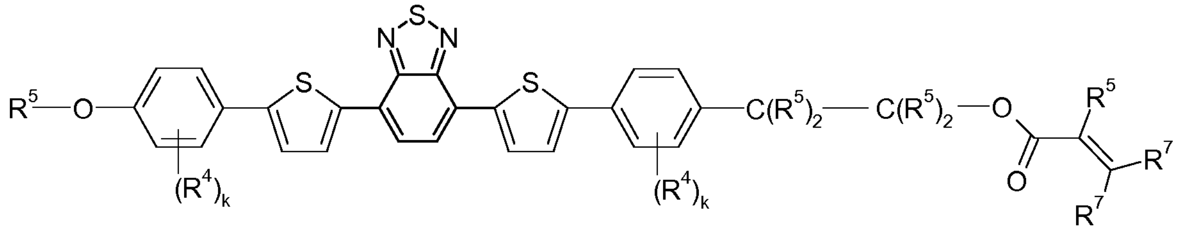

formule (Ib),

dans lesquelles :

les groupes $R^4$ et $R^5$ rencontrés sont définis tel que pour la formule (I) selon la revendication 1 ; et

$R^7$ est pour chaque occurrence, de manière identique ou différente, hydrogène ou un groupe alkyle qui comporte de 1 à 5 atome(s) de C, lequel peut être substitué par un radical ou par plusieurs radicaux $R^6$ ;
k est, de manière identique ou différente pour chaque occurrence, 0, 1, 2, 3 ou 4 ;
m est, de manière identique ou différente pour chaque occurrence, 0, 1, 2, 3, 4, 5 ou 6 ;
n est, de manière identique ou différente pour chaque occurrence, 1, 2, 3, 4 ou 5.

**14.** Utilisation d'un mélange qui comprend un milieu cristallin liquide et une pluralité de composés de la formule (I) ou de la formule (II) tel qu'indiqué selon la revendication 1, dans un dispositif pour réguler le passage de l'énergie sous la forme de la lumière qui émane du soleil depuis un espace extérieur à l'intérieur d'un espace intérieur, dans laquelle le milieu cristallin liquide présente un point de clarification dans la plage de températures qui va de 70° C à 170° C.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 2009141295 A
- US 20100259698 A
- WO 2013004677 A
- EP 12008320 A
- WO 2004002970 A1
- EP 34832 A
- EP 44893 A
- EP 48583 A
- EP 54217 A
- EP 56492 A
- EP 59036 A
- GB 2065158 A
- GB 2065695 A
- GB 2081736 A
- GB 2082196 A
- GB 2094822 A
- GB 2094825 A
- JP OS55123673 A
- DE 3017877
- DE 3040102
- DE 3115147
- DE 3115762
- DE 3150803
- DE 3201120
- DE 3126108
- DE 3202761
- EP 43904 A
- DE 3123519
- WO 822054 A
- GB 2079770 A
- JP OS5657850 A
- JP OS56104984 B
- US 4308161 A
- US 4308162 A
- US 4340973 A
- EP 60895 A
- EP 68427 A
- WO 821191 A
- EP 2166040 A
- US 20110042651 A
- EP 47027 A
- DE 3110960
- EP 698649 A
- WO 2010118422 A
- US 7038745 B
- US 6099758 A
- US 20110043732 A
- US 7023602 B
- WO 2012052100 A


### In der Beschreibung aufgeführte Nicht-Patentliteratur


- *ChemPhysChem,* 2012, vol. 13, 597
- *Chem. Eur. J.,* 2008, vol. 14, 11231
- *J. Am. Chem. Soc.,* 1995, vol. 117, 6791
- *J. Mater. Chem.,* 2006, vol. 16, 736
- **MANFRED RICHTER.** Einführung in die Farbmetrik. Verlag Walter de Gruyter & Co, 1981
- **B. BAHADUR.** Liquid Crystals - Applications and Uses. World Scientific Publishing, 1992, vol. 3
- **T. UCHIDA ; C. SHISHIDO ; H. SEKI ; M. WADA.** *Mol. Cryst. Liq. Cryst.,* 1977, vol. 39, 39-52
- **H. SEKI ; C. SHISHIDO ; S. YASUI ; T. UCHIDA.** *Jpn. J. Appl. Phys.,* 1982, vol. 21, 191-192
- **JOSEPH R. LAKOWICZ.** Principles of Fluorescence Spectroscopy. Verlag Springer Science+Business Media LLC, 2010, 279
- **D. S. KLIGER et al.** Polarized Light in Optics and Spectroscopy. Academic Press, 1990
- **MANFRED RICHTER.** Einführung in die Farbmetrik. 1991